# EUROPEAN PATENT APPLICATION

(11) **EP 4 548 857 A2**
(43) Date of publication of application: **07.05.2025**
(21) Application number: 25164275.7
(22) Date of filing: 17.03.2020
(51) Int. Cl.: A61B 17/068

(54) **ATRIOVENTRICULAR REGURGITATION REDUCTION SYSTEM ANCHORS**

(30) Priority: 25.03.2019 US 201962823465 P
(62) Divisional of application: 20718087.8
(71) Applicant: Edwards Lifesciences Corporation, Irvine, CA 92614 (US)
(72) Inventor: Passman, Joseph Arthur, IRVINE, CA, 92614 (US); Katbamna, Rohan Yogee, IRVINE, CA, 92614 (US); Ma, Minh T., IRVINE, CA, 92614 (US); Dalton, Timothy Allen, IRVINE, CA, 92614 (US); Stone, Aric Daniel, IRVINE, CA, 92614 (US); Islas, Jose, IRVINE, CA, 92614 (US); Geist, Stephen C., IRVINE, CA, 92614 (US)
(74) Representative: Eisenführ Speiser

(57) **Abstract**

Devices for anchoring catheter systems, such as an atrioventricular heart valve regurgitation reduction system with a coapting element or spacer positioned within the atrioventricular valve leaflets. The spacer may be anchored above the atrioventricular valve with a rail that extends up into the subclavian vein, with tethers that attach to anchors around the annulus, or with a tether that connects to a stent expanded within an adjacent blood flow passage. The spacer may also be anchored below the atrioventricular valve with a rail that terminates in a tissue anchor within the ventricle, with tethers that attach to anchors around ventricle, or with a tether that connects to a stent expanded within an adjacent blood flow passage. The spacer may also be stabilized by a harness extending across the valve annulus or may be self-anchoring with an array of struts that extend into contact with the surrounding tissue.

## Description

### TECHNICAL FIELD

The present disclosure relates generally to devices and methods for anchoring percutaneously-delivered catheter systems such as an atrioventricular regurgitation prevention system.

### BACKGROUND

Heart valve disease, such as valve regurgitation, is typically treated by replacing or repairing the diseased valve during open-heart surgery. However, open-heart surgery is highly invasive and is therefore not an option for many patients. For high-risk patients, a less-invasive method for repair of heart valves is considered generally advantageous.

One solution is seen in U.S. Patent No. 9,474,605, expressly incorporated herein, which discloses a heart valve repair system for reducing regurgitation through a native atrioventricular valve. A flexible rail having a ventricular anchor on the distal end thereof adapted to anchor into tissue within a ventricle is first deployed percutaneously. A repair catheter passes along the flexible rail, and a leaflet coaptation member on a distal end of the catheter is located within the native valve leaflets. Finally, a locking collet on the delivery catheter secures the axial position of the coaptation member and delivery catheter on the flexible rail. When in place, the coaptation member reduces or eliminates regurgitation through the native valve, in particular a tricuspid heart valve.

Alternative anchors are of interest to simplify the procedure and/or increase anchor robustness.

### SUMMARY

The present invention relates generally to devices and methods for maintaining a coaptation member between the atrioventricular leaflets to prevent regurgitation. The devices and methods disclosed herein are particularly useful in reducing regurgitation through the two atrioventricular (AV) valves, which are between the atria and the ventricles - i.e., the mitral valve and the tricuspid valve.

One area in which the present application seeks to maintain a coaptation member between the atrioventricular leaflets is in improved tissue anchors. For instance, several exemplary tissue anchors for medically implanted systems each comprises a tubular sleeve attached to a distal end of an elongated flexible shaft defining a longitudinal axis. A hub attaches to a distal end of an elongated flexible rail concentrically arranged and configured to slide axially within the flexible shaft so as to expel the hub from within the sleeve. The hub is sized to fit within the tubular sleeve and has a plurality of separate prongs with sharp free ends attached to and extending from a distal end of the hub.

In a first tissue anchor configuration, the prongs have an undeployed configuration in which the prongs generally extend distally from the hub and are radially constrained by the sleeve, and a deployed configuration wherein the prongs bend radially outward when unconstrained by the sleeve, the prongs bending approximately 90° radially outward in the deployed configuration.

In a second tissue anchor configuration, the prongs have an undeployed configuration in which the prongs generally extend distally from the hub and are radially constrained by the sleeve, and a deployed configuration wherein the prongs bend radially outward and back in a proximal direction when unconstrained by the sleeve. The prongs each have a series of small side barbs arrayed along a length of the prong from the hub to the free end.

In a third tissue anchor configuration, the prongs having an undeployed configuration in which the prongs generally extend distally from the hub and are radially constrained by the sleeve, and a deployed configuration wherein the prongs bend radially outward and back in a proximal direction when unconstrained by the sleeve. The prongs each have a small bent tip that points outward once the prong is bent in a proximal direction, and wherein each prong is highly flexible and filament-like with a cross-sectional dimension of between about 0.1-2.0 mm.

In a fourth tissue anchor configuration, the hub further includes a plurality of peripheral openings circumferentially aligned with each prong located proximally from the distal end of the hub. The prongs have an undeployed configuration in which the prongs generally extend distally from the hub and are radially constrained by the sleeve, and a deployed configuration wherein the prongs curl radially outward and back in a proximal direction when unconstrained by the sleeve. Further, the prongs curl approximately 360° such that their sharp free ends reside within the openings in the hub to form a closed loop.

In a fifth tissue anchor configuration, the hub further includes a plurality of elongated fingers aligned with each prong located proximally from the distal end of the hub. The fingers are formed in a side wall of the hub cantilevered in a proximal direction terminating in free ends. The prongs have an undeployed configuration in which the prongs generally extend distally from the hub and are radially constrained by the sleeve, and a deployed configuration wherein the prongs curl radially outward and back in a proximal direction when unconstrained by the sleeve. In addition, the fingers having an undeployed configuration in which the fingers generally extend proximally when radially constrained by the sleeve, and a deployed configuration wherein the fingers curl radially outward and back in a distal direction when unconstrained by the sleeve. The prongs and the fingers both curl far enough such that their free ends come into proximity with each other and form a closed loop.

In a still further exemplary tissue anchor for medically implanted systems, a tubular sleeve attaches to a distal end of an elongated flexible shaft defining a longitudinal axis. A plurality of separate prongs are slidably disposed within the sleeve and have inwardly bent free ends. The prongs have an undeployed configuration in which the prongs are located substantially within the sleeve and radially constrained thereby, and a deployed configuration wherein the prongs are advanced distally from within the sleeve. The prongs each have a radially outward spring bias so as to separate in the deployed configuration such that the bent free ends extend generally in a distal direction.

The present application also discloses anchoring systems for preventing heart valve regurgitation that anchor an atrioventricular spacer within an atrioventricular valve between an atrium and the ventricle. The systems each have a spacer with a proximal end and a distal end and is sized to fit within leaflets of the atrioventricular valve. The spacer is configured to coapt against the leaflets to reduce regurgitation therebetween, and has a length such that the proximal end resides within the atrium and the distal end resides within the ventricle.

In one anchoring system, at least one tether connects to the distal end of the spacer and extends outward therefrom. A tissue anchor attached to the tether has an expanded configuration with a flat, disk shape formed by a flexible internal support ring covered in fabric, and a linear collapsed configuration shaped so as to pass through a lumen of a delivery instrument. The tether attaches to a central location on the tissue anchor when in the expanded configuration.

In a second anchoring system, an array of tethers connect to the proximal end and/or the distal end of the spacer and extending outward therefrom, each tether terminating in a tissue anchor suitable for anchoring to tissue within the atrium and/or ventricle. Preferably, each of the tethers engages the spacer such that a length between the spacer and the associated tissue anchor is adjustable and lockable.

In a third anchoring system, an elongated anchoring strip connects to the distal end of the spacer, the anchoring strip having a length sufficient to reach an inner wall of the ventricle and having a plurality of tissue anchors mounted thereon for anchoring a length of the strip within the ventricle.

In a fourth anchoring system, a circumferentially-distributed array of anchoring legs attaches to and extends from the distal end of the spacer. The anchoring legs curl back approximately 180° to extend in a proximal direction and have a length to contact an underside of the leaflets so as to retain the spacer in place.

In a fifth anchoring system, a circumferentially-distributed array of anchoring legs attaches to and extends from the proximal end of the spacer. The anchoring legs each curl back approximately 180° to extend in a distal direction and have a length to contact the valve annulus. A plurality of tissue anchors in the same number as the anchoring legs attach around the annulus, with each anchoring leg being connected to a tissue anchor so as to retain the spacer in place.

In a fifth anchoring system, a harness for stabilizing the spacer within the atrioventricular valve includes at least one filament anchored to opposite sides of the annulus and extending across a midpoint of the atrioventricular valve. The filament(s) each define a snare at an approximate midpoint sized to closely encircle the spacer. Desirably, the spacer is expandable, and the snare has an adjustable size which expands upon expansion of the spacer to impart tension to opposite sides of the annulus and reduce the circumference of the annulus, as in an annuloplasty procedure.

A slightly different anchoring system also has a spacer having a proximal end and a distal end sized to fit within leaflets of the atrioventricular valve that extend inward from a surrounding base annulus. The spacer is configured to coapting against the leaflets to reduce regurgitation therebetween, and has a length such that the proximal end resides within the atrium and the distal end resides within the ventricle. A circumferentially-distributed array of anchoring members attaches to and extends from both proximal and distal ends of the spacer, the anchoring members each extending radially outward and having a length to contact opposite sides of the leaflets or base annulus so as to retain the spacer in place.

A dual-support type of anchoring system for anchoring an atrioventricular spacer within an atrioventricular valve between an atrium the ventricle starts with a spacer having a proximal end and a distal end and is sized to fit within leaflets of the atrioventricular valve that extend inward from a surrounding base annulus. The spacer is configured to coapt against the leaflets to reduce regurgitation therebetween, the spacer having a length such that the proximal end resides within the atrium and the distal end resides within the ventricle.

In one dual-support type of anchoring system, an array of annulus anchoring members attached to and extending from the proximal end of the spacer each extending radially outward and have a length to contact the leaflets or base annulus. Further, an array of atrial elongated anchoring members each extend to atraumatic ends. A spring-loaded shaft is placed between the proximal end of the spacer and the array of atrial anchoring members such that a combination of contact between the atraumatic ends of the atrial anchoring members and an adjacent atrial wall and contact between the array of annulus anchoring members and the annulus holds the spacer in place within the leaflets.

In a second dual-support type of anchoring system, an array of atrial anchoring members each extend to atraumatic ends, and an array of ventricular anchoring members each extending to atraumatic ends. A spring-loaded shaft is placed between the proximal end of the spacer and the array of atrial anchoring members. Another spring-loaded shaft is placed between the distal end of the spacer and the array of ventricular anchoring members. A combination of contact between the array of atrial anchoring members and an adjacent atrial wall and contact between the array of ventricular anchoring members and an adjacent ventricular wall holds the spacer in place within the leaflets.

A further system is disclosed for anchoring an atrioventricular spacer within an atrioventricular valve between an atrium the ventricle, the valve having leaflets. The system comprises a compressible and expandable spacer having a proximal end and a distal end and sized to fit within the leaflets of the atrioventricular valve and configured to coapt against the leaflets to reduce regurgitation therebetween. The spacer has a long dimension with a length such that the proximal end resides within the atrium and the distal end resides within the ventricle, and a compressed delivery configuration and a radially expanded implant configuration. A suspension member connects to the proximal end of the spacer and extends proximally therefrom. A tubular slider coupling defines a longitudinal axis to which the suspension member is hingedly connected such that the long dimension of the spacer may be oriented parallel to the longitudinal axis or pivoted away from the longitudinal axis. A central shaft disposed concentrically through the slider coupling is adapted for sliding longitudinal movement relative to the slider coupling. The slider coupling has a locking mechanism that, when activated, prevents longitudinal movement of the slider coupling relative to the central shaft. The central shaft has a distal implant segment and a proximal delivery segment joined at a separable junction, wherein the segments may be decoupled to leave just the distal implant segment of the central shaft in the body. A distal stabilizing element is fixedly attached to the central shaft on a distal side of the slider coupling. The distal stabilizing element has a compressed delivery configuration and a radially expanded implant configuration adapted to anchor in a vascular ostia opening to the atrium. A pusher shaft is concentrically disposed around and slidable relative to the central shaft, wherein the pusher shaft is shorter than the central shaft and has a locking member at a proximal end adapted to alternately lock and release the pusher shaft for sliding movement relative to the central shaft. The pusher shaft has a distal implant segment and a proximal delivery segment joined at a separable junction, wherein the segments may be decoupled to leave just the distal implant segment of the pusher shaft in the body. A proximal stabilizing element is fixedly attached to the pusher shaft on a proximal side of the slider coupling. The proximal stabilizing element has a compressed delivery configuration and a radially expanded implant configuration adapted to anchor in a vascular ostia opening to the atrium. A constriction sheath is concentrically arranged around the central shaft, distal stabilizing element, pusher shaft, proximal stabilizing element and spacer. The constriction sheath is oriented along the longitudinal axis and sized to surround and maintain the spacer, distal stabilizing element and proximal stabilizing element in their compressed delivery configurations. The central shaft has a caval spanning segment with a length between the distal stabilizing element and proximal stabilizing element sufficient to span across an atrium between two vascular ostia opening to the atrium. In use, the spacer may be pivoted away from the longitudinal axis of the slider coupling and suspended within the atrioventricular valve so as to coapt against the leaflets and reduce regurgitation therebetween.

The system described above preferably further includes a release shaft concentrically disposed around the central shaft and within the pusher shaft and slidable relative to both the central shaft and the pusher shaft. The release shaft is shorter than the central shaft and longer than the pusher shaft and has a locking member at a proximal end that acts directly on the central shaft to alternately lock and release the release shaft for sliding movement relative to the central shaft. The locking member of the pusher shaft acts directly on the release shaft, and the release shaft has a distal implant segment and a proximal delivery segment joined at a separable junction, wherein the segments may be decoupled to leave just the distal implant segment of the release shaft in the body. Preferably, the slider coupling comprises a generally tubular member having opposite ends and the locking mechanism comprises a plurality of flexible petals on the opposite ends that are biased inward and clamp onto the release shaft. Retraction of the release shaft while holding the slider coupling stationary relative to the central shaft pulls the release shaft from within the slider coupling and permits the petals to clamp onto the central shaft. The constriction sheath is desirably connected to a proximal hub with a lock that acts directly on the pusher shaft and is adapted to alternately lock and release the constriction sheath for sliding movement relative to the pusher shaft. The spacer may comprise an open cell foam interior covered with a flexible outer cover formed of a polycarbonate urethane. The distal and proximal stabilizing elements preferably comprise tubular self-expanding Nitinol stents. The system further may include a delivery sheath concentrically arranged around the constriction sheath, central shaft, distal stabilizing element, pusher shaft, proximal stabilizing element and spacer. The delivery sheath is oriented along the longitudinal axis and sized to advance the constriction sheath with the spacer, distal stabilizing element and proximal stabilizing element in their compressed delivery configurations inside the constriction sheath. The suspension member is preferably hingedly connected to the slider coupling so that the spacer pivots away from the slider coupling in one plane. The suspension member may be formed as a flat strip connected at a living hinge to the slider coupling to provide rotational stability.

A further understanding of the nature and advantages of the present invention are set forth in the following description and claims, particularly when considered in conjunction with the accompanying drawings in which like parts bear like reference numerals.

### BRIEF DESCRIPTION OF THE DRAWINGS

To further clarify various aspects of embodiments of the present disclosure, a more particular description of the certain embodiments will be made by reference to various aspects of the appended drawings. It is appreciated that these drawings depict only typical embodiments of the present disclosure and are therefore not to be considered limiting of the scope of the disclosure. Moreover, while the figures may be drawn to scale for some embodiments, the figures are not necessarily drawn to scale for all embodiments. Embodiments of the present disclosure will be described and explained with additional specificity and detail through the use of the accompanying drawings.
Figure 1 is a schematic view of the final configuration of a percutaneous heart valve regurgitation reduction system having a coapting element or spacer positioned within the tricuspid valve and a proximal length of the repair catheter including a locking collet shown exiting the subclavian vein to remain implanted subcutaneously;
Figures 2A and 2B are partial sectional views of a prior art tissue anchor used to secure a rail element of a regurgitation reduction system prior to and after deployment of anchoring prongs;
Figures 3A-3C are longitudinal sectional views through a prior art tissue anchoring system that regulates a deployment force in several stages of operation;
Figures 4A and 4B are partial sectional views of a first embodiment of a tissue anchor of the present application prior to and after deployment of anchoring prongs;
Figure 5A is a partial sectional view of another exemplary tissue anchor with a plurality of serrated anchoring prongs in a deployed configuration, and Figure 5B is an end view of the tissue anchor showing the anchoring prongs in profile;
Figures 6A-6C are schematic views showing deployment into tissue of a still further exemplary tissue anchor having filament-like prongs that deflect outward into tissue and lock into place when subjected to tension;
Figures 7A-7C are several views of an alternative tissue anchor having prongs that curl 360° to form closed shapes;
Figures 8A-8C are various views of a still further tissue anchor having locking prongs;
Figure 9 is a perspective view of a distal end of another tissue anchor of the present application having a plurality of inwardly-bent prongs;
Figures 10A-10C show several steps in deployment of the tissue anchor of Figure 9;
Figure 11 shows one embodiment of a corkscrew-like tissue anchor, and Figure 12 shows a second embodiment;
Figure 13 illustrates a compound corkscrew-like tissue anchor, and corkscrew-like tissue anchor 13A-13C illustrate deployment thereof;
Figure 14 illustrates another embodiment of a corkscrew-like tissue anchor;
Figures 15A and 15B are sectional views of the right side of a human heart illustrating two different placements of a simple suture loop that may be used to anchor a regurgitation reduction system;
Figures 16A-16F show a sequence of steps in deploying a suture loop through tissue used as an anchor for a regurgitation reduction system;
Figure 17 is a sectional view of the right side of the human heart showing a spacer for a regurgitation reduction system anchored in the right ventricle with a compound knot on the end of an anchoring suture, and Figure 17A is a detailed view of the compound knot;
Figure 17B is a side elevational view of a tool used to deploy the compound knot shown in Figure 17A;
Figure 18 is a plan view of another deployed tissue anchor having a flat disk shape, while Figures 18A and 18B illustrate the disk-shaped anchor deployed on the exterior of the right ventricle of the heart and through the inter-ventricular septum, respectively, in use as an anchor for a regurgitation reduction spacer;
Figures 19A-19C illustrate an inner elastomeric ring and outer fabric components of the disk-shaped anchor of Figure 18, and Figures 19D and 19E schematically illustrate how the disk-shaped anchor is loaded into and expelled from a deployment instrument;
Figures 20A-20D illustrate layers of the heart pierced by a puncturing tool when deploying the disk-shaped anchor of Figure 18;
Figures 21A and 21B are partial sectional and top plan views of a regurgitation reduction spacer having an array of both sub- and supra-annular anchors distributed therearound;
Figures 22A-22D illustrate alternative arrays of both sub- and supra-annular anchors distributed around a regurgitation reduction spacer;
Figure 23A is a perspective view of an exemplary control handle for adjusting an array of anchors of the present application, and Figure 23B is an exemplary anchoring array on a regurgitation reduction spacer that may be controlled by the handle;
Figures 24A and 24B are perspective views of an exemplary deployment instrument for a regurgitation reduction system;
Figures 25A-25E are cross-sectional views of a flexible shaft forming a part of the deployment instrument, taken along line 25-25 of Figure 24B, and showing alternative configurations of multiple elongated tethers passed therethrough;
Figure 26 is a side elevational view of a regurgitation reduction spacer having an anchoring strip extending from the distal end thereof capable of deploying a plurality of anchors into ventricular tissue;
Figures 26A-26C illustrate various placements of the anchoring strip shown in Figure 26 in the right ventricle;
Figure 27 illustrates a sub-annular anchor for a regurgitation reduction spacer having a plurality of anchoring legs which extend underneath the valve leaflets;
Figures 27A and 27B are perspective and elevation views of the regurgitation reduction spacer with sub-annular anchoring legs;
Figures 28A-28C are perspective and orthogonal views of one example of the sub-annular anchoring legs of Figure 27;
Figure 29A is a perspective view from above a native valve showing a supra-annular anchor for a regurgitation reduction spacer, and Figure 29B is a vertical sectional view of the same system;
Figures 30A-30H are vertical sectional views illustrating several steps in deployment of the regurgitation reduction spacer and supra-annular anchor of Figure 29A;
Figures 31A and 31B illustrate a regurgitation reduction spacer having retention anchors on both ends that extend radially outward to center the spacer in the annulus;
Figures 32A-32B show a deployment sequence for the spacer of Figures 31A and 31B;
Figures 32C illustrates an alternative version of the spacer of Figures 31A and 31B without a proximal shaft;
Figure 33 is a top plan view of a tricuspid annulus and leaflets showing one embodiment of a supra-annular harness for anchoring a regurgitation reduction spacer;
Figures 34A and 34B are perspective views of the tricuspid annulus showing alternative supra-annular harnesses;
Figures 35A and 35B schematically illustrate deployment of a regurgitation reduction spacer using a supra-annular harness extending diametrically across the valve annulus;
Figures 36A and 36B schematically illustrate deployment of a regurgitation reduction spacer using a supra-annular harness extending diametrically in a cross configuration across the valve annulus;
Figures 37A and 37B are perspective pre- and post-deployment views of a regurgitation reduction spacer within a supra-annular harness;
Figures 38A-38C schematically illustrate steps in deploying one of the supra-annular harnesses;
Figure 39 is a schematic illustration of a human body showing a femoral access path for a delivery sheath for placing a regurgitation reduction spacer and associated atrial anchors at the tricuspid valve, with an enlarged view of the heart in section;
Figures 40A-40D illustrate several steps in deploying a regurgitation reduction spacer and associated atrial anchors at the tricuspid valve;
Figures 41A and 41B are perspective and side views of an exemplary regurgitation reduction spacer and associated atrial anchors;
Figure 42 is a schematic sectional view of the right side of the heart and one means for positioning an exemplary regurgitation reduction spacer and associated atrial anchors;
Figure 43 is a side view of an exemplary regurgitation reduction spacer having both atrial and ventricular anchors;
Figure 44 illustrates a still further alternative for anchoring a regurgitation reduction spacer by deploying a stent in a supra-annular flow passage;
Figures 45A and 45B show two steps in deployment of another alternative anchoring solution for a regurgitation reduction spacer in the tricuspid valve including deploying a stent beyond the adjacent pulmonary valve;
Figures 46A and 46B are side and perspective views of a single-stent anchoring system for an adjustably positioned regurgitation reduction spacer;
Figures 47A-47C schematically illustrate three steps in positioning the regurgitation reduction spacer having the single-stent anchoring system of Figures 46A and 46B;
Figure 48 is a longitudinal sectional view through a portion of the single-stent anchoring system of Figures 46A and 46B;
Figures 49A-49C schematically illustrate three steps in positioning a regurgitation reduction spacer utilizing an alternative single-stent anchoring system;
Figure 50 is a longitudinal sectional view through a portion of the single-stent anchoring system of Figures 49A-49C, and Figure 50A is an enlarged view of a portion thereof;
Figure 51 is a longitudinal sectional view through a portion of a still further single-stent anchoring system that utilizes just three shafts;
Figure 52 is a schematic view showing a dual-stent anchoring solution for an adjustably positioned regurgitation reduction spacer of the present application suspended across the right atrium;
Figure 53 is a side view of the dual-stent anchoring system and spacer of Figure 52 when deployed, and Figure 54 shows the assembly in a crimped configuration;
Figures 55A and 55B show a spacer locking mechanism prior to and after deployment;
Figure 56 is an enlarged view of one embodiment of a disconnect between two tubular shafts;
Figures 57A-57U show a sequence of operation for the dual-stent anchoring system and spacer of Figures 52-53;
Figures 58A and 58B are enlarged views of the dual-stent anchoring system and spacer of Figures 52-53;
Figure 59 is a perspective view of an exemplary control handle which may be used with various valve regurgitation reduction systems described herein, and Figure 60 is a partial exploded view thereof;
Figures 61A and 61B are elevational and perspective views of a control shaft for use in the exemplary control handle of Figure 60;
Figures 62A-62C are several views of a wire wrapping sleeve used in conjunction with the control shaft of Figures 61A and 61B for securing a proximal end of a deflection wire;
Figure 63 is an elevational view of the control shaft showing a deflection wire extending therealong, a slide nut and the wire wrapping sleeve;
Figures 64A-64D are snapshots of an exemplary sequence for wrapping the deflection wire around the wire wrapping sleeve;
Figures 65A-65D are views showing alternative sequences for wrapping the deflection wire around the wire wrapping sleeve; and
Figures 66A and 66B are perspective views of an alternative assembly of the control shaft, slide nut and wire wrapping sleeve where a cable sheath surrounds the deflection wire for use in a tight-radius deflection system.

### DETAILED DESCRIPTION OF THE PREFERRED EMBODIMENTS

The following description refers to the accompanying drawings, which illustrate specific embodiments. Other embodiments having different structures and operation do not depart from the scope of the present disclosure.

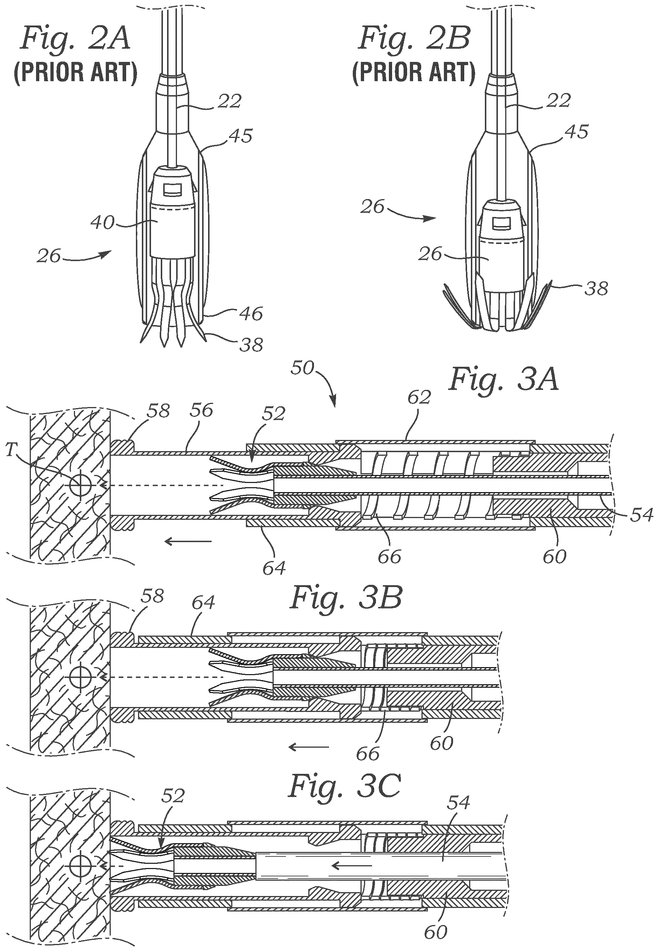

A particular heart valve regurgitation reduction system may be implanted within the left or right side of the heart and extends out of the heart into the vasculature, for example, to the subclavian vein. However, the principles disclosed herein for anchoring such an implanted device are suitable for other applications as well. Moreover, it is important to state that the combination of any two or more of the various anchoring solutions is contemplated, except for those that would be mutually exclusive or impractical. Several supra-annular anchors are disclosed along with a number of sub-annular anchors, and though one or more may be shown utilized alone, its combination with other anchors is entirely possible. For instance, Figures 3-19 show different sub-annular anchors that are deployed in the ventricle, and any may be combined with any of the supra-annular anchors disclosed elsewhere.

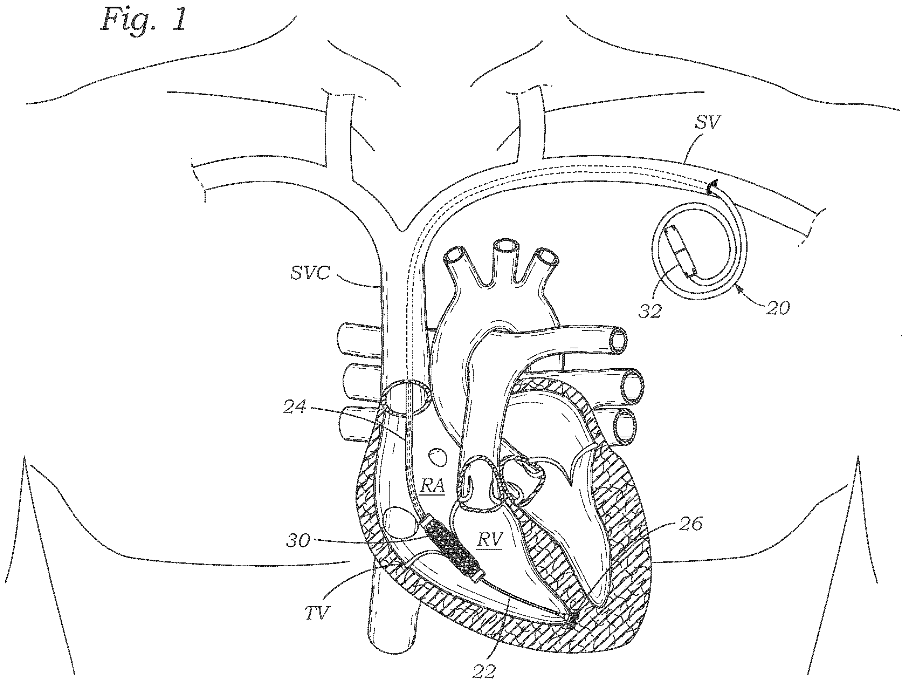

Figure 1 is a schematic view of the final implanted configuration of a percutaneous heart valve regurgitation reduction system having a coapting element or spacer positioned within the tricuspid valve and a proximal length of the repair catheter including a locking collet shown exiting the subclavian vein to remain implanted subcutaneously. The system includes a repair catheter 20 percutaneously delivered into the right side of the heart to reduce tricuspid valve TV regurgitation. Therefore, anatomical structures of the right atrium RA and right ventricle RV will be explained in greater detail, though it should be understood that the devices described herein may equally be used to treat the mitral valve MV on the left side of the heart.

The repair catheter 20 enters the right atrium RA from the superior vena cava SVC after having been introduced to the subclavian vein SV using well-known methods, such as the Seldinger technique. The repair catheter 20 preferably tracks over a smaller diameter pre-installed anchor rail 22 that has also been inserted into the subclavian vein SV and steered through the vasculature until it resides and is anchored at or near the apex of the right ventricle RV, as shown. The repair catheter 20 includes an elongated hollow shaft 24 that may be reinforced, for example, with an embedded braided or coiled structure.

A distal device anchor 26 secures a distal end of the rail 22 at the apex of the right ventricle RV, or to other anatomical features within the ventricle. The anchor rail 22 may be constructed as a braided wire rod, or cable, and is desirably hollow to enable passage over a guide wire (not shown). Further details of the anchor rail 22 and device anchor 26 are seen in U.S. Patent Nos. 8,932,348 and 9,474,605, the entire disclosures of which are expressly incorporated herein by reference.

The repair catheter shaft 24 carries a coapting element or spacer 30 on its distal end portion that is ultimately positioned within the tricuspid valve TV, the leaflets of which are shown closed in systole and in contact with the spacer 30. As mentioned above, the regurgitation reduction systems can be effectively deployed at either the tricuspid or mitral valves, the former which typically has three leaflet cusps defined around the orifice while the latter has just two. A variety of coapting elements may be utilized, the common feature of which is the goal of providing a plug of sorts within the heart valve leaflets to mitigate or otherwise eliminate regurgitation. In the illustrated embodiment, the spacer 30 includes an expandable body that may be adjusted *in vivo,* such as disclosed in U.S. Patent Provisional No. 62/675,914 (ADV-8656), while other coapting elements are disclosed in U.S. Patent Nos. 9,474,605 and 9,636,223, the entire disclosures which are expressly incorporated herein by reference. The spacer 30 is delivered in a radially contracted state to reduce the size of the incision used and facilitate passage through the vasculature and is then expanded within the valve leaflets.

A locking mechanism is provided on the regurgitation repair catheter 20 to lock the axial position of the spacer 30 within the tricuspid valve TV and relative to the fixed anchor rail 22. For example, a locking collet 32 along the length of the repair catheter shaft 24 permits the physician to selectively lock the position of the shaft, and thus the connected spacer 30, along the anchor rail 22. There are of course a number of ways to lock a catheter over a concentric guide rail, and the application should not be considered limited to the illustrated embodiment. For instance, rather than a locking collet 32, a crimpable section such as a stainless-steel tube may be included on the repair catheter shaft 24 at a location near the skin entry point and spaced apart from the location of the spacer 30. The physician need only position the spacer 30 within the leaflets, crimp the catheter shaft 24 onto the anchor rail 22, and then sever both the catheter and rail above or proximal to the crimp point.

A proximal length of the repair catheter 20 including the locking collet 32 exits the subclavian vein SV through a sealed puncture and remains implanted subcutaneously; preferably coiling upon itself as shown. In the procedure, the physician first ensures proper positioning of the spacer 30 within the tricuspid valve TV, locks the repair catheter 20 with respect to the anchor rail 22 by actuating the locking collet 32, and then severs that portion of the repair catheter shaft 24 that extends proximally from the locking collet. The collet 32 and/or coiled portion of the repair catheter shaft 24 may be sutured or otherwise anchored in place to subcutaneous tissues outside the subclavian vein SV. It is also worth noting that because the repair catheter 20 initially slides with respect to the anchor rail 22, it may be completely removed to withdraw the spacer 30 and abort the procedure during implantation. The implant configuration is like that practiced when securing a pacemaker with an electrode in the right atrium muscle tissue and the leads extending to the associated pulse generator placed outside the subclavian vein. Indeed, the procedure may be performed in conjunction with the implant of a pacing lead.

Various solutions for anchoring a delivery catheter or flexible rail to securely position a coaptation member between atrioventricular leaflets are disclosed which may be combined in a variety of ways. For instance, for the system of the prior art described above, the spacer 30 on the repair catheter shaft 24 may be anchored to the anchor rail 22 which is secured to tissue at both ends. However, one or both of the proximal and distal anchors may be removed and replaced with a different anchor. Likewise, the two anchors for the anchor rail 22 may be supplemented by a device to directly anchor the spacer 30 or repair catheter shaft 24 to tissue. In short, the present application contemplates the combination of any two or more of the various anchoring solutions, except for those that would be mutually exclusive.

Figures 2A and 2B are partial sectional views of a prior art tissue anchor 26 used to secure and anchor rail 22 of a regurgitation reduction system. The tissue anchor 26 features a plurality of anchoring prongs 38 extending distally from a common hub 40 arranged to slide longitudinally within a generally tubular sleeve 45. In use, the anchor rail 22 and tissue anchor 26 are advanced until a distal end 46 of the tubular sleeve 45 contacts tissue, at which point the rail 22 and anchor 32 are advanced longitudinally. Sharp ends of the anchoring prongs 38 are first directed axially into the tissue, and then by virtue of their resiliency and preset shape, tend to bend outwards as shown in Figure 2B. Ultimately, the array of outwardly bent prongs 38 remain embedded within the tissue, and the tubular sleeve 45 may be removed. The tissue anchor 26 shown in Figures 2A and 2B is disclosed in U.S. Patent No. 8,932,348, previously incorporated herein by reference.

### Pressure Regulation

Figures 3A-3C are longitudinal sectional views through a prior art tissue anchoring system 50 that regulates a deployment force in several stages of operation, as disclosed in U.S. Patent Provisional No. 16/107,718, filed August 21, 2018, the entire disclosure of which is expressly incorporated herein by reference. A tissue anchor 52 having forward prongs is attached to a distal end of a guide rail 54 and arranged to slide within a tubular sleeve 56. The sleeve 56 has a distal ring 58 with good visibility *in vivo* (e.g., under fluoroscopy or ultrasound) that contacts the tissue into which the anchor will be deployed. Forward advancement of a driver 60 displaces an external sleeve 62 and a secondary sleeve 64 forward against the bias of an internal spring 66. The secondary sleeve 64 also has good visibility *in vivo,* and eventually is advanced into proximity with the distal ring 58 of the sleeve 56, as seen in Figure 3B. This union of the secondary sleeve 64 and distal ring 58 can easily be seen from an external viewer, indicating that a predetermined force has been set up between the sleeve 56 and tissue. At this point, the user advances the guide rail 54 through the sleeve 56 so that the prongs of the tissue anchor 52 embed into the tissue. By first establishing a predetermined spring force of the sleeve 56 against the tissue, the tissue anchor 52 is more reliably secured to the tissue. It should be understood that the force regulation provided by the tissue anchoring system 50 of Figures 3A-3C may be combined with any of the succeeding tissue anchors described herein, as long as practicable.

### Radial Prongs

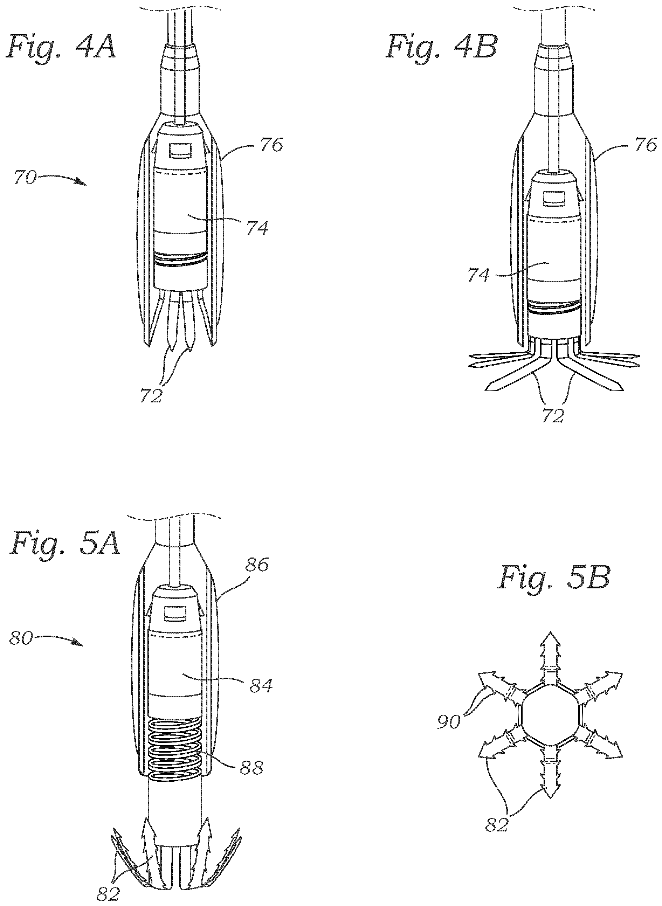

Figures 4A and 4B are partial sectional views of a first embodiment of a tissue anchor 70 of the present application prior to and after deployment of anchoring prongs. The tissue anchor 70 is configured much the same as the tissue anchor 26 described above with respect to Figures 2A and 2B. As such, the anchor 70 has a plurality of sharp, distal prongs 72 attached to a common hub 74 which reciprocates axially within a sleeve 76. The prongs 72 have an outward bias that are held generally in a linear configuration within the sleeve 76, as seen in Figure 4A. In contrast to the generally rearwardly curved prongs of the earlier embodiment, the prongs 72 are preshaped to bend approximately 90° directly radially outward when expelled from the sleeve 76, as seen in Figure 4B. This configuration is believed to better anchor the prongs 72 in the tissue because the sharp ends do not tend to pierce back out of the tissue in which the anchor is embedded.

### Barbed Prongs

Figure 5A is a partial sectional view of another exemplary tissue anchor 80 with a plurality of serrated anchoring prongs 82 in a deployed configuration. The prongs 82 are mounted to a common hub 84 which again slides within a generally tubular sleeve 86. A coil spring 88 may be provided as part of the hub 84 to enable the force regulation apposition as described above with respect to Figures 3A-3C. When expelled from the distal end of the sleeve 86, the prongs 82 tend to curl backwards as shown in Figure 5A, or may be preshaped to assume a 90° bend as seen in the embodiment of Figure 4B. Figure 5B is an end view of the tissue anchor showing the anchoring prongs 82 in profile. Each prong 82 is serrated and has a series of small side teeth or barbs 90 arrayed along their length from the hub 84 to an outer tip. The barbs 90 may be configured in a variety of different ways and are illustrated in the exemplary view with a conventional arrowhead shape. The number of barbs 90 on each side of each prong 82 may vary, and partly depends on the length of the prong 82 that extends outward from the hub 84. In one embodiment, the prongs 82 have a length of approximately 1 cm and there are four small barbs 90 on both sides along the length of each prong.

### Flexi-Anchor

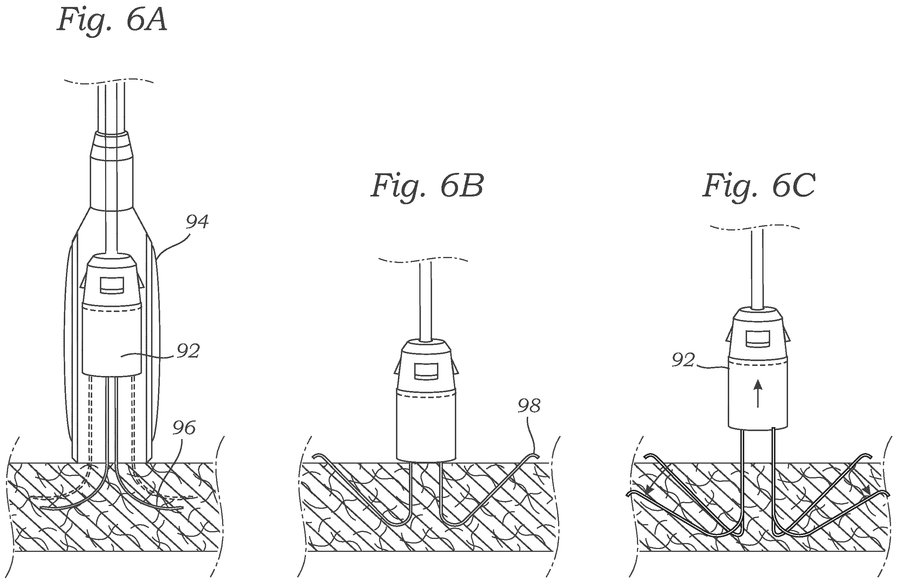

Figures 6A-6C illustrate deployment into tissue of a still further exemplary tissue anchor 92 which is carried within and expelled from a surrounding generally tubular sheath 94. The tissue anchor 92 has a plurality of filament-like highly flexible prongs 96 that, when expelled from the sheath 94, deflect outward. By placing or pressing the distal end of the sheath 94 against tissue and advancing the tissue anchor 92 forward, the prongs 96 embed themselves into the tissue and gradually splay outward and invert back out of the tissue, as seen in Figure 6B. Each prong 96 has a small bent tip 98 that points outward once the prong is bent backward toward the central hub and facilitates piercing into the tissue in a secondary locking step. That is, after the prongs 96 are fully embedded in the tissue, and the tips 98 emerge backward from the tissue, the tissue anchor 92 may be retracted slightly as seen in Figure 6C which places the prongs 96 in tension. Pulling back on the prongs 96 in this manner causes the outer or free ends thereof to pivot toward and into the tissue by means of a fulcrum effect. In conjunction with the filament-like nature of the prongs, this device placement is both atraumatic and secure, and minimizes tissue trauma during deployment. The term filament-like means that the prongs 96 have a relatively small cross-sectional dimension, circular, square, rectangular or otherwise, with a small dimension of between about 0.1-2.0 mm. The prongs 96 are desirably formed of a super elastic material such as Nitinol. There may be four, six, eight or even more prongs.

### Locking Anchors

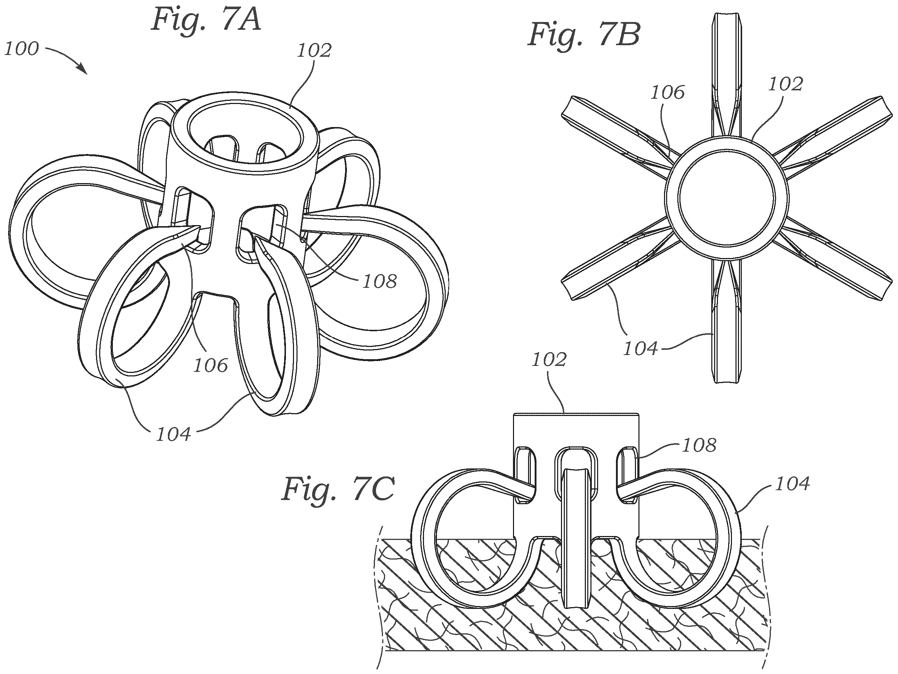

Figures 7A-7C are several views of an alternative tissue anchor 100 having a central tubular hub 102 and a plurality of evenly splayed prongs extending from one end thereof that curl 360° outward in their relaxed configuration when unconstrained to form closed shapes. More particularly, each of the prongs 104 terminates in a distal tip 106 that extends into one of a plurality of apertures 108 formed in the tubular hub 102. Instead of apertures 108, small recesses that do not pass entirely through the wall of the hub 102 may be substituted.

Preferably, the distal tips 106 are sharpened so as to easily pass into tissue and then revert outward into their relaxed curled shapes, as seen in Figure 7C. In the illustrated embodiment, there are six prongs 104 evenly spaced around the hub 102 at 60° intervals, though there may be as few as three and as many as twelve. The hub 102 and prongs 104 are desirably formed of a monolithic, homogeneous, highly flexible material such as Nitinol. More specifically, the tissue anchor 100 may be formed by laser cutting a blank from a tube and then heat setting the prongs 104 in their curled configurations. Each of the prongs 104 can then be temporarily bent outward and straightened so that the tissue anchor 100 may be loaded into a surrounding delivery sheath, much like the earlier tissue anchors described above. Although not shown, the hub 102 is easily mounted on the distal end of a delivery shaft or rail for passage through a surrounding catheter to the deployment site.

Enabling the prongs 104 to curl back on the hub 102 and into the apertures 108 forms a plurality of closed circles, as seen in Figure 7C. This closed configuration of prongs tends to better anchor in the trabeculae carneae, which are rounded or irregular muscular columns projecting from the inner surface of the right ventricle of the heart. Consequently, even if the prongs 104 do not embed themselves deeply within the myocardial wall, they will inevitably capture some of the trabeculae carneae within the closed loops thus formed.

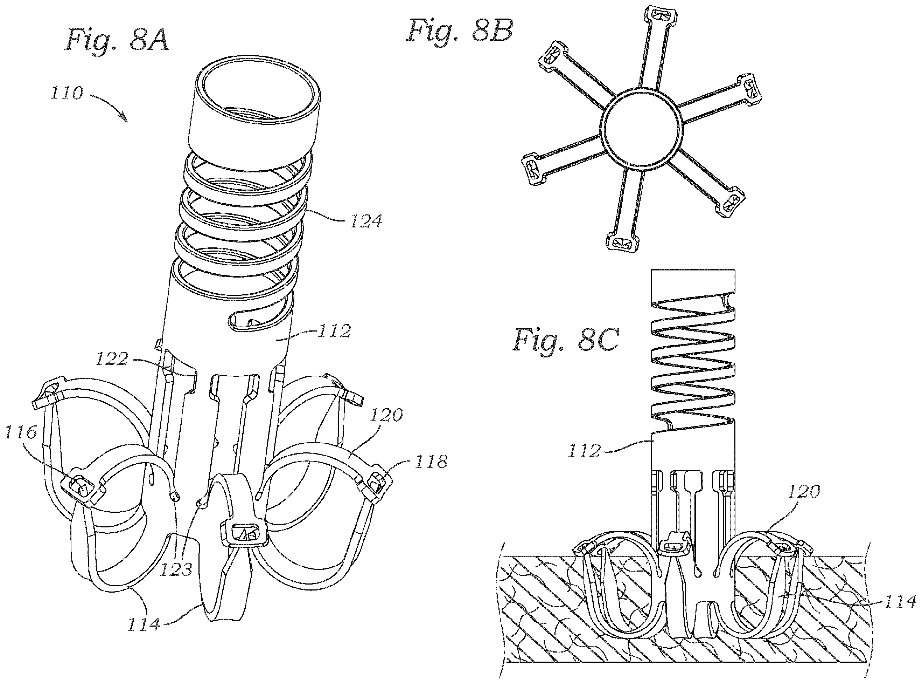

Figures 8A-8C are perspective, plan, and operational views of a still further tissue anchor 110 having locking prongs. The tissue anchor 110 again includes a tubular hub 112 with a plurality of prongs 114 projecting from a distal end thereof. Again, there may be six evenly spaced prongs 114 as illustrated, or more or less (e.g., 3-12). The prongs 114 have a relaxed configuration which forms an approximately 180° curl so that sharpened distal tips 116 extend in a proximal direction when unconstrained. Each of the prong tips 116 extend into an aperture 118 of an enlarged head 119 formed in an outwardly-curled finger 120 corresponding to that prong 114. That is, there are the same number of prongs 114 as fingers 120, and a plurality of meeting pairs of prongs in figures evenly spaced around the hub 112. The fingers 120 also have an outwardly-curled relaxed shape when unconstrained, albeit curled in the opposite direction than the prongs. In alternative configuration, the fingers 120 do not have the apertures 118 but instead are solid, the prongs 114 and the fingers 120 simply curling in opposite directions into contact with each other to form a closed loop.

In a preferred embodiment, the entire tissue anchor 110 is formed of a monolithic, homogeneous, highly flexible material such as Nitinol. Desirably, the tissue anchor 110 is created by laser cutting the various elements from a single tubular blank of Nitinol. The prongs 114 are initially aligned with the tubular hub 112 and extend in a distal direction. Heat setting enables the prongs 114 to form their relaxed, curled configurations. Likewise, the fingers 120 are initially cut from a side wall of the tubular blank and heat set into their outwardly-curled shapes. Each finger 120 preferably includes an elongated rectangular portion and the enlarged generally rectangular head 119, whose negative is seen in the enlarged cutout portion 122. The fingers 120 are cantilevered from the tubular sidewall of the hub 112 and may be provided with a pair of stress-relieving circular cutouts 123 at their junctions with the hub.

The tubular hub 112 is somewhat elongated, as compared to the hub 102 described above, and has a helical section 124. The helical section 124 may function as a spring, enabling regulation of the force applied to the tissue when deploying the anchor 110, again as described above with reference to Figures 3A-3C.

In use, the tissue anchor 110 mounts on a distal end of a shaft or rail and is advanced to a target location, such as a location within the right ventricle. Also, as before, the tissue anchor 110 may be housed within a surrounding tubular sheath which constrains both the prongs 114 and fingers 120 in axial alignment coincident with the tubular hub 112. Once the distal end of the tubular sheath contacts or is pressed against the target location, the user advances the tissue anchor 110 such that the sharp prongs 114 pierce the tissue. Eventually, the tissue anchor 110 has been fully expelled from the surrounding delivery sheath such that the prongs 114 curled backward upon themselves and the fingers 120 are released to curl outward. The sharp tips 116 of the prongs 114 are received within the apertures 118 of the fingers 120, thus closing a plurality of tissue anchor loops. As with the earlier embodiment of Figures 7A-7C, the closed prong loops tend to better anchor in the trabeculae carneae. Consequently, even if all the prongs 114 do not embed themselves into the myocardial wall, some or all will, along with the fingers 120, capture some of the trabeculae carneae within the closed loops thus formed.

### Claw Anchor

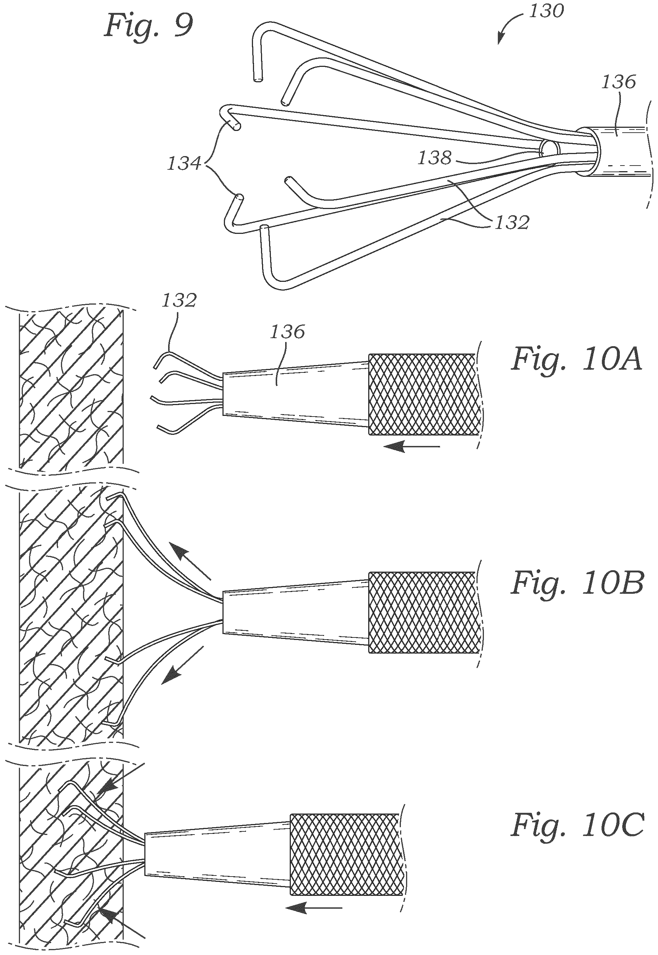

Figure 9 is a perspective view of a distal end of another tissue anchor 130 of the present application having a plurality of wire-like anchor prongs 132. The prongs 132 are evenly arrayed around the central axis and have distal tips 134 that are bent radially inward, such as by 90°. The prongs 132 are initially housed within and can be projected from a tubular sheath 136. For example, a concentric inner tube 138 may be provided which contacts inner surfaces of each of the prongs 132 and causes them to translate axially back and forth within the sheath 136. Each of the prongs 132 has a relaxed shape which causes them to splay outward once they are expelled from within the surrounding sheath 136, as seen in Figure 9. This assembly is similar to a standard grabbing device for picking up small items.

Figures 10A-10C show several steps in deployment of the tissue anchor 130 of Figure 9. Initially, the prongs are constrained by the outer sheath 136 which is advanced to the target location. Prior to contacting the tissue, the prongs 132 are expelled to a certain degree from the sheath 136 such that the bent tips 134 are oriented toward the tissue, as seen in Figure 10B. The prongs are then advanced to make contact with the tissue, and then by advancing the sheath over the prongs they are forced to move towards each other, thereby grabbing or biting into that tissue, as seen in Figure 10C. This tissue anchor 130 also anchors well in trabeculae carneae.

### Corkscrew Anchors

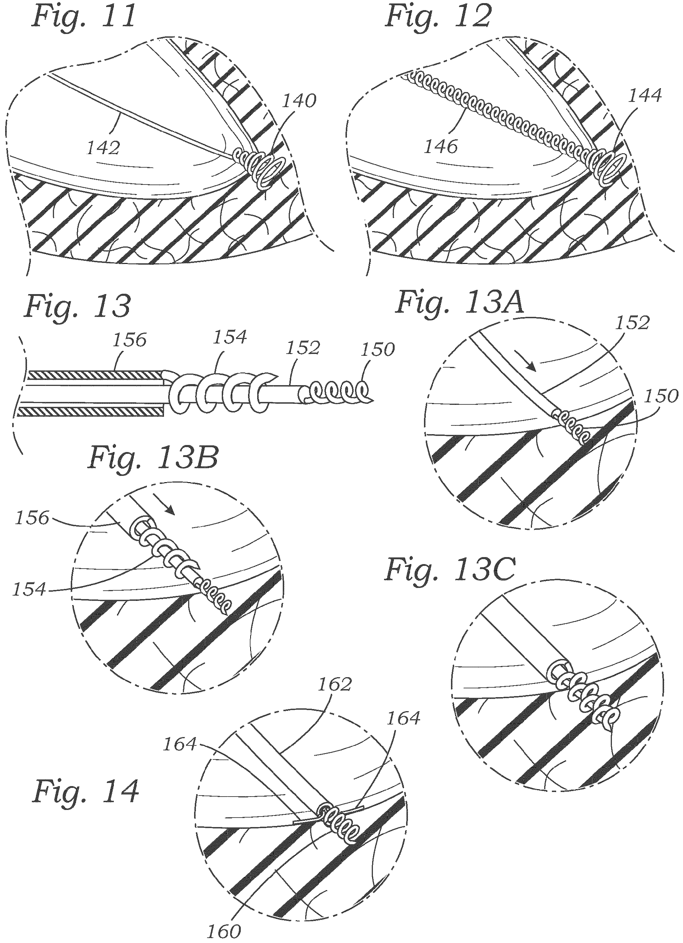

Figures 11 and 12 are views of alternative anchoring members utilizing conical corkscrew-like or coil spring tissue anchors. Figure 11 shows a modified helical anchor 140 with a conical shape, i.e. a circular cross-section of increasing size towards the distal end. The conical spring anchor 140 could be provide at the end of an anchor rail 142, as previously described. Such an anchor design could increase retention force by increasing the cross-sectional area of contact between the anchor coil and the tissue. Additionally, as the initial cut of the anchor 140 into the tissue would be largest followed by decreasing coil diameter as the anchor is screwed in, the anchor could effectively "cinch" in a volume of tissue into a compacted space. Such a feature could potentially minimize the risk for anchor tear-out by increasing the local tissue density at the anchor site. The conical spring 140 could be comprised of any shape memory material capable of collapsing or wrapping down to a smaller constant diameter to fit through a catheter lumen, then capable of expanding to the natural conical shape upon exiting the delivery sheath into the RV.

Alternatively, a conical anchor 144 as in Figure 12 could be connected via an elongated helical section 146 at its proximal end designed to remain in the RV (not screwed into the tissue but directly next to it). The elongated helical section 146 provides shock absorption capabilities against compressive/tensile stresses, thus reducing tear-away stresses on the RV apex, and also flexibility capabilities under bending stresses.

Using helical structures for anchoring the devices described herein in the right ventricle holds a number of advantages (e.g. ease of delivery, acute removability, minimal tissue damage, etc.). However, one potential challenge could be the tendency of a helical structure to "unscrew" itself out of the tissue, either acutely or over time due to the contractile motions of the ventricle. To address this issue, an anchor system in Figure 13 includes concentric corkscrew anchors; an inner anchor 150 at the end of an inner tube 152, and an outer anchor 154 on the end of an outer tube 156. Figures 13A-13C illustrate steps in installation of the anchoring device, in which first the inner anchor 150 having a clockwise orientation is screwed into the tissue. Next, the slightly larger second anchor 154, having a counterclockwise orientation, and its tube 156 slide over the first anchor 150 and tube 152 and screws into the tissue in the opposite direction. Finally, the two anchors could be fixed together with a locking mechanism (e.g., pin-through-hole style). The resulting structure seen in Figure 13C would resist unscrewing out of the tissue, since each helical coil opposes the twisting motion of the other.

Figure 14 shows another configuration with a helical corkscrew-type anchor 160 on the end of a tube 162, and a pair of struts 164 that may be independently expelled from the distal end of the tube into contact with the tissue surrounding the anchor. Rather than screwing in a second relatively similar anchor in the opposite direction to prevent twist-out, the struts 164 pass through the tube lumen and extend outwards in an L-shaped manner to provide an anti-rotation anchor to the device. These struts 164 should be thick enough to press against the RV apex tissue and apply friction thereto to prevent twisting motion of the anchor 160. Alternatively, the struts 164 may be angled so as to slightly embed within the tissue and further prevent the anchor 160 from rotating back out of the tissue.

### Anchor Placement

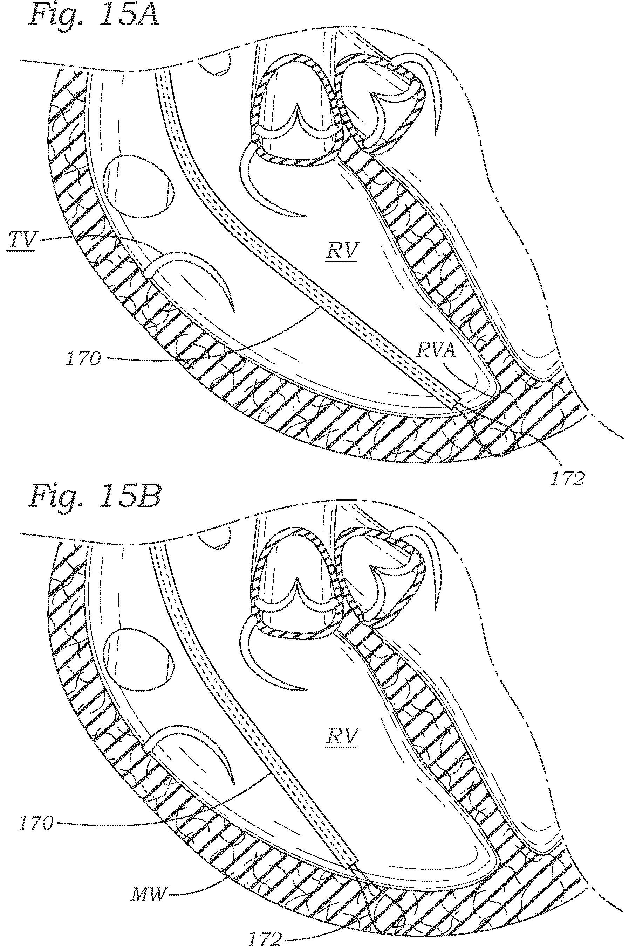

Figures 15A and 15B are sectional views of the right side of a human heart showing two different placements of a simple suture loop that may be used to anchor a regurgitation reduction system. A catheter 170 or other such access tube is used to deploy the anchor suture 172, using a number of techniques some of which are shown below. Figure 15A shows a distal end of the catheter 170 positioned adjacent an inner wall of the right ventricle RV close to the right ventricular apex RVA. This position is believed to be desirable for placement of a regurgitation reduction system spacer within the tricuspid valve TV. Alternatively, Figure 15B shows the catheter 170 positioned to place the anchor suture 172 in the myocardial wall MW a short distance from the right ventricular apex RVA. For example, the anchor suture 172 may be positioned within 50 cm of the right ventricular apex RVA. Depending on the anatomical impediments, spacer configuration, and surgeon preference, among other factors, anchoring the regurgitation reduction system in either of these two locations or ones in between or nearby may be chosen. It should be understood that these exemplary anchor placements may be used for any of the anchors disclosed herein.

### Suture Loop Deployment

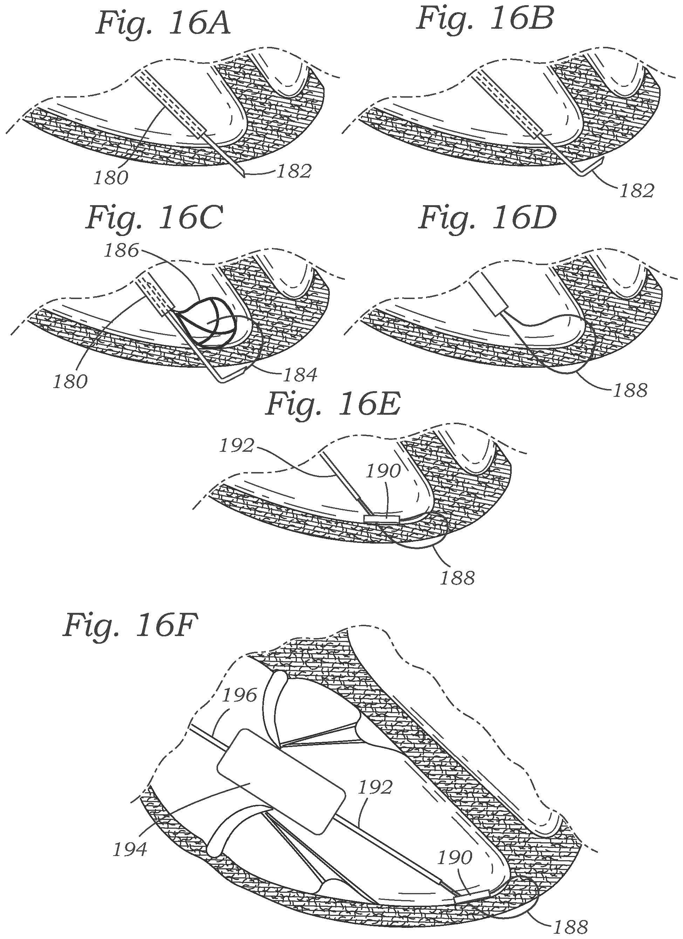

Figures 16A-16F show a sequence of steps of deploying a suture loop through tissue for anchoring a regurgitation reduction system. Initially, a deployment catheter 180 delivers a hollow puncture needle 182 to the target location within the right ventricle, such as either of the two locations described above with reference to Figures 15A and 15B. The user advances the needle 182 until it passes through the heart wall. The needle 182 is configured to curl or bend adjacent its distal tip, as seen in Figure 16B. Subsequently, two things happen, as seen in Figure 16C. First, a length of suture 184 is expelled from within the bent needle 182 and caused to pierce back through the heart wall into the interior of the right ventricle. For example, the length of suture 184 may have a curved needle (not shown) secured to a distal end which facilitates passage through the heart wall. Secondly, the user advances a snare 186 from within the delivery catheter 180 to engage with the suture 184. Various ways of capturing and retracting a length of suture 184 are known in the art and will not be further described herein. In the illustrated embodiment, the snare 186 as a net-like configuration that expands once it is expelled from the delivery catheter 18. Ultimately, the user pulls the snare 186 and suture 184 back into the delivery catheter 180 and secures the suture at a proximal end of the catheter, after which the puncture needle 182 is retracted. This leaves a suture loop 188 through the heart wall, as seen in Figure 16D, and two strands extending proximally out of the body.

Subsequently, as seen in Figure 16E, a locking element 190 such as a clip or other suitable device is deployed either through the delivery catheter 180 or separately along the two strands of the suture loop 188. The locking element 190 is positioned adjacent the inner wall of the right ventricle and holds the two strands of the suture loop 188 together at the target anchor location. A system anchor rail 192 is advanced along the two strands of the suture loop 188 into proximity with the locking element 190. Finally, Figure 16F shows a regurgitation reduction spacer 194 positioned within the leaflets of the tricuspid valve. The spacer 194 may be advanced with or along the anchor rail 192 and may have a proximal sheath 196 extending therefrom which is anchored above the tricuspid valve, as explained above.

### Anchoring Knot

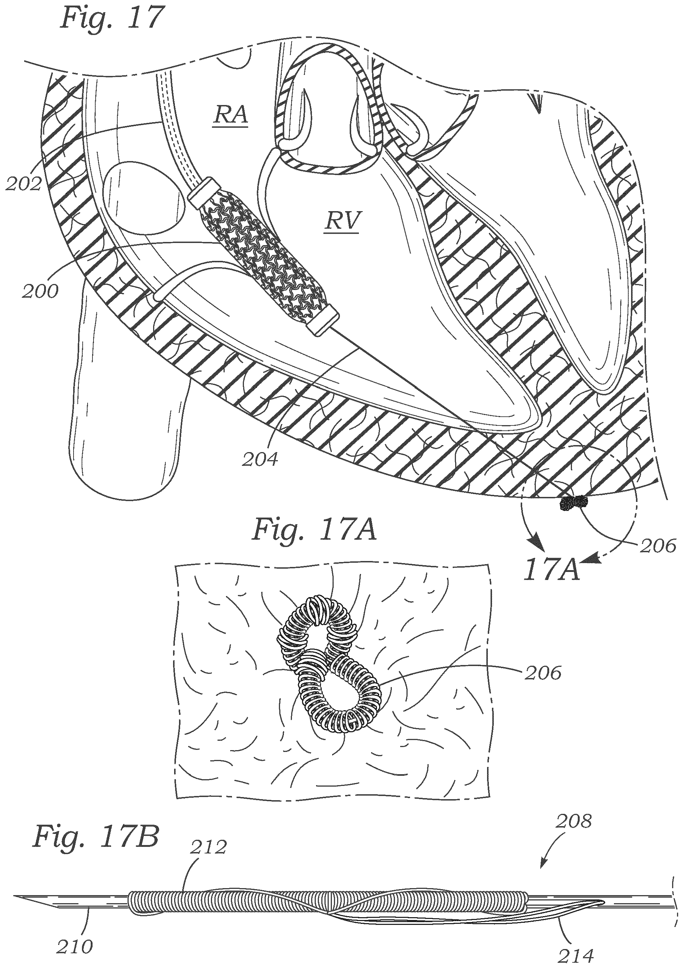

Figure 17 is a sectional view of the right side of the human heart showing a spacer 200 for a regurgitation reduction system anchored in the right ventricle, with a proximal sheath or shaft 202 connected thereto and extending proximally out of the right atrium RA. The spacer 200 may be anchored using an anchoring suture 204 and a compound knot 206 positioned exterior to the right ventricle RV. In this embodiment, the spacer 200 is adjustable in size. Specifically, the spacer 200 has an elongated generally tubular body formed of a latticework of struts arranged to be auxetic, or have a negative Poisson's ratio. An exemplary spacer configured in this manner is disclosed in U.S. Patent Provisional No. 62/675,914 (ADV-8656), the disclosure of which has previously been incorporated herein.

Figure 17A is a detailed view of the compound knot 206 which is formed by a pair of coiled lengths of suture defining a figurative "figure 8." The compound knot 206 may be deployed using a tool 208 shown in Figure 17B. The tool 208 includes an elongated rigid body 210 having a sharp distal point that resembles a harpoon. A coiled length of suture 212 wraps around the rigid body 210 and two free ends 214 thereof extend through a side aperture and into a lumen of the rigid body, passing to a proximal end thereof. The compound knot 206 is formed on the exterior of the heart wall as shown by delivering the tool 208 into the right ventricle RV and piercing the heart wall from the inside to the outside. Once the entire coiled length of suture 212 is located to the exterior of the heart wall, it is released to form the compound knot 206 followed by retraction of the rigid body 210 into the right ventricle RV. Because the two free ends 214 remain within the rigid body 210, they may be retracted fully from the body and used to anchor to the spacer 200. An example of such a deployment tool 208 is seen in U.S. Patent Publication No. 2018-0289480, the disclosure of which is hereby expressly incorporated herein.

### Expandable Disc Anchor

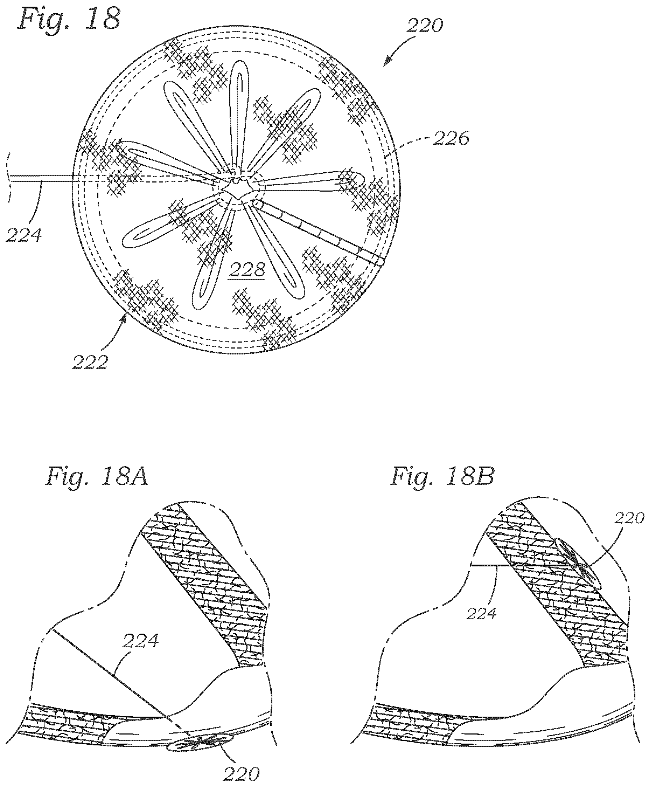

Figure 18 is a plan view of another deployed tissue anchor 220 having an expanded flat disk 222 connected to a tether 224. The disk 222 comprises a fabric cover 228 having an internal support ring 226 arranged in a circle around a periphery of the disk 222. Figure 18A illustrates the disk-shaped anchor 220 deployed on the exterior of the right ventricle of the heart in use as an anchor for a regurgitation reduction system, wherein the tether 224 extends to a spacer positioned within the tricuspid valve. This anchor could also be placed across the intraventricular septum or septal wall SW, such that the anchor is deployed in the LV and pulled against the LV side of the septum to anchor the spacer within the tricuspid valve. For instance, Figure 18A shows the disk-shaped anchor 220 deployed through the inter-ventricular septum. Deployment in both cases involves piercing the respective tissue wall with a needle, passing a guidewire through the wall, enlarging the puncture hole and passing a deployment tool through the tissue wall to deploy the anchor 22, as described below for the exterior heart wall.

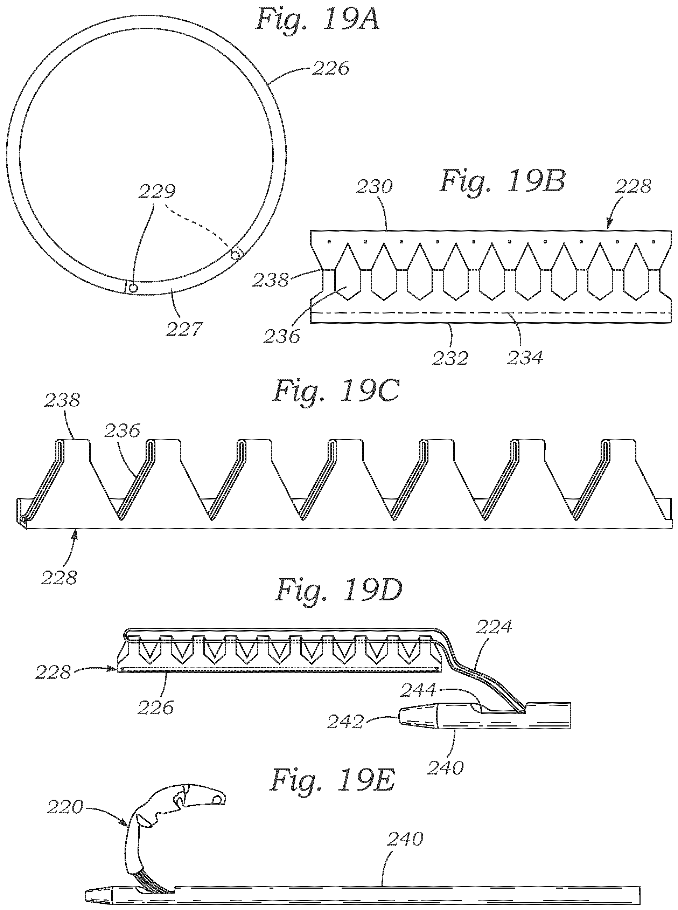

Figures 19A-19C illustrate individual components of the disk-shaped anchor 220 of Figure 18, and Figures 19D and 19E schematically illustrate how the disk-shaped anchor is loaded into and expelled from a deployment instrument. The support ring 226 preferably has a body having overlapping ends 227. The overlapping ends 227 may include through holes 229 for coupling to the cover 228, such as with sutures. The support ring 226 is desirably made of a material that is flexible, such that the ring may move from a linear configuration (see Figure 19D) to the ring-shaped configuration shown in Figure 19A. In one embodiment, the support ring 226 is made of a super elastic or shape-memory material, such as Nitinol, and is shape set to automatically move from the linear configuration to the relaxed ring-shaped configuration.

Figure 19B illustrates a laid flat view of the cover 228 unfolded, showing a top edge 230 and an opposite bottom edge 232. The bottom edge 232 includes a folded-up portion that is secured using a line of stitches 234 to form a linear through pocket. The cover 228 includes a plurality of cut-outs 236 repeated along the length of the cover each having an asymmetric diamond shape. A length of the cover 228 in its laid flat configuration is preferably between about 70-100 mm. The cut-outs 236 are shaped to leave narrow bridge portions 238 between the top and bottom edges 230, 232. The bridge portions 238 are configured to fold, as indicated by fold lines thereon. The cover 228 is desirably a woven or non-woven fabric that may include materials such as ultra-high-molecular-weight polyethylene (UHMwPE) (*for example,* DYNEEMA^{®} fabric or laminate, Koninklijke DSM, the Netherlands) or polyethylene terephthalate (PET, *for example,* DACRON^{®} fabric, Invista, Wilmington, Delaware).

Figure 19C shows the cover 228 folded up along longitudinal lines so that the top edge 230 meets the bottom edge 232 in a precursor to the disk shape of Figure 18. The top and bottom edges 230, 232 may be secured together using stitches. The cover 228 in Figure 19C has a slightly different configuration of cut-outs 236 than shown in Figure 19B. Namely, the cut-outs 236 are symmetrical as folded upon the narrow bridge portions 238.

Figure 19D shows the cover 228 still in its precursor linear configuration with the tether 224 looped through the folded bridge portions 238 and the support ring 226 secured in a linear configuration within the pocket formed at the bottom edge 232. The two strands of the tether 224 pass into a lumen of a puncturing tool 240 having a soft-tipped tapered distal end 242. For example, the tool 240 may have a side opening 244 adjacent the distal end 242. The assembly of the linear support ring 226 and cover 228 are then retracted into the lumen of the puncturing tool through the side opening 244.

The tissue anchor 220 is thus delivered to a deployment site in a linear configuration within the tool 240, and then expelled from the side opening 244 as seen in Figure 19E. In the context of deploying the anchor 220 outside the right ventricle, the tool 240 is advanced within the ventricle and caused to traverse the heart wall so that the side opening 244 is external to the ventricle. The puncturing tool 240 slides over a pre-installed guidewire, and the soft-tipped distal end 242 dilates the myocardial wall. Ultimately, the side opening 244 extends to the outside of the heart wall, but inside the surrounding pericardium, and the tissue anchor 220 expelled.

As the tissue anchor 220 is being expelled, tension on the tether 224 cinches the folded bridge portions 238 together so that the cover 228 is drawn towards a central axis. The cover 228 may be configured to be drawn towards the central axis such that a central opening entirely closes. The resiliency of the support ring 226 facilitates this conversion. Ultimately, the cover 228 assumes the disc-shaped configuration of Figure 18, and the tether 224 extends from the cover 228 at the central axis. The tissue anchor 220 in the configuration shown in Figure 18 may have a diameter of between about 20-25 mm, although other diameters may be utilized as desired. By withdrawing the puncturing tool 240 back through the heart wall, the two strands of the tether 224 may be retracted and used to anchor to a regurgitation reduction spacer, as explained elsewhere herein.

Visualization of delivery system location while passing through the myocardium and pericardium is challenging using fluoroscopy or echo techniques. Consequently, the present application contemplates the provision of radiopaque indicators to identify delivery system location. The system deploys radiopaque indicators when passing through the myocardium and pericardium. For example, as the delivery system passes through the myocardium and enters the pericardial space, a feature large enough to visualize in fluoroscopy would deploy into the space. The same action would occur as the delivery system exits the pericardium. At this point, with positive visual indication, the anchor can be deployed. After anchor deployment, the visualization features can be retracted, and the delivery system withdrawn.

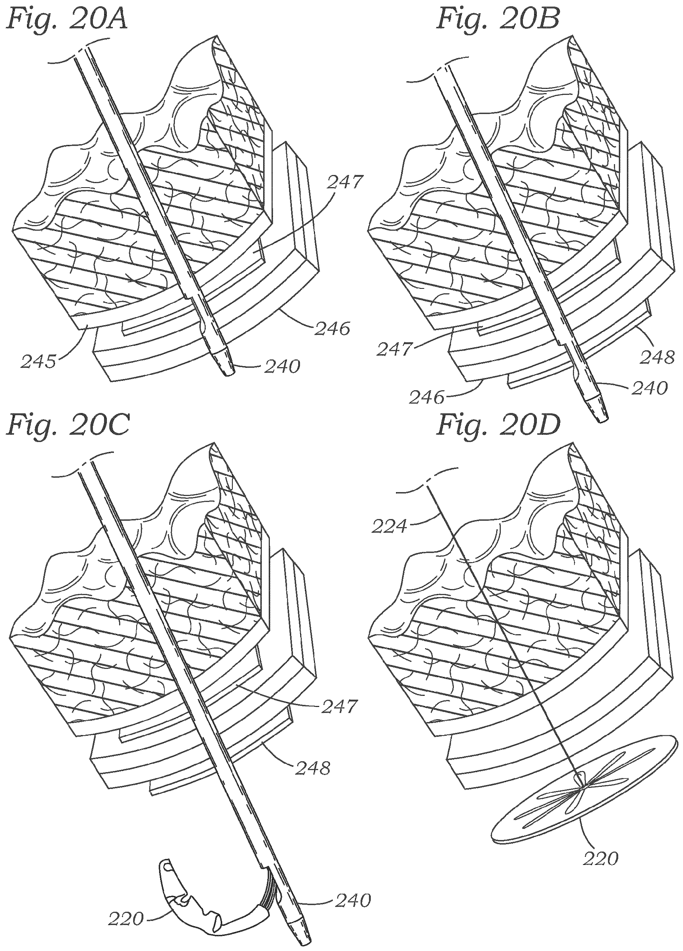

Figures 20A-20D illustrate layers 245, 246 of the heart pierced by the puncturing tool 240. Initially, as seen in Figure 20A, the puncturing tool 240 passes through the myocardial layer 245 of the heart wall and applies a radiopaque marker 247 in between the myocardial and pericardial layer 246. Figure 20B shows further advancement of the puncturing tool 240 and deployment of another radiopaque marker 248 outside the pericardial layer 246. The radiopaque markers 247, 248 may be formed as thin flexible disks which expand much like the tissue anchor 220 and are tethered (not shown) so that they may be retracted within the tool 240 after use. In Figure 20C the expandable tissue anchor 220 is seen emerging from the tool 240 outside of the pericardial layer 248. Finally, in Figure 20D the tissue anchor 220 is shown fully expanded and connected to the tether 224, which may be a suture. The radiopaque markers 247, 248 have been retracted into the tool 240.

One potential problem with placing the tissue anchor 220 though the exterior heart wall is knowing if you have exited the myocardium and the pericardial tissue, and without piercing any vital arteries in the way. The space between the pericardial layer and the ribs is also quite small, limiting the amount of space into which the anchor must deploy. The needle actuation process for successful deployment also must be performed quite quickly and positively as you are piercing through the right ventricle wall, to ensure that the heart wall does not indefinitely tent or get pinned against the inner rib space. This entire deployment process is cumbersome and stressful for the operator.

To address some of these problems with the "trans-ventricular" anchor deployment process, the alternate "transseptal" technique shown in Figure 18B was developed to place the anchor 220 through the inter-ventricular septum instead of outside the heart. The side exit design of the deployment tool 240 shown in Figures 19-20 is one proposal for a streamlined trans-septal procedure. Preferably, a catheter (not shown) is actuated to facilitate perpendicular apposition against the septal wall, after which a small puncture is made and a standard guide wire is passed through the septum. Once the guide wire is in place the deployment tool 240 is then advanced over the guide wire. The soft introducer tip 242 dilates the septal wall site as it is slowly pushed through. The deployment tool 240 also preferably has a contrast port aligned with the side window 242, which will allow the operator to see exactly when the wall is crossed. That is, contrast media flows from the contrast port and shows up under fluoroscopy on both sides of the septal wall after successful penetration. Once certain the wall is crossed, the operator can then deploy the tissue anchor 220 through the side exit 244. After the tissue anchor 220 is deployed, the operator simply withdraws the tool 240 leaving the anchor in place.

### Multiple Tethers

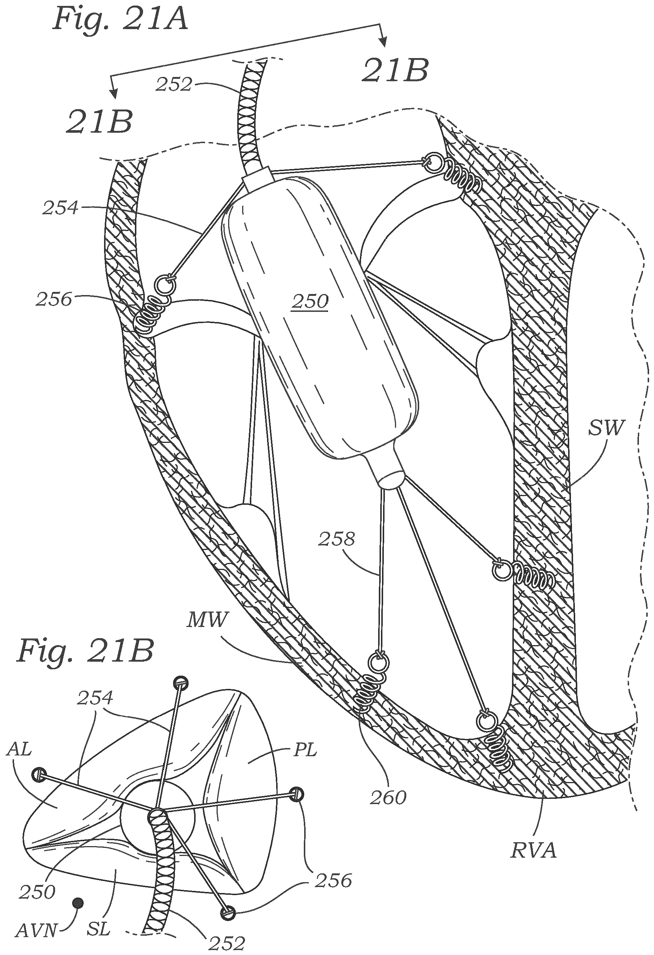

Figures 21A and 21B are partial sectional and top plan views of a regurgitation reduction spacer 250 positioned within the leaflets of a tricuspid valve and tethered to surrounding tissue using an array of both sub- and supra-annular anchors. The spacer 250 may be delivered on the distal end of a flexible shaft 252 that may also be anchored to tissue, typically subcutaneously outside the subclavian vein, as explained above. In the illustrated embodiment, there are at least two supra-annular tethers 254 connected to a proximal end of the spacer 250 and attached to annulus anchors 256. The anchors 256 may be any of the tissue anchors described herein and are illustrated as simple corkscrew-type anchors. Preferably, there are at least two of the supra-annular anchors 256 to center the spacer 250 within the tricuspid valve. As seen in Figure 21B, however, four tethers 254 and anchors 256 are shown. The anchors 256 are desirably distributed generally evenly around the tricuspid annulus, though preferably in a manner that avoids placement near to the sensitive atrioventricular node AVN. In the illustrated embodiment, there are two anchors 256 secured adjacent the anterior leaflet AL, one adjacent the posterior leaflet PL, and the fourth secured adjacent the septal leaflet SL close to the commissure between the septal and posterior leaflets.

In addition to supra-annular anchors, the tethering arrangement of Figure 21A also shows a plurality of sub-annular anchors. Specifically, there are three pairs of tethers 258 and anchors 260 connected to a distal end of the spacer 250 and attached to tissue within the right ventricle. The anchors 260 are shown attached to the mitral wall MW, adjacent the right ventricular apex RVA, and in the septal wall SW. The embodiment illustrated thus includes at least seven tether/anchor pairs, four supra-annular and three sub-annular.

Deployment of the system shown in Figure 21A preferably involves first securing the sub-annular anchors 260 and then the supra-annular anchors 256, and extending the associated tethers 254, 258 outside of the body. The tethers 254, 258 are then connected to the spacer 250, or routed through the spacer to a control handle for length adjustment, as disclosed below. Finally, the physician advances the spacer 250 along the array of tethers 254, 258 until it is properly positioned within the valve leaflets, as may be determined under fluoroscopy. Finally, after adjusting the length or slack in the tethers to produce good regurgitation reduction, the tethers 254, 258 are locked with respect to the spacer 250.

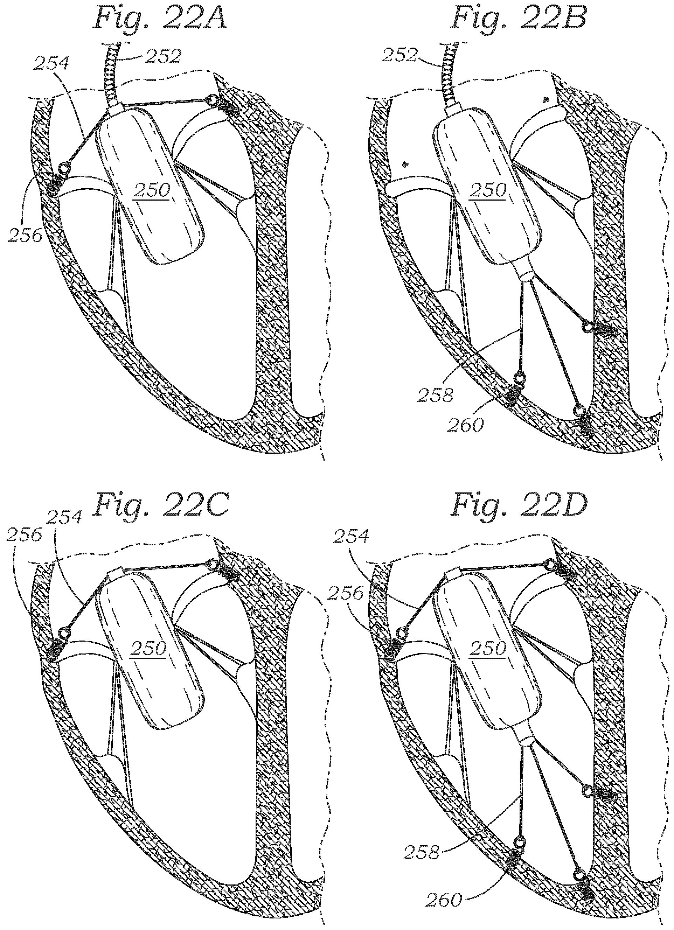

Figures 22A-22D illustrate alternative arrays of sub-annular and supra-annular tether/anchor pairs distributed around a regurgitation reduction spacer 250. Figure 22A omits the sub-annular anchors and just shows a plurality of supra-annular tethers 254 and corresponding anchors 256. Conversely, Figure 22B shows just a plurality of sub-annular tethers 258 and anchors 260. Figure 22C also has only supra-annular tethers 254 and anchors 256, and also omits the proximal shaft 252. Finally, Figure 22D shows an arrangement with both supra-annular and sub-annular tether/anchor pairs, as shown in Figure 21A, but without the proximal shaft 252. The ability to customize the placement of the tether/anchor pairs greatly expands the spacer anchoring choices.

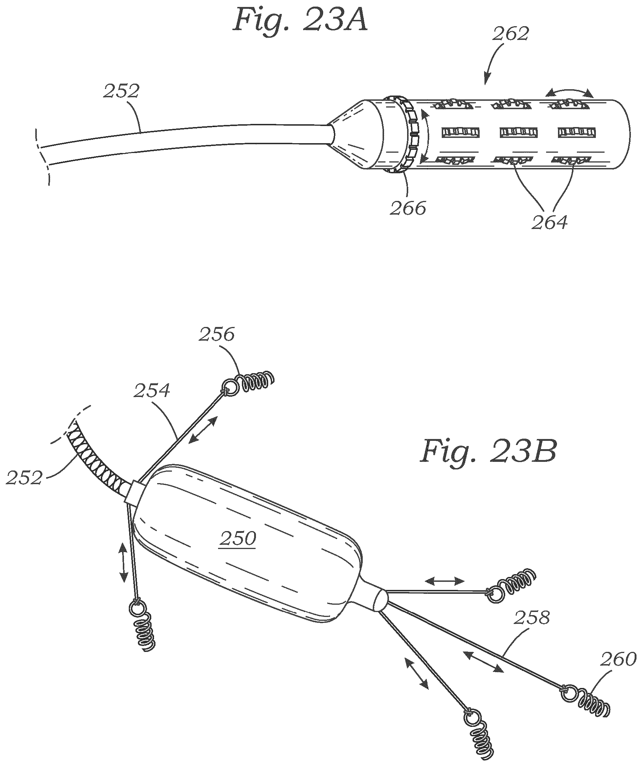

In addition to being able to choose from a variety of anchoring solutions, the present application also contemplates adjustable tethers for each of the tether/anchor pairs. For example, Figure 23A is a perspective view of an exemplary control handle 262 that enables adjustment of the lengths of the tethers using a plurality of thumb wheels 264. In addition, a control ring 266 may be provided for locking the position of the tethers 254, 258 with respect to the spacer 250 once an optimum position is attained.

Figure 23B is an exemplary anchoring array on a regurgitation reduction spacer 250 represented of any of the tether arrangement described above. By connecting each of the individual supra-annular and sub-annular tethers 254, 258 to a different thumbwheel 264, the relative lengths between the spacer 250 and the corresponding anchors 256, 260 can be adjusted. The adjustments can be made post-implant while the heart is beating and under fluoroscopy so that a real-time evaluation of the efficacy of the spacer 250 in reducing regurgitation is available. Slight adjustments to different tethers 254, 256 can then be made to improve the results.

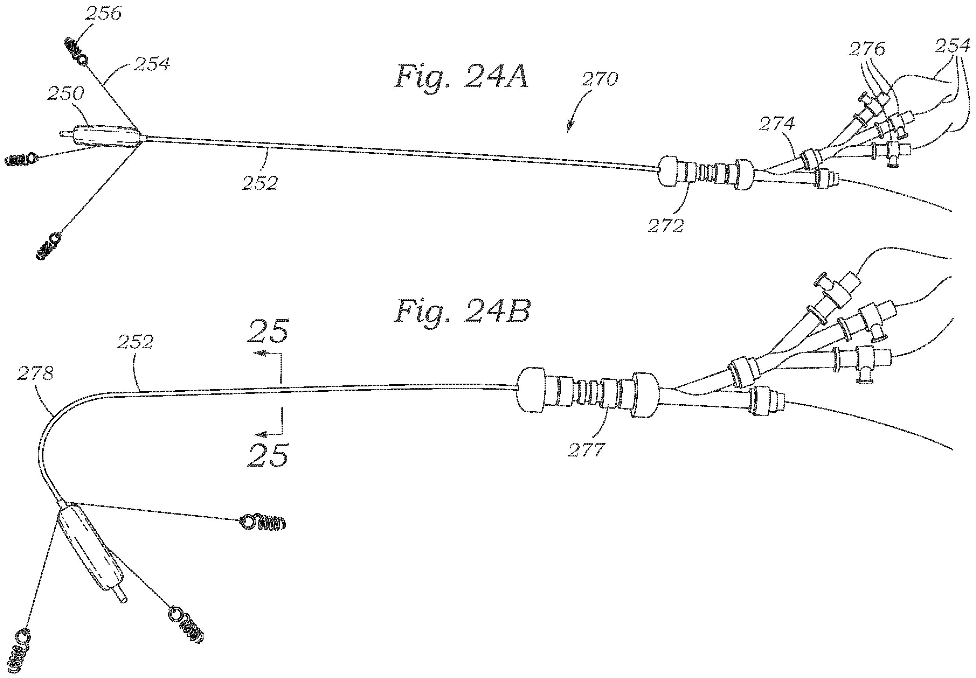

Figures 24A and 24B are perspective views of an exemplary deployment instrument 270 for a regurgitation reduction system such as described above with reference to Figures 21-22. Specifically, the system has a regurgitation reduction spacer 250 on the end of a flexible shaft 252, and a plurality of tether/anchor pairs 254, 256 connected to a proximal end of the spacer. The deployment instrument 270 includes a proximal handle 272 that typically remains outside the body and to which the shaft 252 attaches. The plurality of tethers 254 may pass approximately through the shaft 252 and handle 272 and are then routed through one branch of a Y-junction 274 and into separate access ports 276. In this manner, the length of each of the individual tethers 254 can be separately adjusted, such as on a real-time basis under fluoroscopy.

A control ring 277 on the handle 272 may be used to bend the entire flexible shaft 252 a location just proximal from the spacer 250. For example, a bend 278 may be formed by rotating the control ring 277. The ability to bend the flexible shaft 252 coupled with rotation of the entire shaft about its axis provides great flexibility in positioning the spacer 250 and orienting the tether/anchor pairs 254, 256. Although not shown, a second set of tether/anchor pairs that attach to the distal end of the spacer 252 may be provided, such as those shown above with reference to Figures 21-23. In that case, additional access ports 276 for the extra tethers may be provided.

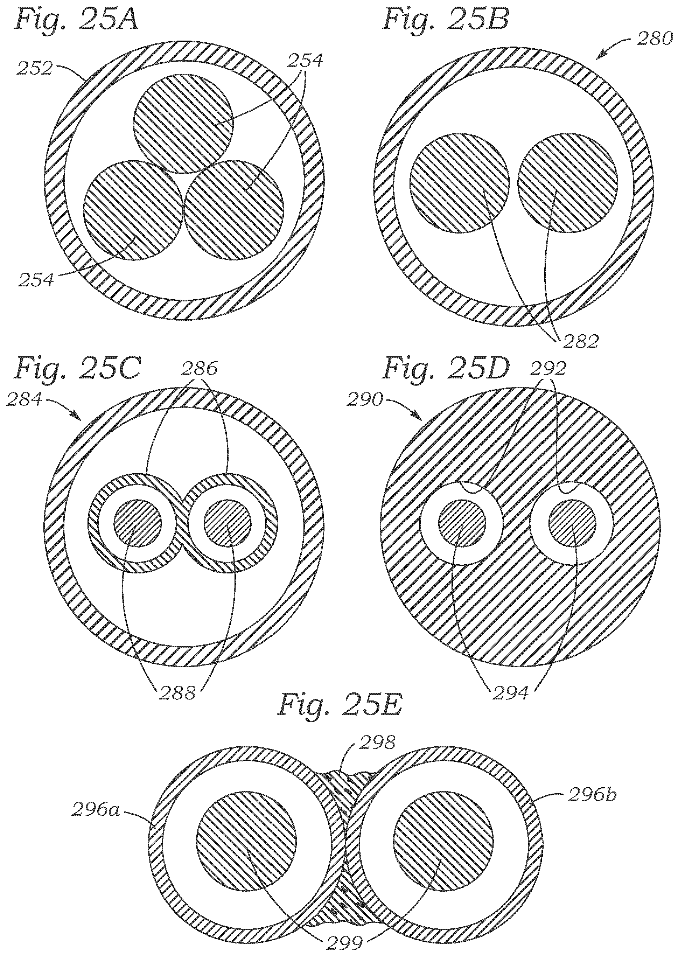

Figures 25A-25E are cross-sectional views of the flexible shaft 252 forming a part of the deployment instrument 270, taken along line 25-25 of Figure 24B. The different sectional views show alternative configurations of multiple elongated tethers passed therethrough, such as tethers 254.

In the embodiment of Figure 25A, the flexible shaft 252 is shown as tubular with a single concentric lumen and the three tethers 254 therein are cylindrical. In one embodiment, the tubular flexible shaft 252 is made of a suitable polymer, such as Polytetrafluoroethylene (PTFE), Polyurethane (PU), Polyamide (Nylon), Polyamide (Pebax), Polycarbonate-based PU, high-density Polyethylene (HDPE), or Polyether ether ketone (PEEK). Exemplary dimensions include an outer diameter of about 0.155 inches, an inner diameter of about 0.132 inches (meaning a wall thickness of about .012 inches). The diameter of each of the three tethers 254 is such that sufficient clearance remains within the shaft for movement of the tethers, both linear and radial/circumferential with respect to the shaft and each other so that the tethers are free to slide and re-orient. With such exemplary dimensions for the shaft 252, the diameter of each of the three tethers 254 is preferably between 0.04-0.06 inches, and more preferably about 0.05 inches. The tethers 254 may be formed of flexible wires, such as nitinol, or polymeric material, such as any suitable suture material.

Of course, there may be more than three tethers 254 such as in a delivery system of Figure 21A with five tethers. Desirably, the relative diameter of the tethers and shaft in each case is such that a clearance remains around a close-packed collection of tethers within the shaft, as seen in the triangular arrangement of Figure 25A. The clearance is at least 0.01 inches, and preferably between 0.01-0.02 inches.

Figure 25B is a sectional view through a shaft 280 that houses just two of the tethers 282 within a single concentric lumen, such as in the embodiment of Figure 22A. With the exemplary sizes described above this arrangement provides more free space within the shaft 280 and thus greater freedom of movement for the tethers 282.

Figure 25C shows an alternative arrangement of an outer tubular shaft 284 that contains an inner shaft 286 defining two separate lumens therein that each house a tether 288. The inner shaft 286 is shaped as a "figure 8" such that the two separate lumens are completely segregated from each other by a central bridge, though a single lumen inner shaft may also be used. In this embodiment, the spaced between the outer shaft 284 and the inner shaft 286 may be free to accommodate fluid flow, such as blood flow or saline flow for inflating devices.

Figure 25D illustrates a still further possible cross-section, where a shaft 290 is substantially solid aside from two smaller lumens 292 defined side-by-side which receive tethers 294. This arrangement adds stiffness to the assembly to enhance the ability to twist and otherwise manipulate a spacer on the end of the shaft 290 within the atrioventricular valve.

Finally, Figure 25E depicts an assembly of two separate shafts 296a, 296b bonded together at a bridge 298, such as with adhesive or welding. Each of the shafts 296a, 296b defines a lumen within which a tether 299 slides. One advantage of this arrangement is greater torsional strength to the assembly in contrast with a single tubular shaft.

### Anchoring Strip

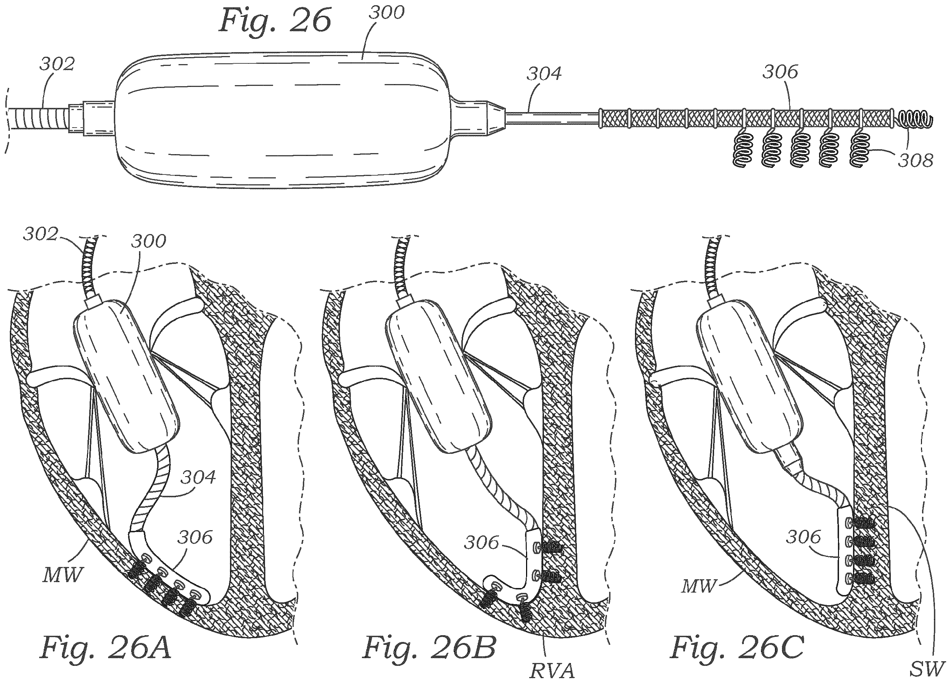

Illustrating another anchoring solution, Figure 26 is a side elevational view of a regurgitation reduction spacer 300 at the distal end of the flexible shaft 302. An anchor rail 304 extends from the distal end of the spacer 300 leading to an anchoring strip 306 capable of deploying a plurality of anchors 308 into ventricular tissue. The anchors 308 are shown as corkscrew-type anchors, but other known tissue anchors, such as those described herein may be utilized. The exemplary embodiment shows five evenly-spaced anchors 308 extending perpendicularly from the longitudinal axis of the strip 306, and a single anchor 308 extending axially from a distal end thereof. Various numbers of anchors and configurations are contemplated. Similar anchoring strips are known in the art for other therapeutic applications, such as the CardioBand Mitral Reconstruction system available from Edwards life-sciences of Irvine, California, expressly incorporated herein. The CardioBand system is essentially an annuloplasty band having a plurality of corkscrew-like anchors that are sequentially deployed as the annuloplasty band is anchored around the annulus.

Figures 26A-26C illustrate various placements of the anchoring strip 306 of Figure 26 in the right ventricle. In Figure 26A, the strip 306 is shown within the right ventricle positioned against the interior of the outer myocardial wall MW with the anchors 308 embedded therein. Figure 26B shows the strip 306 in a bent position straddling the right ventricular apex RVA with the anchors 308 embedded in adjacent tissue. Finally, Figure 26C shows the anchoring strip 306 flush against the septal wall SW, with the anchors 308 deployed therein. Again, the ability to swivel the anchoring strip 306 and deploy it in a variety of orientations and placements provides great flexibility to the surgeon. For each of these placements, the anchoring strip 306 may be manipulated by virtue of a steering mechanism such as pull cables that extend to the distal end thereof, as well as the ability to swivel the entire structure about its axis. The installation procedure typically comprises embedding a first anchor 308 closest to the distal end of the strip 306, and then sequentially embedding the rest of the anchors 308 one by one from distal to proximal.

### Annulus Anchors - Sub-Annular

Up to this point, all the anchoring solutions involve tether, rails, or other such connections between a particular regurgitation reduction spacer and remote anchors or stents. However, it may be preferable to secure the spacer directly to the annulus in a self-anchoring manner.

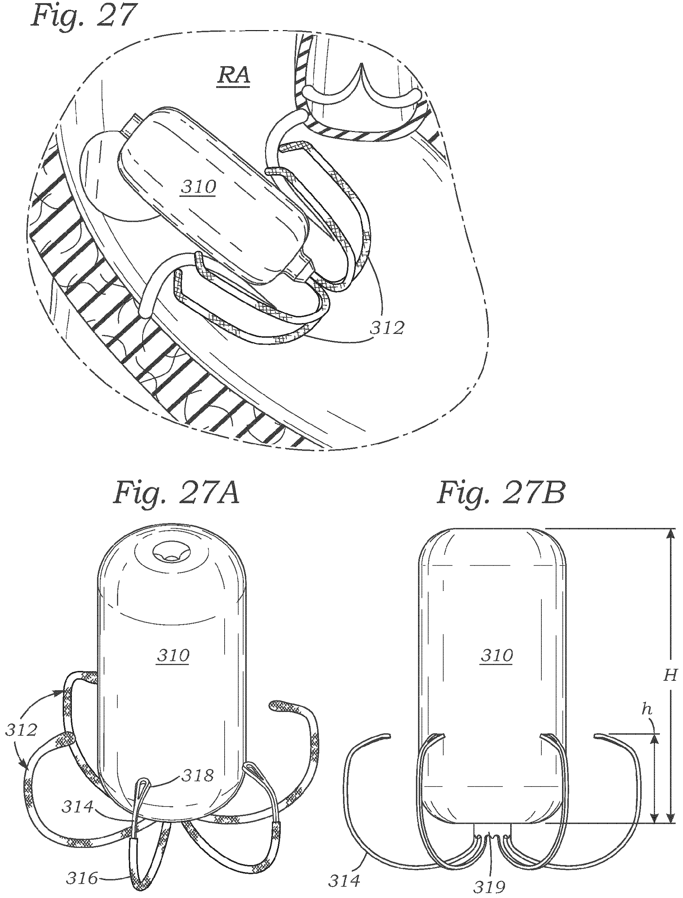

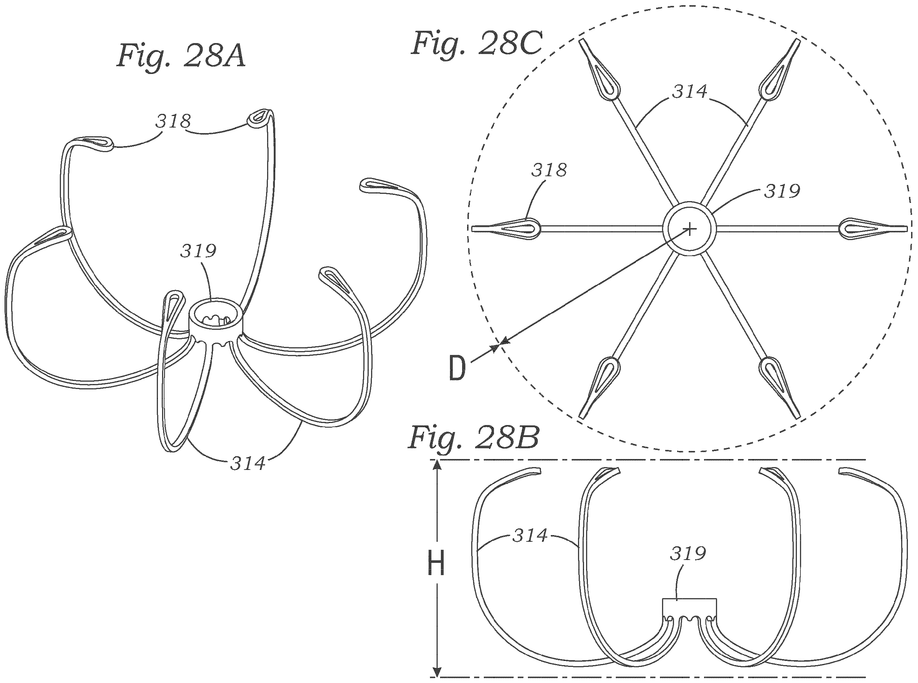

Figure 26 illustrates a regurgitation reduction spacer 310 having a sub-annular anchor comprising plurality of anchoring legs 312 which extend underneath the valve leaflets, and Figures 27A and 27B are perspective and elevation views of the spacer 310 with the attached sub-annular anchoring legs 312 extending therefrom. Figures 28A-28C are perspective and orthogonal views of one example of the sub-annular anchoring legs of Figure 27.

With reference to Figure 26, the spacer 310 resides between the leaflets of the tricuspid valve to reduce regurgitation therethrough, and is held in place by the anchoring legs 312 which extend from a distal end and curl back approximately 180° under the leaflets so as to retain the spacer in place. Although not shown, the anchoring legs 312 are in contact and entangled with the complex structure below the leaflets; mainly chordae tendineae which connect the leaflets to right ventricular structures such as papillary muscles and trabeculae carneae. In this regard, discrete tissue anchors may not be required. Preferably there are six evenly spaced anchoring legs 312 as shown, though there may be as few as three and as many as twelve.

Figure 27A shows the anchoring legs 312 in perspective emerging from the distal end of the spacer 310. Each anchoring leg is formed by an inner strut 314 enclosed in a fabric covering 316. The fabric covering 316 is partially removed in two of the legs to illustrate terminal ends of the struts 314 comprising rounded eyelets 318. Figure 27B shows the assembly of the spacer 310 and an integral structure of the uncovered struts 314 connected to a central annular hub 319. Desirably, the annular hub 319 attaches to a projection or other such structure distal end of the spacer 310 such as with adhesive or by crimping. The overall height H of the spacer 310 is shown relative to the smaller height h of the legs 312 extending up past the bottom of the spacer 310. In a preferred embodiment, the percent ratio of the heights h/H is between about 20-50%, and is more preferably between about 25-35%.

Figures 28A-28C show the integral structure of the inner struts 314 of the anchoring legs 312 connected with the central annular hub 319. This structure may be formed as a monolithic, homogeneous piece, such as by laser cutting and then heat setting a tubular blank of Nitinol. In one embodiment, the inner struts 314 are formed from a 5 mm diameter tubular Nitinol blank which is laser cut and then microblasted and electropolished for smoothness. Each of the struts 314 projects in a distal direction from the annular hub 319 and gradually curls outward until the struts generally extend in a proximal direction. Each of the terminal eyelets 318 bends radially inward, preferably by an angle of between 45-90°, and more preferably between about 60-80°. An overall diameter D circumscribed by the inner struts 314 as well as the overall height H of the struts 314 and hub 319 are shown. In a preferred embodiment, the diameter D is between about 40-75 mm, while the height H is between about 15-40 mm.

The eyelets 318 are rounded so as to be atraumatic to tissue they may come in contact with, which is also assisted by the fabric covering 316. The fabric may be polyethylene terephthalate (PET). The eyelets 318 each define a through hole which may be used to attach to anchors, as will be shown below.

Although not shown, delivery of the spacer 310 and attached anchoring legs 312 occurs by first constricting the assembly to a smaller profile, such as by enclosing the assembly within a tubular sheath. The sheath is then delivered to the native valve annulus and the anchoring legs 312 are expelled first to expand below the valve leaflets. As the sheath is removed, the spacer 310 may self-expand, or may be inflated or otherwise actively expanded.

### Annulus Anchors - Supra-Annular

One of the challenges with some regurgitation reduction systems is the necessity for a secure a ventricular anchor embedded into the heart to position and hold the entire system. As mentioned elsewhere, securing a ventricular anchor can be challenging. The present application this contemplates a number of anchoring solutions which eliminates the distal ventricular anchor and the consequent challenges in placing the anchor.

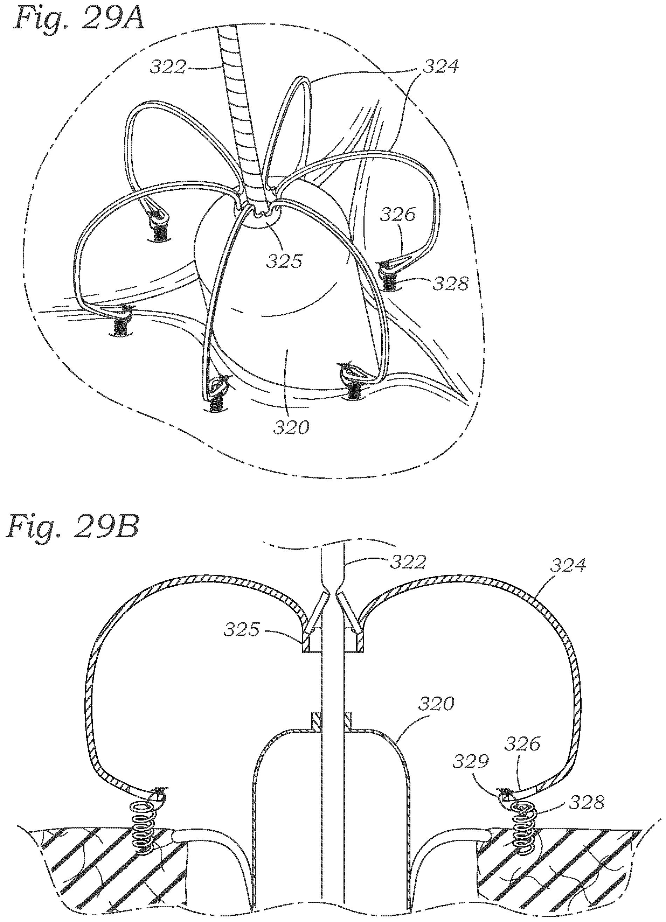

Figure 29A is a perspective view from above a native heart valve showing a regurgitation reduction spacer 320 at the distal end of a flexible shaft 322, and Figure 29B is a vertical sectional view of the same system. The spacer 320 is stabilized within the valve by a plurality of curved struts 324 attached to and extending from a proximal end thereof. More particularly, the struts 324 all emanate from an annular hub 325 attached to the proximal end of the spacer 320. The struts 324 first extend in a proximal direction and then gradually curve outward and around so as to end up extending generally in a distal direction. Each of the struts 324 terminates in an inwardly bent eyelet 326. The shape and construction of the assembly of the struts 324 and annular hub 325 may be the same as described above with respect to the anchoring struts 314 and hub 319 of Figures 28A-28C.

Each of the struts 324 is desirably anchored to the surrounding valve annulus by attachment to embedded anchors 328. In the illustrated embodiment, the anchors 328 are shown as corkscrew-type anchors, but of course they may be any variety of known tissue anchors, such as those described herein, or even simple sutures securing the eyelets 326 in place. Each of the struts 324 may be secured to a tissue anchor 328 via a suture 329 that passes through the eyelet 326 of the strut. As described above, there are preferably six evenly-distributed struts 324, though there may be as few as three and as many as twelve.

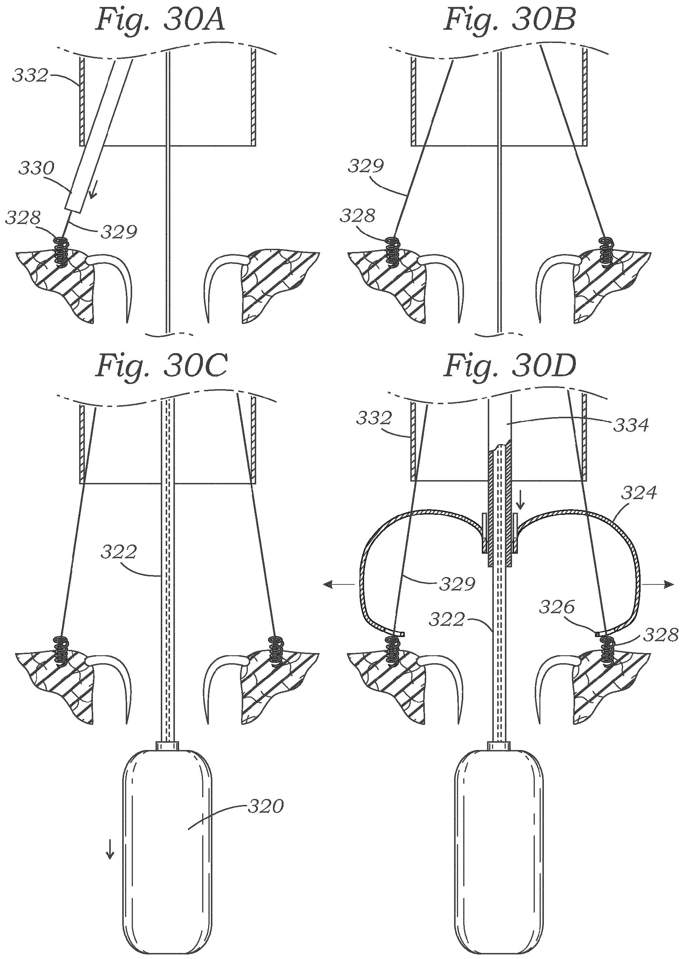

Figures 30A-30H are vertical sectional views illustrating several steps in deployment of the regurgitation reduction spacer 320 and supra-annular anchor of Figures 29A and 29B. initially, as seen in Figure 30A, a deployment catheter driver 330 is advanced distally through an access tube 332. The deployment catheter driver 330 carries with it a tissue anchor 328, preferably pre-attached to the suture 329. In the illustrated embodiment, the tissue anchor 328 is a corkscrew-type and the catheter driver 330 simply screws it into the tissue, though other anchors and corresponding drivers are known. A single deployment catheter driver 330 may be used to deliver and secure each of the tissue anchor 328, or separate deployment catheters may be utilized, but the end result is seen in Figure 30B with a plurality of anchors 328 distributed around the annulus each having a suture 329 attached thereto and extending proximally through the access tube 332.

Subsequently, as seen in Figure 30C, the spacer 320 is advanced at the distal end of the flexible shaft 322 through the access tube 332. The user advances the spacer 320 past the native valve leaflets into the ventricular cavity.

Figure 30D is a snapshot just after advancement of the anchoring struts 324 past the distal end of the access tube 332. The anchoring struts 324 and central hub 325 are mounted for delivery on the distal end of a shaft 334 which slides over the flexible shaft 322. The anchoring struts 324 are made of a highly flexible material, such as Nitinol, and may be constricted to pass through the narrower access tube 332. As such, the struts 324 are shown expanding outward by the movement arrows. It should be understood that for the sake of clarity the proportional shape of the access tube 332 is somewhat enlarged from what would be its size in reality. In actual use, an access tube 332 of no greater than 33 French would be used.

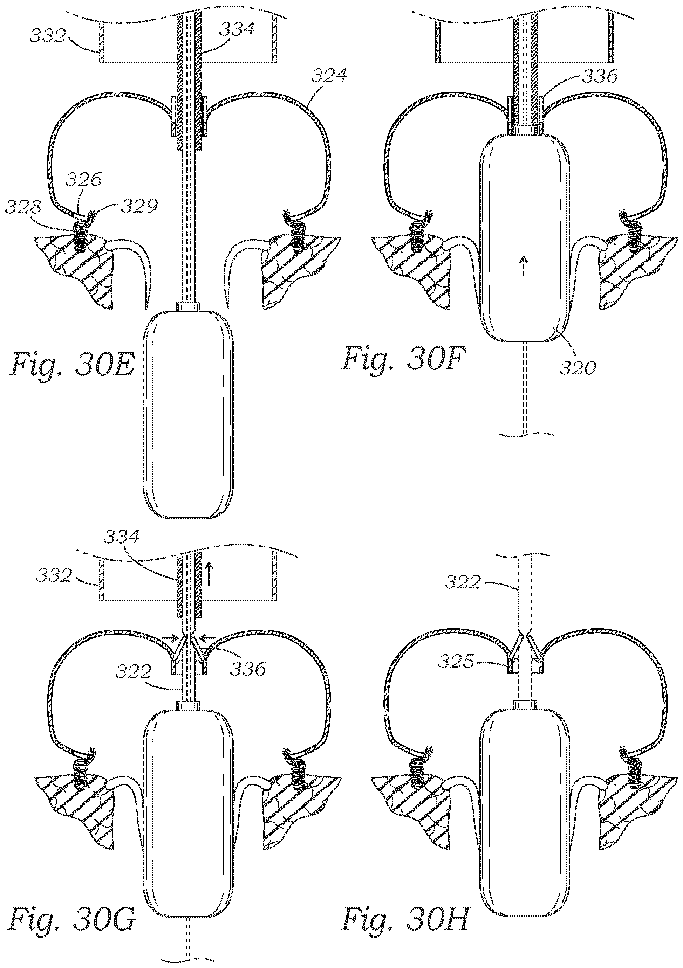

Each of the connecting sutures 329 passes through an eyelet 326 of one of the struts 324. Consequently, once the struts 324 are delivered to the annulus, they abut one of the tissue anchors 328. The next step shown in Figure 30F is to tie off and then sever each of the sutures 329 at the eyelets 326. This secures the terminal ends of the anchoring struts 324 to the annulus.

Figure 30E then shows retraction of the spacer 320 to a desired position between the leaflets. This operation may be done under fluoroscopy so as to evaluate the efficacy of the regurgitation reduction. The shaft 334 is then retracted proximally as seen in Figure 30G. A crimp preferably comprising a plurality of small teeth 336 in a crown shape attached to the central hub 325 is then caused to clamp onto the flexible shaft 322. The teeth 332 may be spring-biased inward, or a separate tool may be used to plastically crimp them onto the shaft 322. Of course, there are other ways to attach the central hub 325 to the shaft 322. Finally, Figure 30H shows the fully implanted spacer and supra-annular anchoring struts 324 after removal of the shaft 334 and access tube 332.

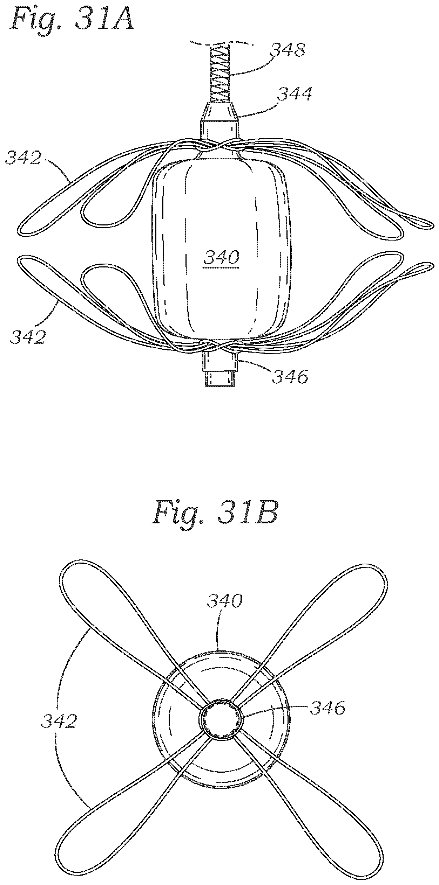

Another regurgitation reduction system that does not rely on a ventricular anchor is illustrated in Figures 31-32. As seen in Figures 31A and 31B system includes a regurgitation reduction spacer 340 with retention anchors 342 extending radially outward from both ends that center the spacer in the annulus and prevent it from migrating in either direction. In the illustrated embodiment, the retention anchors 342 comprise flower-shaped Nitinol wires attached to proximal and distal hubs 344, 346 at the end of the spacer 340. In one embodiment, a proximal shaft 348 remains attached to the proximal hub and may be secured to a secondary anchor above the valve annulus, such as a stent in one of the adjacent vessels or a subcutaneous anchor positioned outside of the subclavian vein, such as seen in Figure 1.

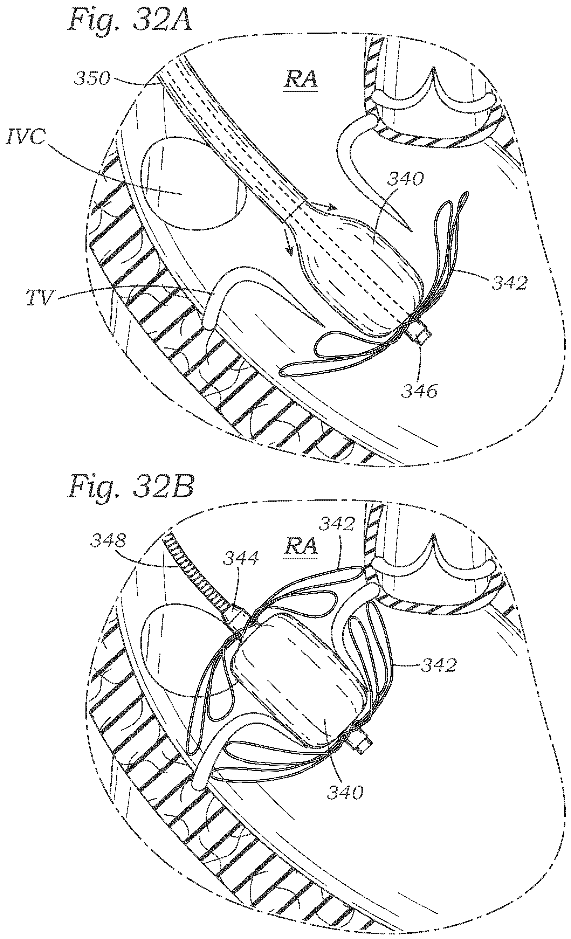

With reference to Figures 32A and 32B, an exemplary deployment sequence is shown. A delivery sheath 350 may be introduced via the subclavian vein into the right atrium RA carrying the system including the spacer 340. Alternatively, the delivery sheath 350 may be introduced via the femoral vein up through the inferior vena cava IVC. The delivery sheath 350 is advanced until a distal end is within or slightly beyond the tricuspid valve TV. Either by displacing a pusher (not shown) behind the spacer 340 or by holding the spacer stationary and retracting the delivery sheath 350, first the distal retention anchors 342 are released and then the expandable spacer 340 within the valve leaflets, as seen in Figure 32A. The distal set of flower-shaped retention anchors 342 are sized and slightly curled in a proximal direction so as to fit underneath the tricuspid valve TV leaflets and contact the tricuspid valve TV annulus within the right ventricle.

Further expulsion of the spacer 340 or retraction of the sheath 350 releases the entire spacer and the proximal set of retention anchors 342, as seen in Figure 32B. Again, the proximal retention anchors 342 are desirably sized to extend outward a sufficient distance in the right atrium RA to engage the tricuspid valve TV annulus, and may be curled in a distal direction towards the distal set of retention anchors. All of the retention anchors 342 are atraumatic in that they have relatively rounded or loop-shaped terminal ends. The aggregate of the proximal and distal retention anchors 342 clamps the tricuspid annulus therebetween in an atraumatic manner. Preferably, both sets of retention anchors 342 are curled or slightly vaulted so as to avoid interfering with movement of the tricuspid valve TV leaflets. The spacer 340 is securely held in place to reduce regurgitation through the tricuspid valve TV.

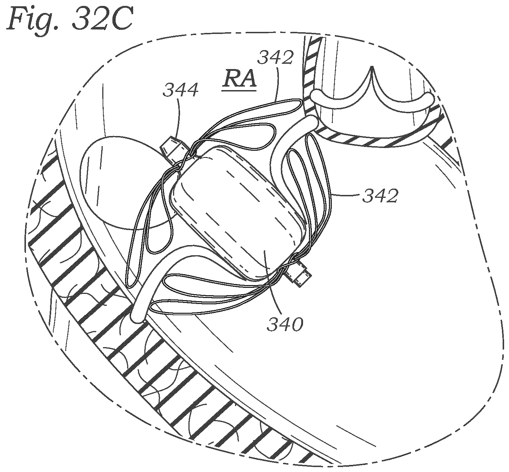

Figure 32C illustrates an alternative version of the spacer 340 having the flower -shaped proximal and distal retention anchors 342. In this embodiment, there is no shaft attached to the proximal hub 344 such that the retention anchors 342 provide the sole holding force. Again, this simplifies the deployment procedure by eliminating the secondary anchor above the annulus.

The retention anchors 342 are preferably formed of nitinol wire in a flower-like shape created by heat setting. The combination of the retention anchors 342 on both proximal and distal sides of the valve forms a floating spacer in the annulus that cannot be pushed out of the annulus by any flow or pressure. The flower-shaped anchors 342 keep the spacer in position. One advantage of this design is that much less imaging is required than for other devices. To deploy, one only needs to know where the valve plane is via fluoroscopy. Moreover, as mentioned above, this device does not need an anchor in the ventricle, which removes certain risks of complications from the anchor deployment. Another advantage is that this design can accommodate somewhat larger spacer sizes, which might otherwise be limited by the strength of tissue anchors.

### Cross-Annulus Harnesses

In addition to directly anchoring the regurgitation reduction spacer to the native annulus, either supra- or sub-annularly, the present application contemplates a system of harnesses extending across the annulus which serve to both center and otherwise stabilize the spacer, and also to provide an annular reduction (annuloplasty). It should be understood that the harnesses comprise flexible filaments that are anchored on opposite sides of the annulus, either tricuspid or mitral, and extend across the flow orifice to engage and stabilize the spacer. The flexible filaments may be sutures, bands, or the like suitable for long-term implant in the body, and the anchors used may be any known tissue anchor including those disclosed in various embodiments of the present application.

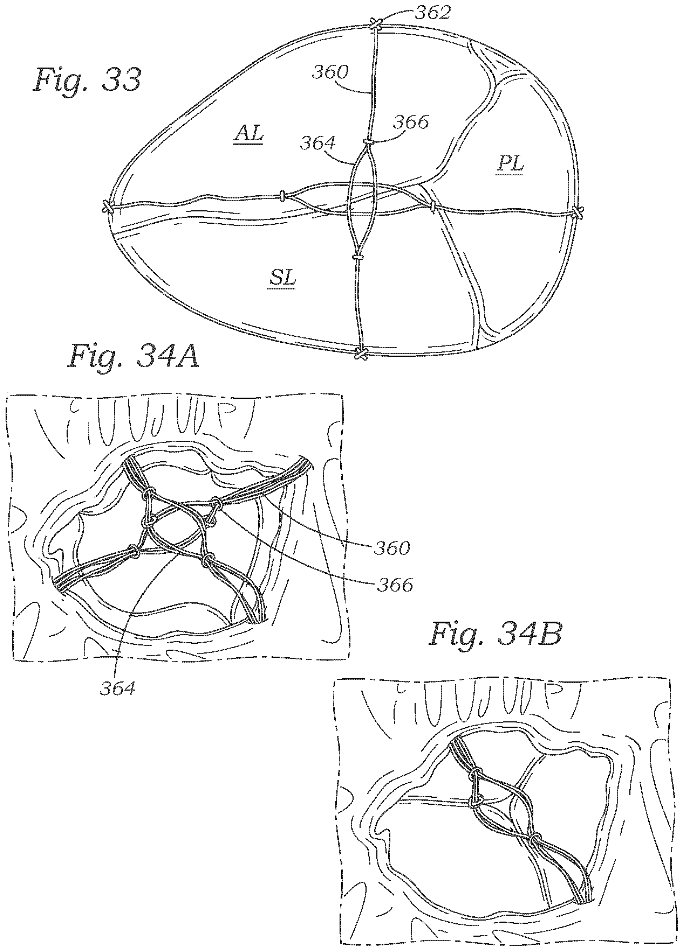

Figure 33 is a top plan view of a tricuspid annulus and leaflets showing one embodiment of a supra-annular harness for anchoring a regurgitation reduction spacer, and indicating the three leaflets: anterior leaflet AL, posterior leaflet PL, and septal leaflet SL. The tricuspid annulus has a somewhat ovoid (egg-shaped) periphery with a longer major axis oriented horizontally in the figure and a minor axis oriented vertically. The harness comprises a plurality of filaments 360 secured around the annulus at anchors 362, shown schematically. The illustrated embodiment includes a single filament 360 extending generally along the major axis across the valve, and a single filament extending generally along the minor axis across the valve.

Each of the filaments 360 is separated in a midportion so as to form a snare 364. The opposite ends of each snare 364 are delineated by junctions 366 on either end. As will be understood below, the junctions 366 may comprise knots in the filaments 360, crimps, eyelets, or other such devices. The snares 364 are sized to closely surround a spacer which will thus be secured by the filaments 360. Because of the crossed nature of the filaments 360, the two snares 364 provide stabilization in two dimensions to the spacer, as will be shown below.

Figures 34A and 34B are perspective views of the tricuspid annulus showing alternative supra-annular harnesses. In these views, the tissue anchors are not shown, but can be assumed to be embedded within the annulus. Figure 34A illustrates a crossed arrangement of filaments much like that shown in Figure 33, although multiple filaments extend across in each direction. The junctions are shown as small circular crimps that gather multiple filaments together. A knot is shown within the snares of each of the filaments indicating that in each direction only a single filament is used. In the illustrated embodiment, there are four segments of the single filament that cross the annulus, the four being separated into two filaments on either side of the snares. Likewise, Figure 34B shows a harness which includes a filament across only one direction of the annulus. Once again, there are four segments of a single filament having its free ends knotted within the snare and with small circular crimps used for the junctions.

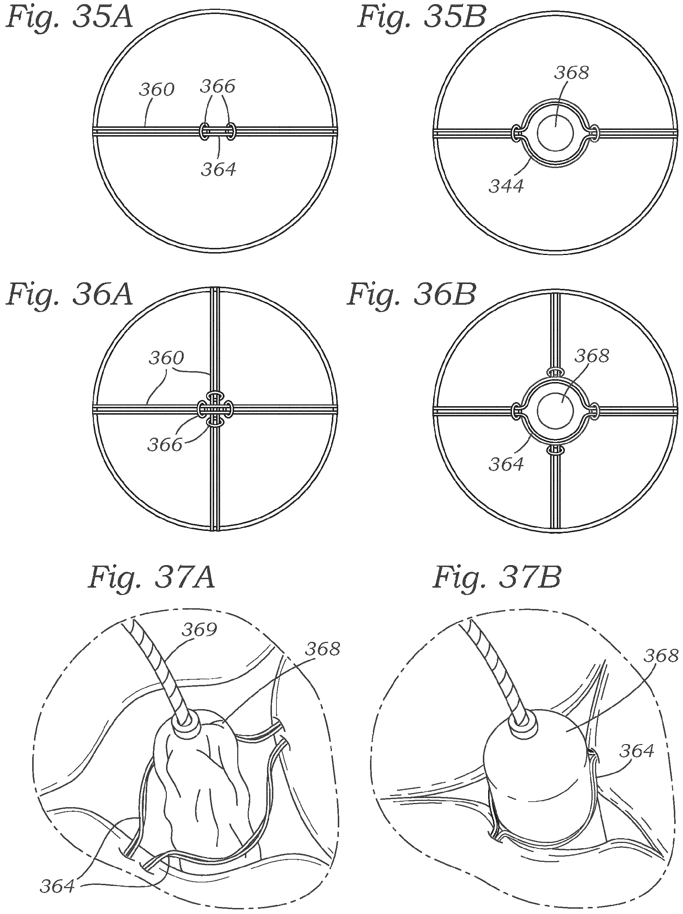

Figures 35A and 35B schematically illustrate deployment of a regurgitation reduction spacer using a supra-annular harness extending diametrically across the valve annulus. First of all, the harness in the form of a filament 360 is implanted across the annulus, and the junctions 366 properly positioned. Figure 35B shows a spacer 368 captured within the snare 364 and expanded. In a preferred embodiment, the junctions 366 limit the size of the snare 364 to be approximately the same size as the expanded spacer 368 which restricts further movement of the spacer. Moreover, as the spacer 368 expands within the snare it exerts tension on the filaments 360 extending outward and tightens the harness.

Figures 36A and 36B show a supra-annular harness extending diametrically in a crossed configuration across the valve annulus. Once again, the crossed filaments 360 are first implanted, and the junctions 366 properly positioned. The spacer 368 is advanced within both of the snares 364 in a constricted configuration and then expanded. As seen in Figure 36B, the snares 364 are sized to closely surround the expanded spacer 368 so as to constrain its movement in two directions. Again, the expanding spacer 368 exerts tension on the filaments 360 and tightens the harness.

Figures 37A and 37B are perspective pre- and post-deployment views of a regurgitation reduction spacer 368 within a supra-annular harness having a snare 364. The radially contracted spacer 368 is first advanced on the end of a flexible shaft 369 within the snare 364. Subsequently, the spacer 368 is allowed to expand (or actively expanded) within the snare 364. As the spacer 368 expands within the opening defined by the crossed filaments, the surrounding annulus must constrict to allow the opening to expand around the spacer 368. This constriction causes an annular reduction or annuloplasty.

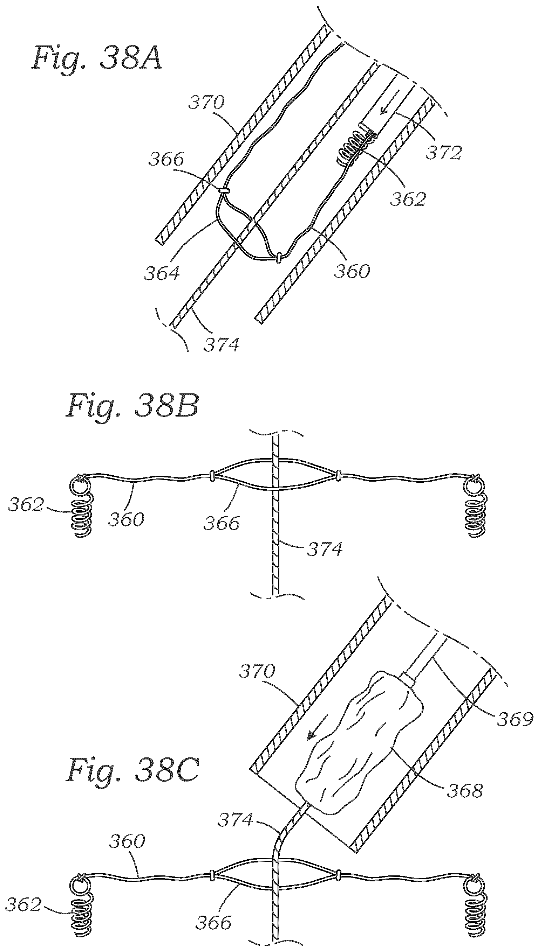

Figures 38A-38C schematically illustrate steps in deploying one of the supra-annular harnesses. The user advances and access tube 350 into proximity with the annulus and then advances one or more anchor drivers 352 therein. Each of the anchor drivers 352 carries an anchor 362 having one free end of a filament 360 passed therethrough. Again, the tissue anchor 362 is a corkscrew-type and the driver 352 simply screws it into the tissue, though other anchors and corresponding drivers are known. The snare portion 364 of the filament 360 is also carried to the annulus around a guide wire 354.

Figure 38B schematically shows the anchors 362 implanted and the filaments 360 secured thereto, such as by being tied off. The filament 360 and snare 366 in the midportion extends across the annulus with the guidewire 354 through the snare. Figure 38C then shows the access tube 350 in proximity to the snare 366. The spacer 368 in a contracted configuration on the end of the flexible shaft 369 advances over the guidewire 354 and through the access tube 350. The user then advances the contracted spacer 368 into its proper position within the snare 366. Expansion of the spacer 368 completes the process.

### Intra-Atrial Anchors

As explained elsewhere herein, anchoring spacers in the right ventricle can be problematic because the right ventricle is very trabeculated and difficult to anchor in. Placing a myocardial anchor is challenging. Clinical complications such as anchor dislodgements and right ventricle perforations have been encountered in clinical testing. Consequently, the present application contemplates anchoring the various regurgitation reduction spacers without interacting with the right ventricle and/or placing an active anchor in the myocardium.

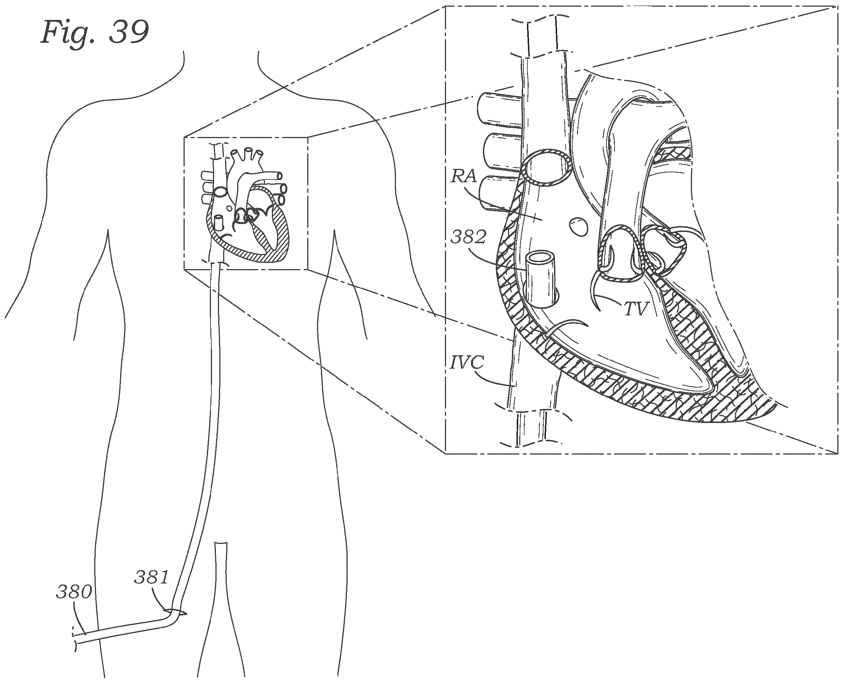

Figure 39 is a schematic illustration of a human body showing a delivery sheath 380 inserted along a femoral access path in the process of placing a regurgitation reduction spacer and associated atrial anchors at the tricuspid valve, in accordance with various embodiments described herein. The sheath 380 is percutaneously placed within the femoral vein through a small incision 381 in the upper leg using the so-called Seldinger technique. In this process, the physician accesses and then punctures a desired vessel or cavity with a sharp hollow needle, and advances a round-tipped guidewire through the lumen of the needle. The needle is withdrawn, and a sheath or blunt cannula can now be passed over the guidewire into the cavity or vessel. The process may also include insertion and subsequent removal of one or more obturators over the guidewire to define gradually increasing diameter access holes into the vessel.

With reference to the enlarged view of the heart in section, a distal end 382 of the sheath 380 emerges from the inferior vena cava IVC within the right atrium RA and adjacent to the tricuspid valve TV. As will be understood by those of skill in the art, the access pathway to the right atrium RA shown in Figure 39 can be used to deploy a number of the regurgitation reduction spacers and associated anchors described herein.

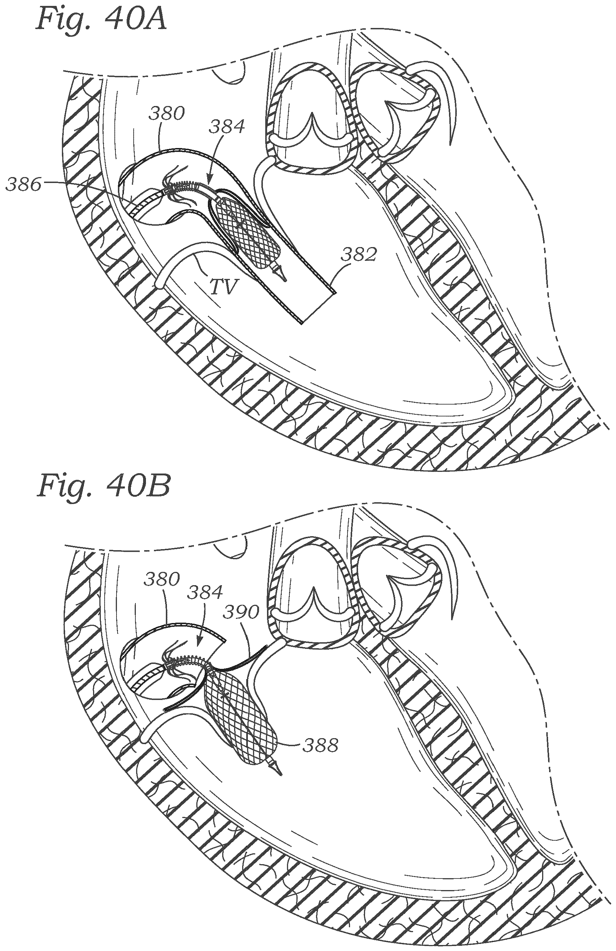

Figures 40A-40D illustrate several steps in deploying a dual-support type of anchoring system 384 comprising a regurgitation reduction spacer and associated atrial anchors at the tricuspid valve. Initially, the delivery sheath 380 is advanced such as through the inferior vena cava IVC as described above so that the distal end 382 extends through the tricuspid valve TV. A flexible delivery shaft 386 having sufficient column strength is attached to a proximal end of the regurgitation reduction system 384 and advances the system through the sheath 380, as seen in Figure 40A.

Figure 40B shows the delivery sheath 380 retracted to expose and permit expansion of a regurgitation reduction spacer 388 between the leaflets of the tricuspid valve TV. As explained above, the spacer 388 expands once it is released from within the sheath 380 and may be adjustably sized. The sheath 380 has been retracted far enough to also release a plurality of expandable annulus anchors 390 extending radially outward from a proximal end of the spacer 388.

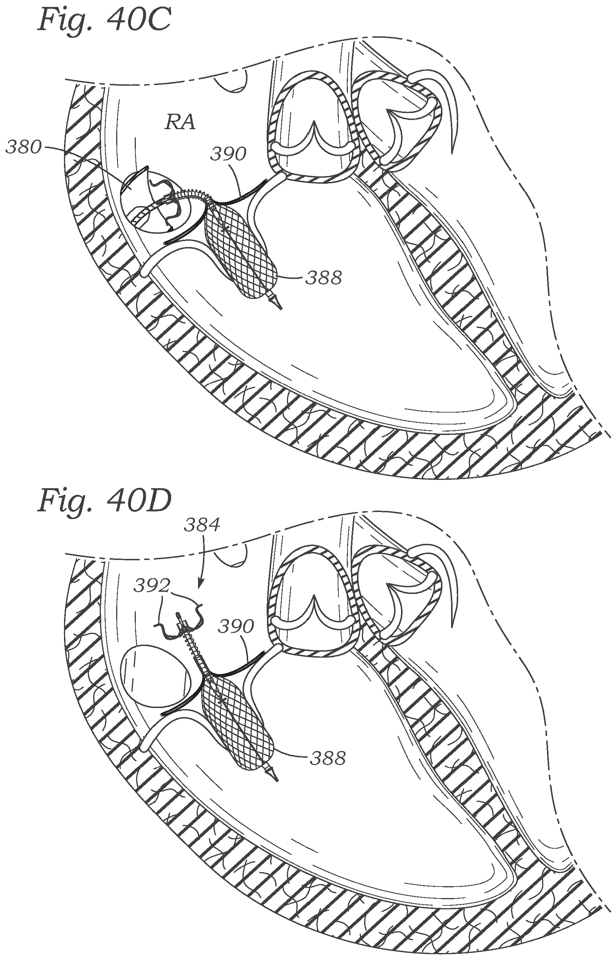

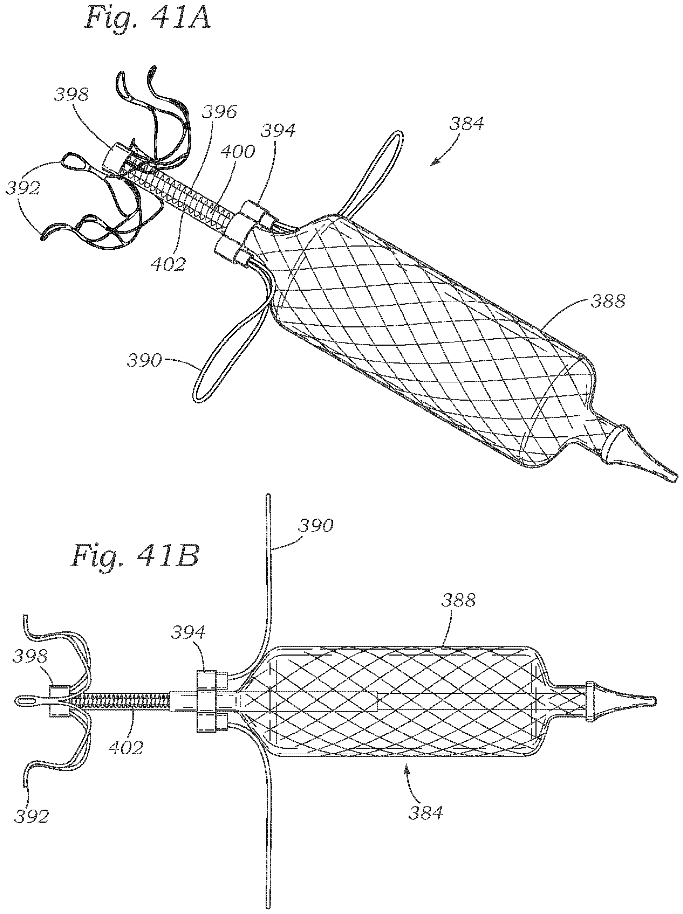

Figure 40C shows further retraction of the delivery sheath 380 to expose the entire regurgitation reduction system 384 within the right atrium RA, and Figure 40D illustrate a final position of the system once the sheath 380 is removed and the flexible delivery shaft 386 disconnected. A plurality of proximal anchors 392 are positioned in contact with the wall of the right atrium RA. A better understanding of the system 384 will be obtained after a description of the particular structure as illustrated in Figures 41A and 41B.

Figures 41A and 41B are perspective and side views of the exemplary dual-support type of anchoring system 384 of the regurgitation reduction spacer 388 and associated anchors 390, 392. The spacer 388 has a proximal hub 394 from which the annulus anchors 390 extend. In a preferred embodiment, the anchors 390 comprise Nitinol wires formed in loops so as to provide a traumatic contact with the valve annulus. As with the embodiment of Figures 31-32, the anchors 390 may be curled or slightly vaulted so as to avoid interfering with movement of the tricuspid valve TV leaflets. The anchors 390 desirably extend radially outward far enough to contact bases of the leaflets, or the tricuspid valve annulus. In the illustrated embodiment, there are two anchors 390, although three or more may also be provided.

The hub 394 on the proximal end of the spacer 388 desirably connects to a longitudinally-oriented tube 396 which terminates in a second hub 398. The proximal anchors 392 which contact the atrial wall attached to and extend radially outward from the second hub 88. Again, the proximal anchors 392 provide an array of atraumatic pad-like feet for contacting and applying pressure against the wall of the right atrium RA. Desirably, there are at least four and preferably at least six of the anchors 392 evenly spaced around the hub 398. A central shaft 400 and spring 402 arrangement within the tube 396 provides a spring force biasing apart the two hubs 394 and 398. That is, the assembly of the second hub 398 and attached atrial anchors 392 is spring-loaded relative to the first hub 394.

When deployed as seen in Figures 40C and 40D, the proximal anchors 392 are initially depressed toward the spacer 388 and permitted to spring outward against the wall of the right atrium RA. The system 384 relies on the stabilizing action generated by displacing the two pad-like structures of the anchors 390, 392 against opposing surfaces with a tensioning mechanism such as the spring 402. The valvular spacer 388 is suspended from this tensioned anchor. The tensioned anchor apposes the atrial roof and the valvular annulus. This anchoring mechanism is similar to tension rods which are used to hang curtain rods.

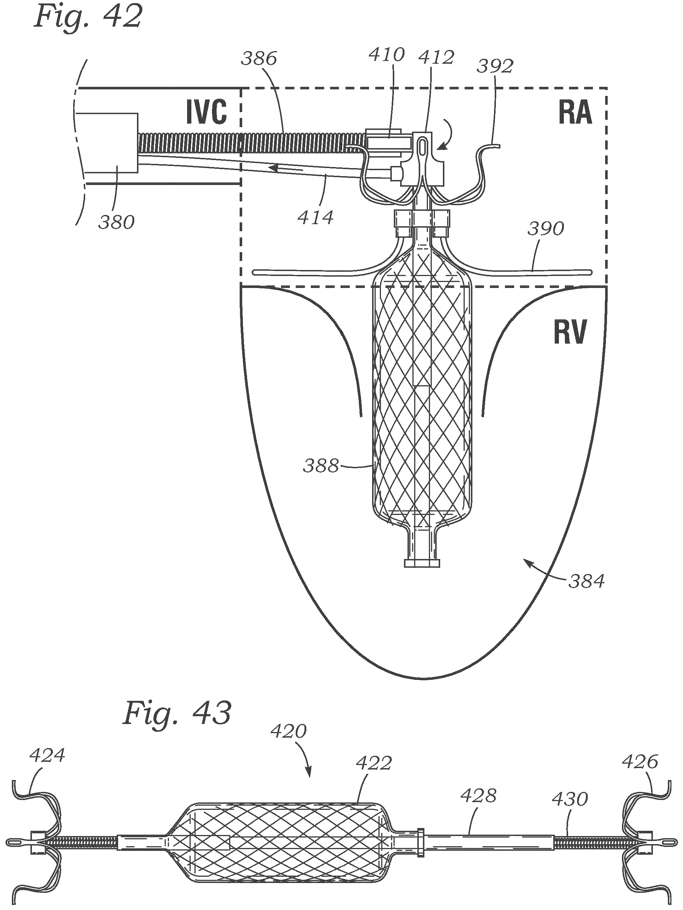

Figure 42 is a schematic sectional view of the right side of the heart and one means for positioning the exemplary dual-support type of regurgitation reduction anchoring system 384. As before, the system 384 is delivered by advancing it through a delivery sheath 380 on the distal end of a delivery shaft 386. The delivery shaft 386 terminates in a first hinge member 410 which is pivotally connected to a second hinge member 412. Although not shown, the second hinge member 412 connects around the second or proximal hub 398 from which the proximal anchors 392 emanate. A flexible control member 414 such as a pull rod extends through the delivery sheath 380 in parallel with the shaft 386 and attaches to a distal part of the second hinge member 412.

Once expelled from the sheath 380 in the right atrium RA, the system 384 may be rotated so that the spacer 388 extends through the tricuspid valve into the right ventricle RV. That is, by pulling on the control member 414 as indicated by the movement arrows, the second hinge member 412 may be rotated perpendicular to the longitudinal axis as indicated by the rotation arrow. At this point, fluoroscopy or other visualization means may be utilized to properly position the spacer 388 as well as the distal anchors 390 around the valve annulus. Once properly positioned, a release mechanism (not shown) between the second hinge member 412 and the proximal hub 398 is actuated to release the system 384 into the right atrium RA. The spring force between the two hubs 394, 398 then causes contact between the pad-like proximal anchors 392 and the wall of the right atrium RA.

Figure 43 is a side view of another exemplary dual-support type of regurgitation reduction system 420 having a spacer 422 and both atrial and ventricular anchors 424, 426, respectively. The proximal end of the system 420 having the atrial anchors 424 may be identical to the proximal end of the system 384 described above. A distal shaft 428 extends from the distal end of the spacer 422 and connects to a second spring-loaded shaft 430. The ventricular anchors 426 may thus be tensioned against structure within the right ventricle RV. This system 420 the stabilizing both ends of the spacer 422, one in the right atrium RA and ones in the right ventricle RV.

### Stent Anchors

It has been observed that larger regurgitation reduction spacers are needed to support extremely large regurgitant orifices observed in some patients. Testing has revealed that the force required to anchor a valvular occluder such as a regurgitation reduction spacer is quadratically proportional to the cross-sectional area of the occluder. Simply, this force will increase very rapidly for larger spacer designs. Some anchor solutions will not support the larger occluders needed to treat this patient population. For instance, the right ventricle is very trabeculated and difficult to anchor in, sometimes resulting in a less than secure anchor which is unsuitable for the large forces with large spacers. One possible solution is to deploy one or more stents within the large vessels adjacent the atrioventricular valve and suspend the spacer therefrom within the valve.

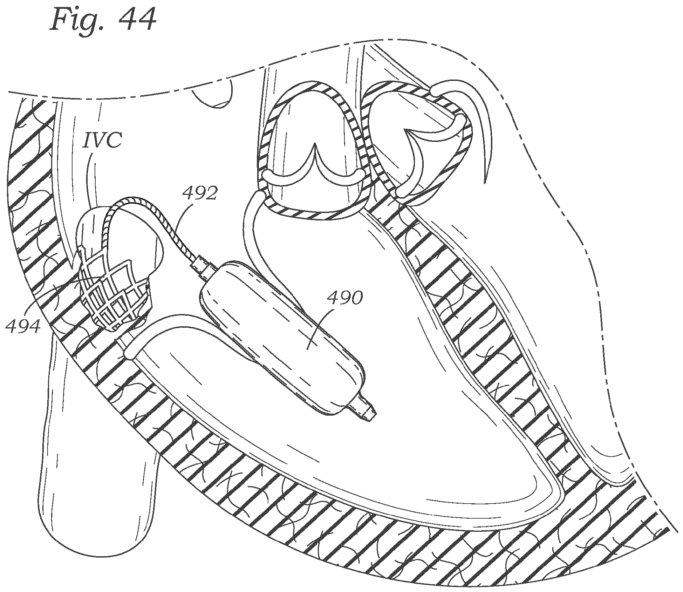

Figure 44 illustrates one solution for anchoring a regurgitation reduction spacer 490 without using anchors that embed within tissue. Namely, the spacer 490 attaches by a tether 492 to a stent 494 placed in a supra-annular flow passage; namely, the inferior vena cava IVC which opens just above the tricuspid valve. The advantages of this anchoring solution include the reduction in trauma to the heart tissue, and the ease of implant. However, the simple nature of the assembly limits the ability to adjust the position of the spacer 490. It is contemplated, however, to couple the supra-annular stent 494 with any of the tether/anchor pairs disclosed herein. For example, the sub-annular tether/anchor pairs 258, 260 disclosed above maybe used in conjunction with the supra-annular stent 494.

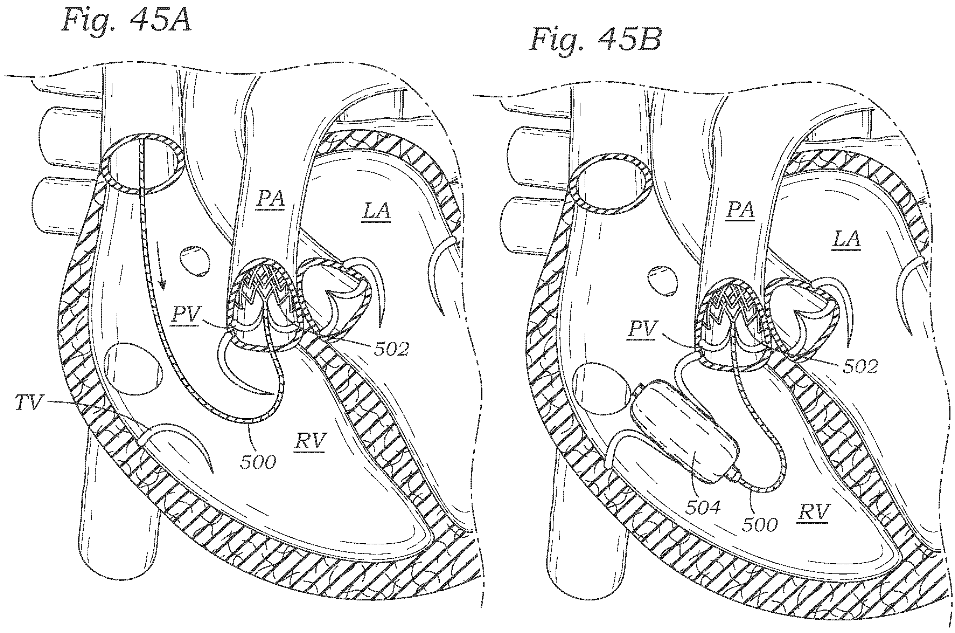

Another potential stent anchor solution is shown in Figures 45A and 45B. Figure 45A shows a first step in deployment of a stent 502 at the distal end of an anchor rail 500. In particular, the anchor rail 500, which may be steerable or advanced over a pre-positioned guide wire, carries the stent 502 in a contracted configuration through the tricuspid valve TV and then through the adjacent pulmonary valve PV into the pulmonary artery PA. This procedure can be done while the heart is beating so that blood flow through the two valves facilitates movement of the rail 500 and stent 502. Indeed, the process for floating the stent 502 along this path is similar to that used when placing a Swan-Ganz catheter. Once the stent 502 is positioned in the pulmonary artery PA, it is expanded to anchor it in place. Preferably, the stent 502 is self-expanding and deployed by constraining it within a sheath which is then retracted. Alternatively, a balloon-expandable stent 502 may be utilized, though this increases the complexity of the procedure.

Figure 45B shows a second step wherein a regurgitation reduction spacer 504 has been advanced along the rail 500 and secured in position within the tricuspid valve TV. The rail 500 may be severed on the proximal end of the spacer 504 so that the only anchor is the connection to the stent 502. This anchoring solution benefits from a reduced amount of guidance using ultrasound echolocation or the like because of the clearly defined target location for the stent 502 in the pulmonary artery. Alternatively, the rail 500 may be left in place and anchored somewhere above the right atrium, such as in the subclavian vein as described above. Still further, supra-annular tether/anchor pairs may be connected to the proximal end of the spacer 504. As such, it should be emphasized that any combination of anchors described herein may be made as long as they are not mutually exclusive.

The ability to adjust the position and orientation of the regurgitation reduction spacers described herein in conjunction with utilizing stent anchors for larger anchoring forces provides a great advantage. The following discussion relates to such systems where the anchoring force is provided by one or two stents implanted above the annulus.

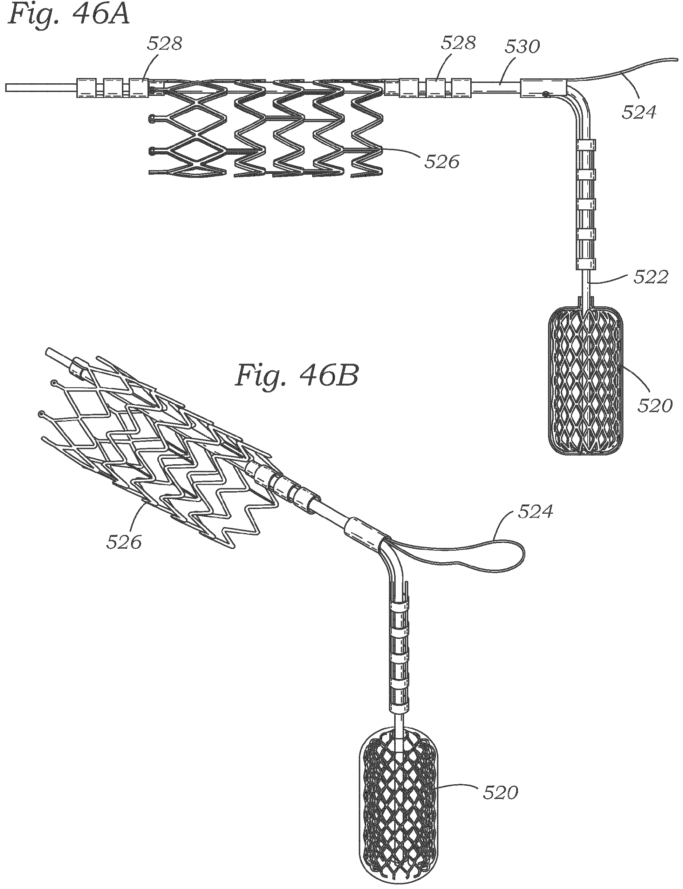

Figures 46A and 46B are side and perspective views of a single-stent anchoring system for an adjustably positioned regurgitation reduction spacer 520. The system includes an elongated tubular suspension shaft 522 that connects to a proximal end of the spacer 520 and receives a guidewire 524 which assists deployment, as will be described. An anchoring stent 526 connects on either end to a pair of stabilizing tubes 528 that slidably receive the suspension shaft 522. The longitudinal position of the suspension shaft 522 may be adjusted and then locked relative to the anchoring stent 526. The suspension shaft 522 further passes through a main shaft 530 which slides within the tubes 528.

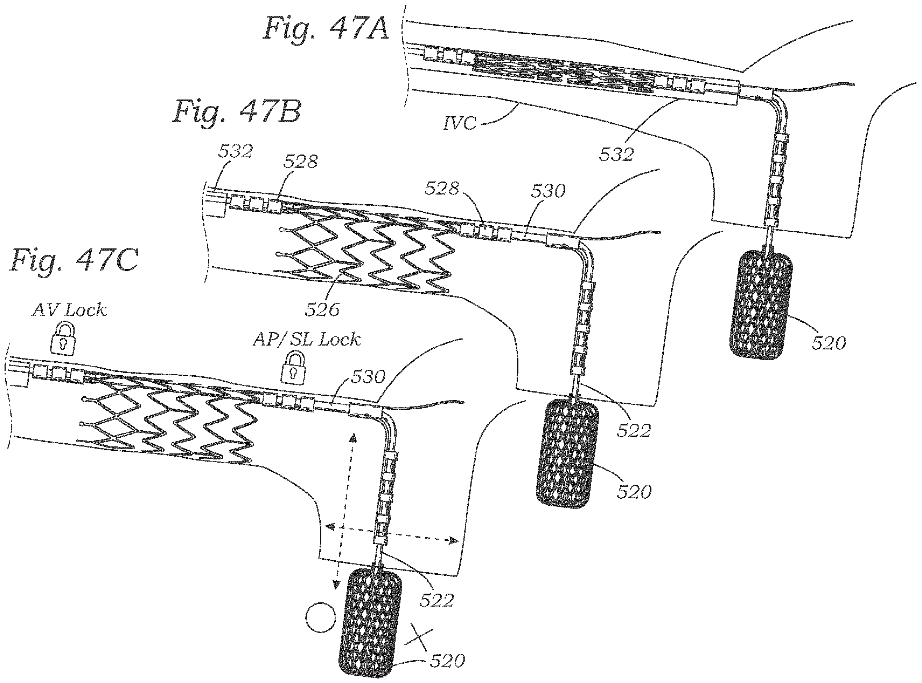

Figures 47A-47C schematically illustrate three steps in positioning the regurgitation reduction spacer having the single-stent anchoring system of Figures 46A and 46B. In Figure 47A, the assembly of the spacer 520 and connected shafts and stent has been advanced through a delivery sheath 532 to the right atrium RA. As explained above, the access pathway may be through the interior vena cava IVC as shown, or through the subclavian vein and superior vena cava. Figure 47B shows the delivery sheath 532 retracted which permits expansion of the anchoring stent 526 in the associated vessel. Finally, in Figure 47C, repositioning of the regurgitation reduction spacer 520 is done, preferably under visualization such as fluoroscopy. First, the entire assembly of the spacer 520 connected with the suspension shaft 522 and main shaft 530 is displaced laterally relative to the stabilizing tubes 528 until the spacer is properly positioned laterally within the valve annulus. At this point, the position of the main shaft 530 is locked relative to the anchoring stent 526. Subsequently, just the spacer 520 and suspension shaft 522 are displaced relative to the main shaft 530, which adjusts the longitudinal position of the spacer within the valve annulus. In this regard, the suspension shaft 522 is flexible and forms a bend as shown between the When the proper position is attained, a second lock is actuated to fix the position of the suspension shaft 522 relative to the main shaft 530.

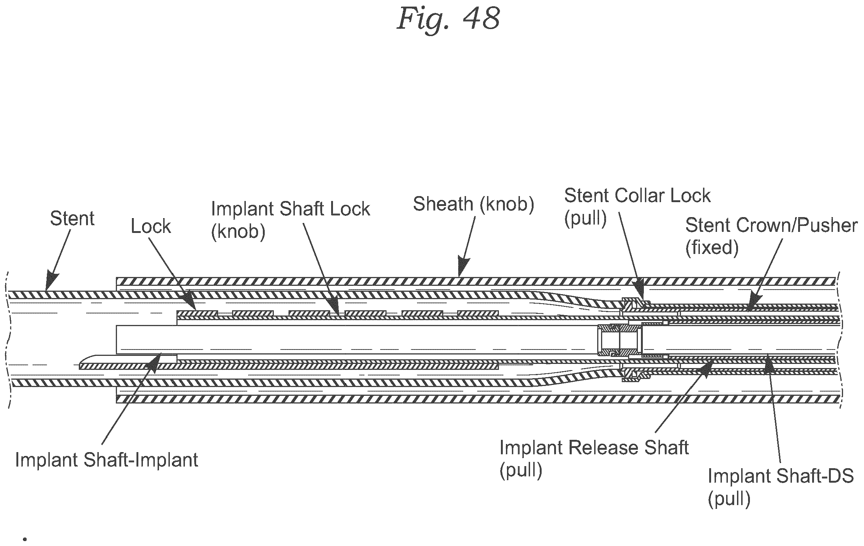

Figure 48 is a longitudinal sectional view through a portion of the single-stent anchoring system of Figures 46A and 46B. This particular assembly requires 8 shafts.

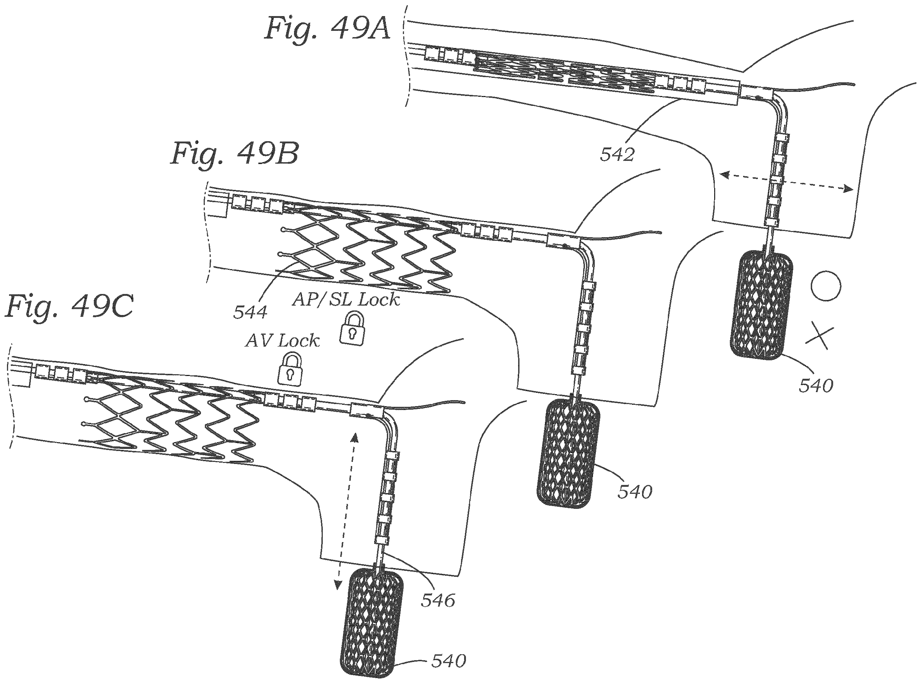

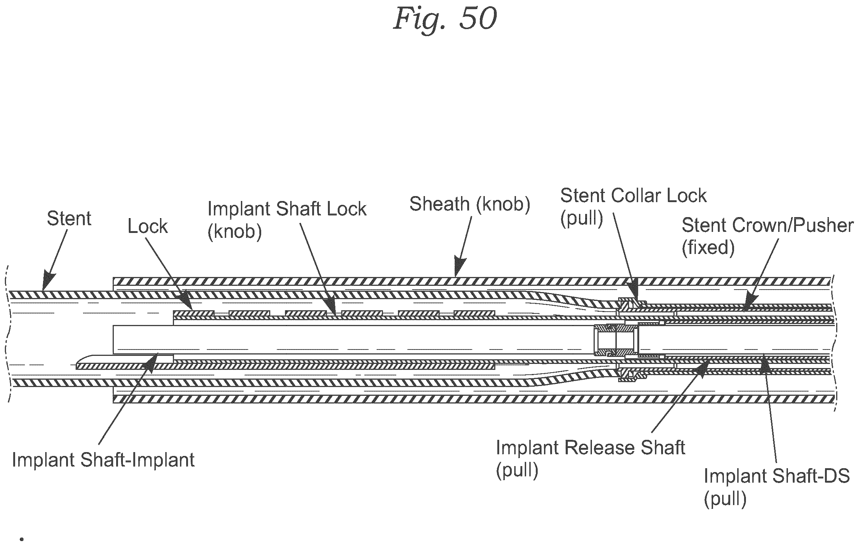

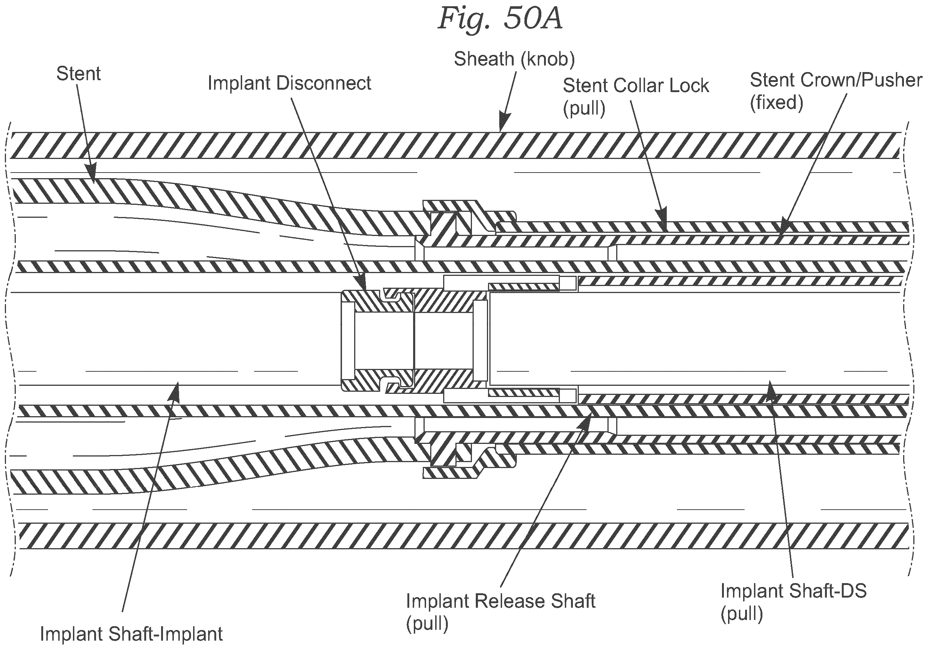

Figures 49A-49C and 50/50A schematically illustrate three steps in positioning a regurgitation reduction spacer 540 utilizing an alternative single-stent anchoring system. The system is similar to that shown above with respect to Figures 46A and 46B, however the deployment assembly as seen in Figures 50 and 50A only requires six shafts. Deployment of the spacer 540 involves first translating the entire delivery system including a delivery sheath 542 laterally to properly positioned the spacer 540 within the valve annulus, as seen in Figure 49A. Subsequently, the sheath 542 is retracted to allow a self-expanding anchoring stent 544 to expand, such as within the inferior vena cava. Finally, in Figure 49C the longitudinal position of the spacer 540 is adjusted relative to the valve leaflets, preferably under visualization. Again, this is done by sliding of a shaft 546 on which the spacer suspends relative to the concentric tubes attached to the anchoring stent 544, and then locking the relative position of the shaft 546.

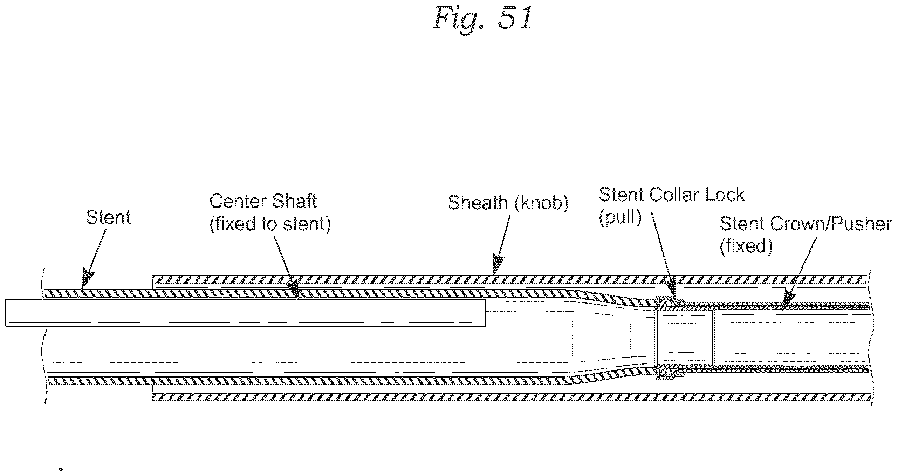

Figure 51 is a longitudinal sectional view through a portion of a still further single-stent anchoring system that utilizes just three shafts. Only three shafts are used by eliminating the adjustment of the spacer 540 relative to the valve leaflets.

### Hanging T-shaped Anchoring System

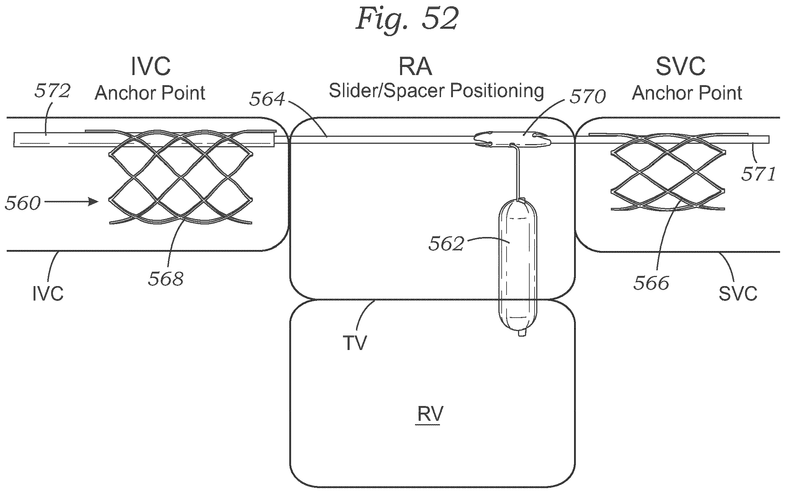

Figure 52 is a schematic view showing a T-shaped dual-stent anchoring solution for an adjustably positioned regurgitation reduction spacer of the present application suspended across the right atrium RA between two vascular ostia opening to the atrium. The body in this sequence is schematically shown with regions indicated by the acronyms IVC, RA, RV and SVC, for, respectively, the interior vena cava, the right atrium, the right ventricle, and the superior vena cava. This method provides a system 560 that passively anchors a tricuspid valve occluder or spacer 562 with a spanning bar 564 that connects to two stabilizing devices or stents 566, 568 in the ostia of the vena cavae. The dual-stent anchoring system 560 is especially useful because it provides a modular platform for structurally supporting larger spacer designs. Again, oftentimes larger spacers are needed to support the extremely large regurgitant orifices observed in certain cases. This concept could potentially be applied to the left side of the heart as well, to address mitral regurgitation. The spacer 562 could reside between the mitral valve leaflets with anchors on both the proximal and distal ends and anchored in two vascular ostia opening to the left atrium.

The T-shaped anchoring system 560 generally comprises four implanted components: a distal stabilizing element or stent 566 in the superior vena cava SVC, a caval spanning bar 564, a locking slider coupling 570, and a proximal stabilizing element or stent 568 in the inferior vena cava IVC. The two stabilizing elements or stents 566, 568 are desirably self-expanding, and preferably formed of Nitinol. The load bearing element is the caval spanning bar 564 which is relatively rigid, and preferably formed of a biostable metal such as titanium, but may also be a polymer such as fiber-reinforced polyamide (Nylon) or other polymer such as polycarbonate. The load bearing capacity of the caval spanning bar 564 can be increased, for example, by changing the diameter and/or wall thickness of the bar.

In use, the T-shaped anchoring system 560 is delivered by first placing and expanding the distal stabilizing stent 566 in the superior vena cava SVC. The slider coupling 570 is then exposed in the right atrium RA, and the tricuspid valve occluder or spacer 562 extends into the tricuspid valve TV annulus. The spacer 562 is then positioned in the septal-lateral and anterior-posterior direction, and the slider coupling 570 is locked in place. Finally, the proximal stabilizing stent 568 is released to expand in the inferior vena cava IVC, and the implant is released. Greater detail will be provided below.

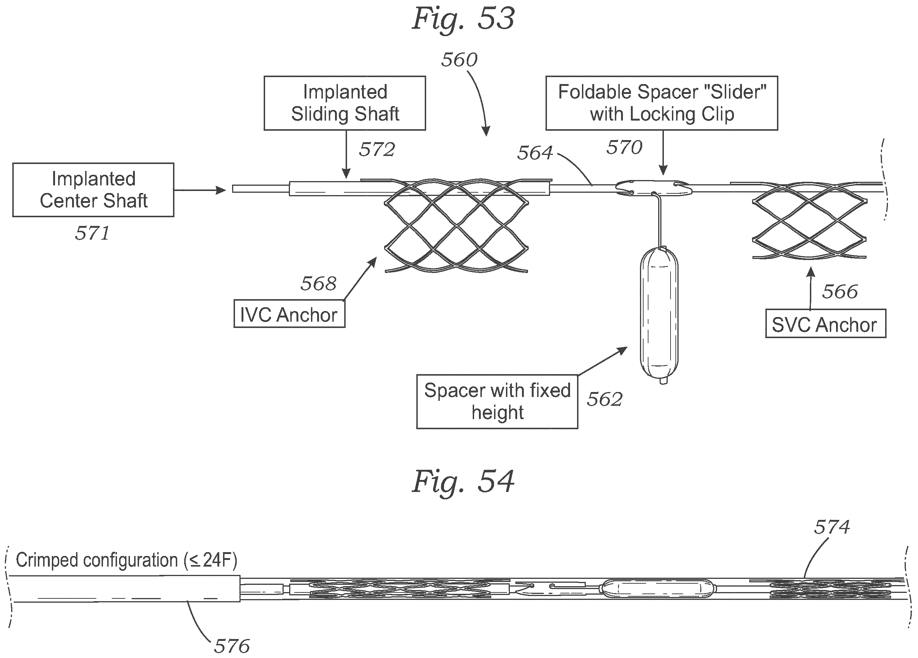

Figure 53 is a side view of the dual-stent anchoring system 560 and spacer 562 of Figure 52 when deployed, and Figure 54 shows the assembly in a crimped configuration. A central shaft 571 extends through the middle of a series of concentric shafts, as will be described, and is fixedly attached to the distal stabilizing stent 566 which is eventually placed within the superior vena cava SVC. A portion of the central shaft 571 remains in the body after deployment of the system 560, and thus has a junction which is used to separate the implanted distal portion shown from a proximal portion that extends out of the body during deployment. An implanted sliding or pusher shaft 572 attaches to the proximal stabilizing stent 568, and is also separated into proximal deployment and distal implanted segments that are ultimately separated. The caval spanning bar 564, which is formed by a segment central shaft 571, slides within the pusher shaft 572 so as to enable relative positioning of the distal stent 566 relative to the proximal stent 568.

In the crimped configuration of Figure 54, an additional constriction sheath 574 surrounds the contracted anchoring system 560, and the entire assembly passes through a delivery sheath 576, which is desirably no more than 24 French in size.

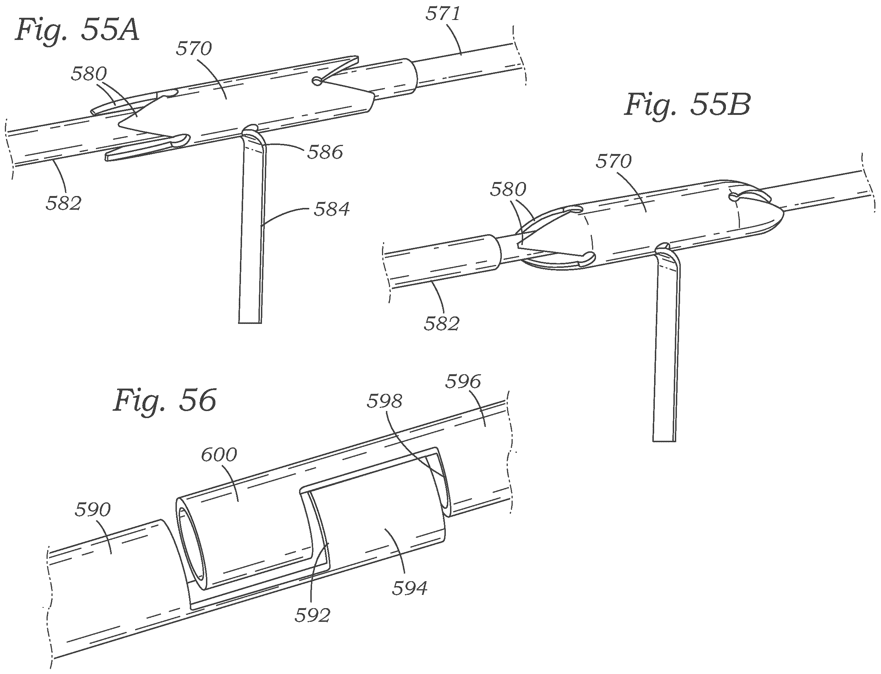

Figures 55A and 55B show an exemplary embodiment of a spacer locking mechanism for use with the slider coupling 570 prior to and after deployment. The slider coupling 570 comprises a generally tubular member having opposite ends formed with a plurality of flexible fingers or petals 580. Initially, the slider coupling 570 is mounted over a tubular release shaft 582 that receives the caval spanning bar 564 for relative sliding movement. The petals 580 are biased inward and each formed with a sharp tip to clamp onto the release shaft 582. As will be explained below, proximal retraction of the release shaft 582 while holding the slider coupling 570 stationary enables the petals 580 to spring inward and clamp onto the caval spanning bar 564. This is one example of a sliding sleeve or coupling that may be locked onto a shaft, and others are contemplated.

The enlarged views of Figures 55A and 55B also show one example of a suspension member 584 that connects between the slider coupling 570 and the regurgitation reduction spacer 562. In the illustrated embodiment, the suspension member 584 comprises an extension of a unitary slider coupling 570 in the form of a tang or finger that can bend from the longitudinal orientation to the perpendicular as shown at a living hinge 586. The suspension member 584 is in the form of a flat strip to provide rotational stability and cause the spacer 562 to pivot away from the slider coupling 570 in one plane. Alternatively, the suspension member 584 may be a separate element that is connected to the slider coupling 570 using a mechanical hinge. Other alternatives are also contemplated.

Figure 56 is an enlarged view of one embodiment of a disconnect between two tubular shafts. As will be clear from the description below, delivery shafts are used to position and deploy the T-shaped anchoring system 560, which delivery shafts are subsequently removed from the body. One way to do this is to provide a proximal delivery shaft 590 having a cutout portion 592 and a distal partial tubular portion 594 between the cutout portion and a distal end. A tubular implant shaft 596 likewise has a cutout portion 598 and a proximal partial tubular portion 600 between the cutout portion and a proximal end. The two partial tubular portions 594, 600 fit closely within the cutout portions 592, 598 and form a continuous junction between two tubes. This junction may be easily held together by a surrounding concentric tube, and may be decoupled by removal of the immediately surrounding tube with minimal manipulation. Of course, other solutions for releasing one tube from another are contemplated.

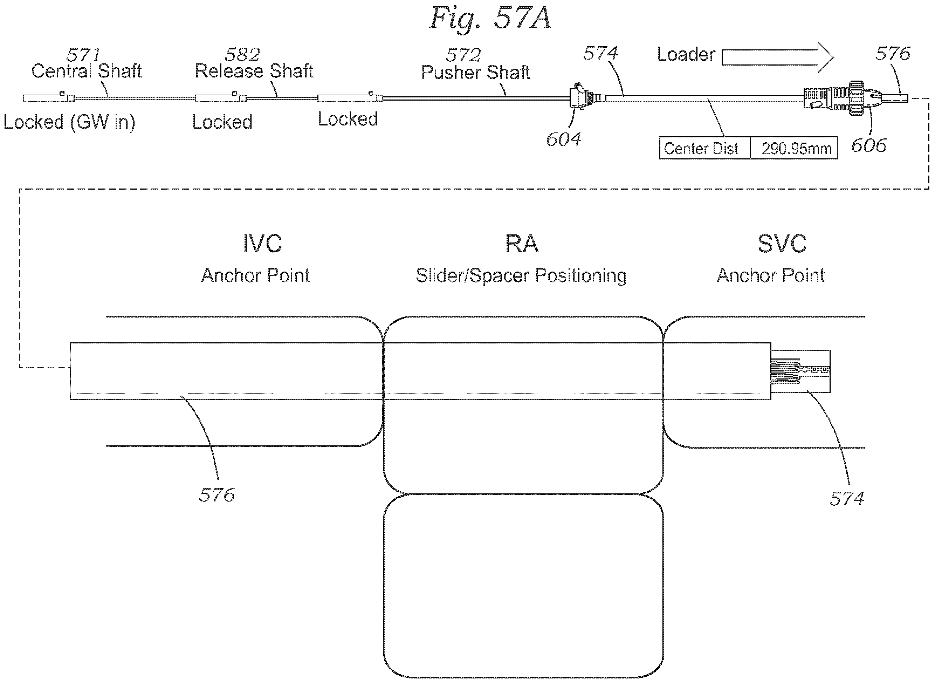

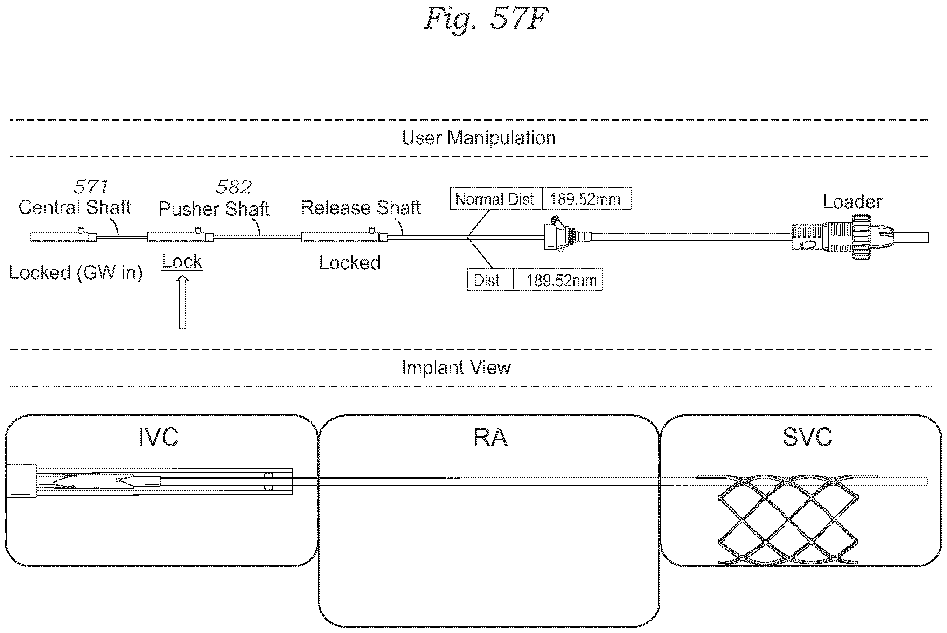

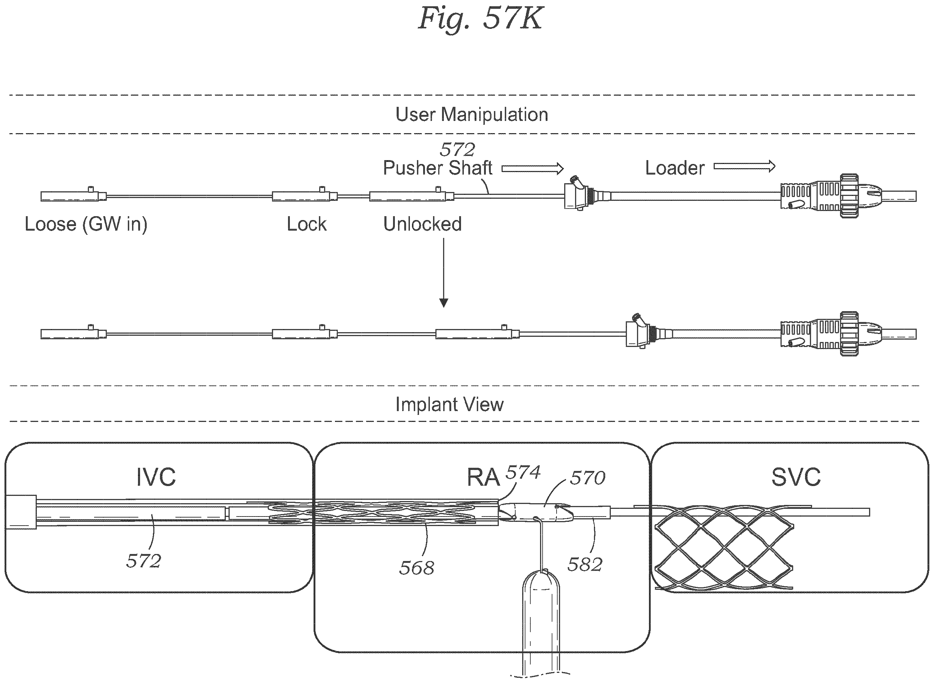

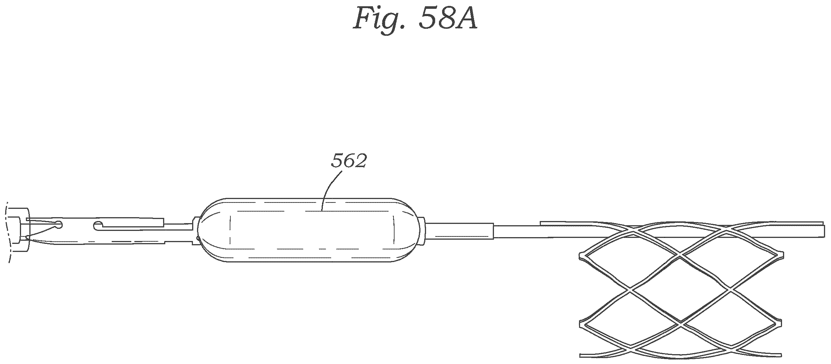

Figures 57A-57U and 58A-58B show a sequence of operation for the dual-stent anchoring system and spacer of Figures 52-53. The delivery system includes a series of concentric tubes which may be selectively locked with respect to one another. The sequence is relatively self-explanatory by annotations, but a description of the sequence follows.

Various locking devices between concentric tubes are known and which may be used herein, one of which is a type of compression fitting known in the industry as a Tuohy-Borst adapter. The Tuohy-Borst adapter has a compressible tubular or ring-shaped elastomeric seal that fits within a bore on the proximal end of a proximal hub. A threaded compression cap fits onto the proximal end of the proximal hub. When the compression cap is tightened, it compresses the elastomeric seal axially, which causes the lumen of the seal to narrow and seal against the inner tube. Thus, by relative rotation of the threaded compression cap with respect to the proximal hub, the elastomeric seal is compressed this actuating the locking mechanism. The various locking/unlocking devices between immediately adjacent concentric tubes may be a Tuohy-Borst type or other known locking solutions. The term Tuohy-Borst will be used as shorthand to indicate any of these types of locks, and should not be considered limiting.

Figure 57A schematically illustrates insertion of the dual-stent anchoring system 560 into the body. The body in this sequence is schematically shown with regions indicated by the acronyms IVC, RA, and SVC, for, respectively, the interior vena cava, the right atrium, and the superior vena cava. In this sequence, the right ventricle is not shown, but is understood to be directly below the right atrium RA. Also not shown is a guidewire which is typically advanced first and then the system advances over the guidewire.

The series of concentric shafts include the central shaft 571 within the release shaft 582, with the release shaft 582 positioned within the pusher shaft 572. Each of the three shafts 571, 572, 582 has a locking devices associated therewith to lock that shaft around the next smaller adjacent concentric shaft, though the smallest central shaft 571 locks around the guide wire (not shown). The locking devices are all locked during initial advancement into the body. These three shafts 571, 572, 582 extend through a loader assembly 604 to which the constriction sheath 574 attaches, and which, in turn, passes through a hub 606 on the right side connected to the delivery sheath 576. A distal end of the constriction sheath 574 is shown just slightly projecting from out of the distal end of the delivery sheath 576 within the SVC. This is the initial position of the system 560 after advancement through the vasculature.

The maximum diameter 24 French delivery sheath 576 is attached to the hub 606 and may be placed into the vascular system using conventional methods, such as via an obturator using the Seldinger technique of widening an initial puncture. The access sheath hub 606 remains outside the body, so that the concentric shafts and their relative movements may be manipulated manually. That is, the upper portion of the schematic views in Figures 57A-57U shows parts of the system 560 outside the body, as noted by the "User Manipulation" label.

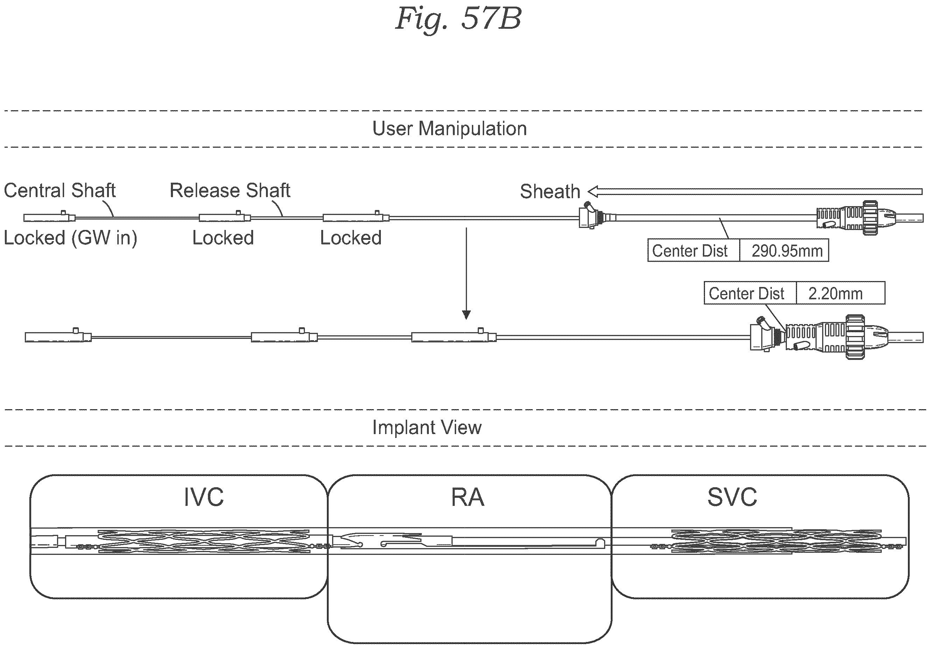

Figure 57B shows a first step in the sequence after introduction, wherein the delivery sheath 576 and its hub 606 retract by about 28.5 cm over the constriction sheath 574 and loader 604 until the distal end of the delivery sheath is positioned within the IVC. As indicated by the marked center distances, this movement may be approximately 280 mm, though that distance can vary depending on the patient's anatomy. Although not numbered, the constriction sheath 574 remains surrounding both of the stabilizing elements or stents 566, 568 to prevent them from expanding.

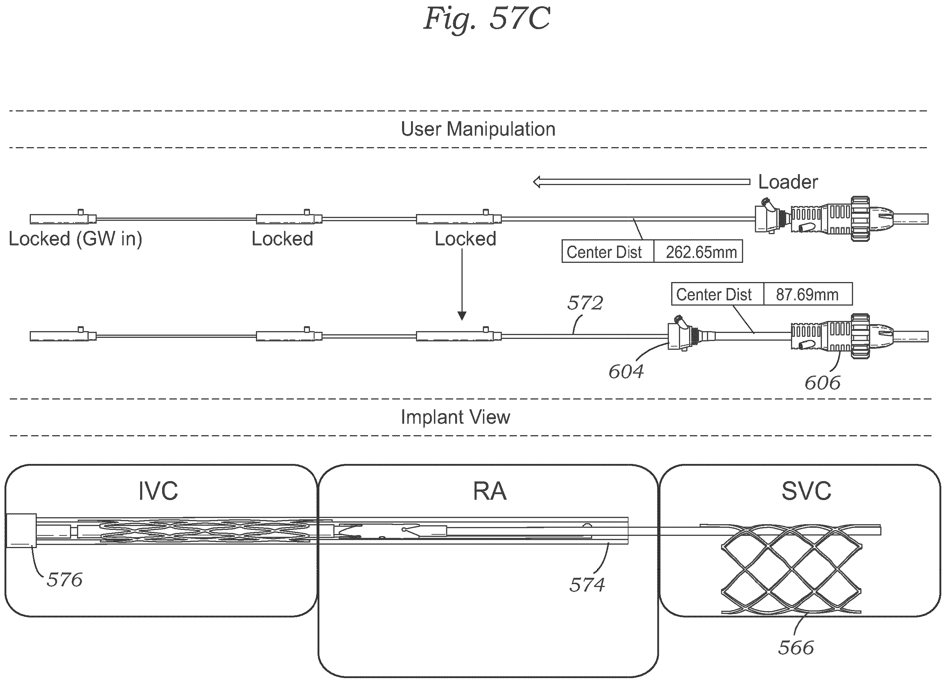

Figure 57C shows partial retraction of the loader 604 by about 8.5 cm relative to the hub 606 and delivery sheath 576 over the pusher shaft 572. The distal end of the constriction sheath 574 remains in the right atrium RA, but the distal stabilizing stent 566 expands within the superior vena cava SVC. The distance that the constriction sheath 574 retracts may be around 8.5 cm. The delivery sheath 576 remains in place.

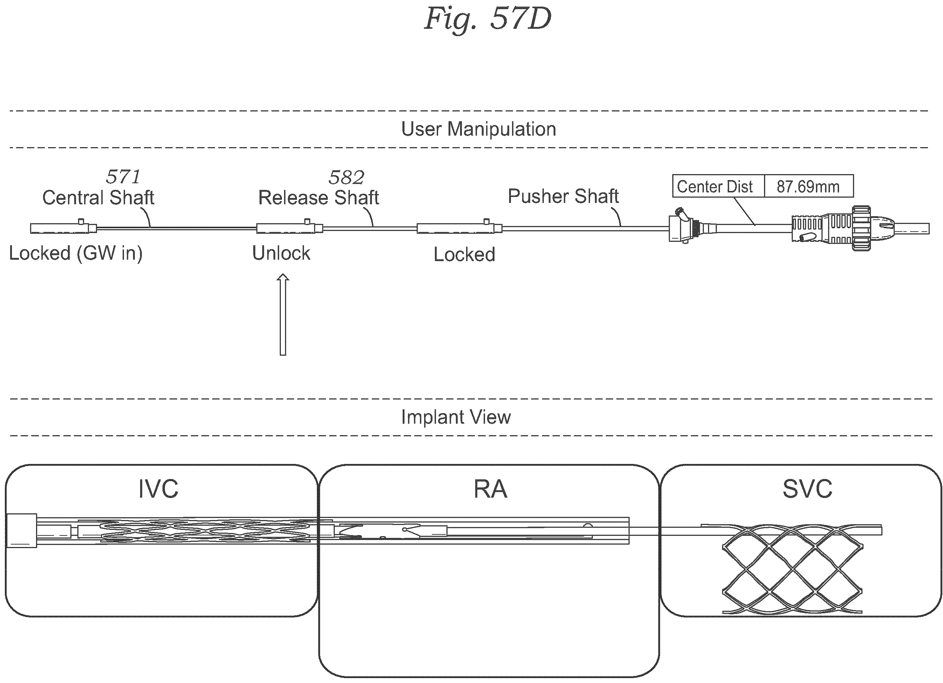

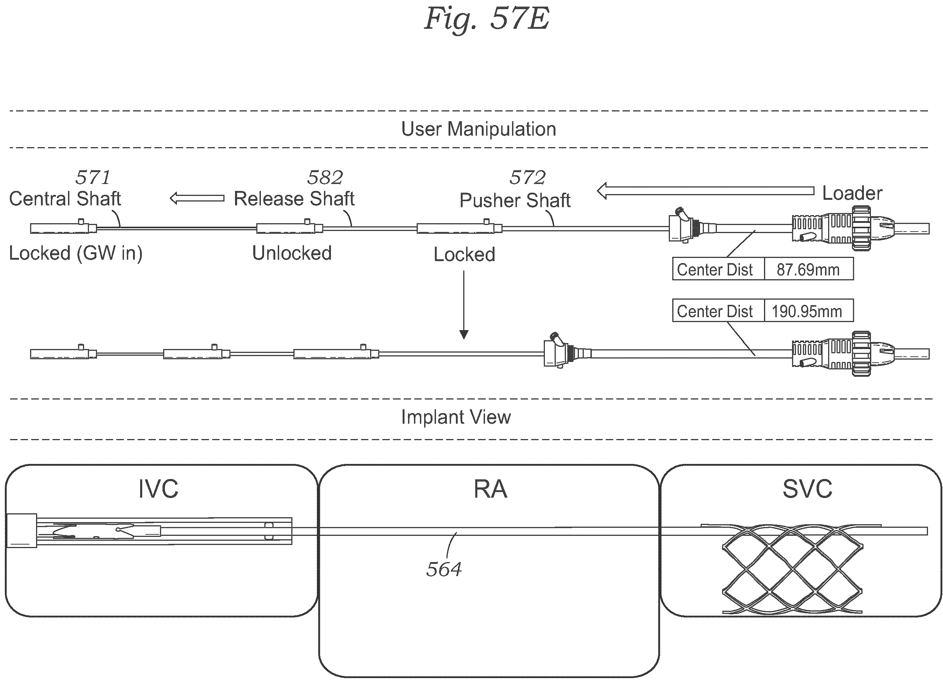

The next step, shown in Figure 57D, involves unlocking the release shaft 582 from around the central shaft 571. Figure 57E then indicates retraction of the release shaft 582 by about 12.5 cm over the central shaft. Due to a lock within the loader onto the pusher shaft 572, this action retracts every component of the system aside from the central shaft 571, caval spanning bar 564 and distal stabilizing stent 566. The total distance of retraction may be around 12.5 cm. As seen in the lower portion of Figure 57E, the distal end of the constriction sheath 574 remains in the IVC.

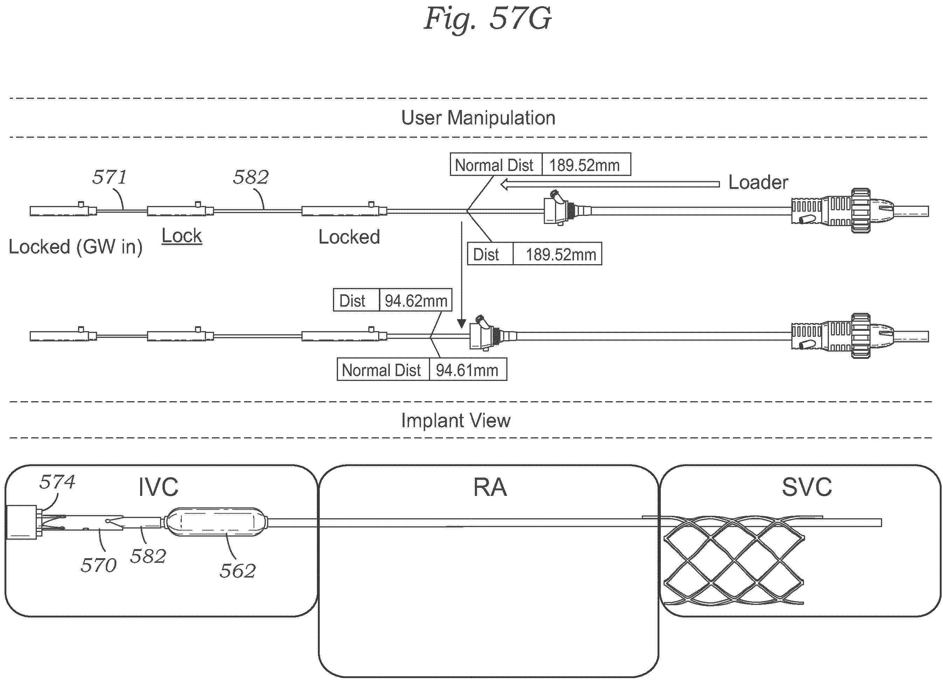

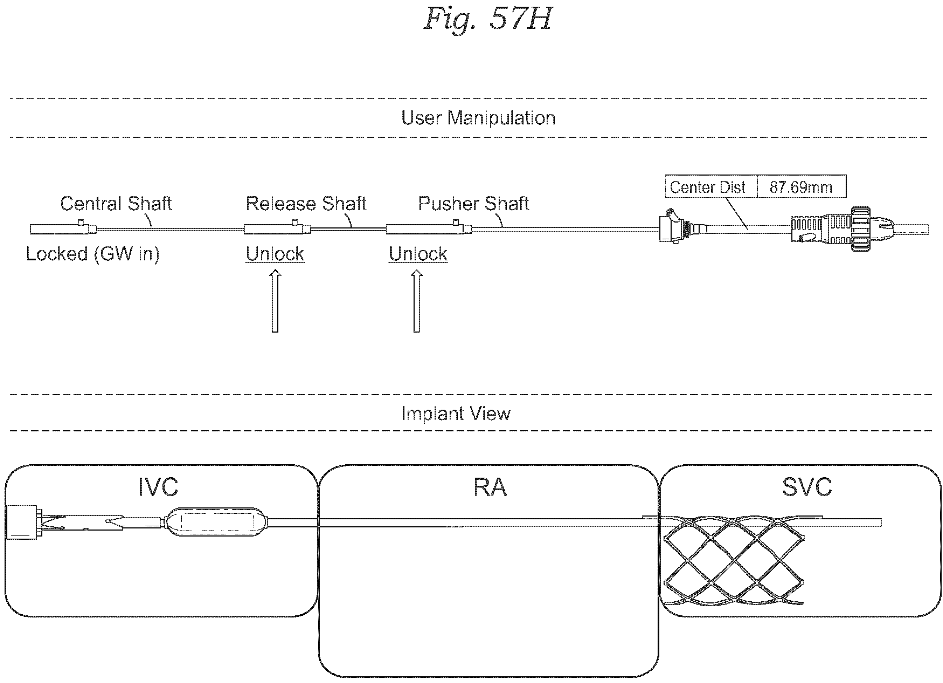

Subsequently, Figures 57G and 57H illustrate first locking of the Tuohy-Borst between the release shaft 582 and the central shaft 571, and then retraction of the loader 604 over the pusher shaft 572 by about 21 cm, which also retracts the constriction sheath 574. This exposes the spacer 562 which self-expands to its implanted shape within the interior vena cava IVC. The spacer 562 is desirably a compressible cylindrical shape with rounded ends. Such a spacer is disclosed in Figure 28 of U.S. Patent No. 9,474,605, which discloses an open cell foam interior covered with a flexible outer cover desirably formed of a polycarbonate urethane (e.g., Carbothane from Lubrizol, Bionate from DSM, ChronoFlex from Advansource). In such a configuration, removal of the constriction sheath 574 around the compressed spacer 562 enables the foam inner to expand the spacer to its implanted shape. Of course, a variety of configurations are possible for use as the spacer 562, including most of the other embodiments disclosed in U.S. Patent No. 9,474,605, which has been incorporated by reference herein.

Retraction of the constriction sheath 574 also exposes the slider coupling 570 which remains in contact with the release shaft 582.

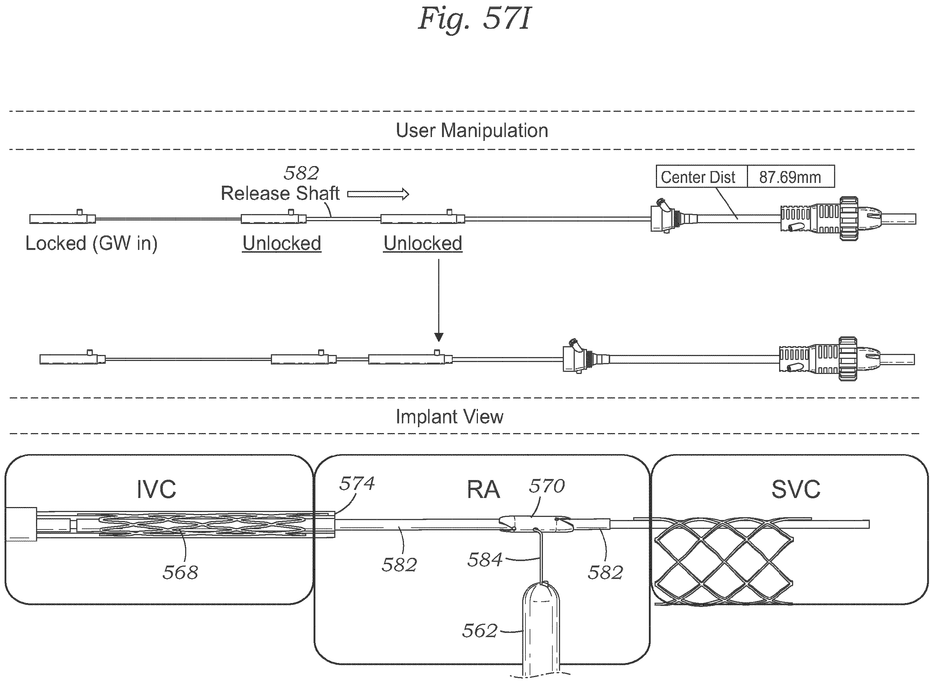

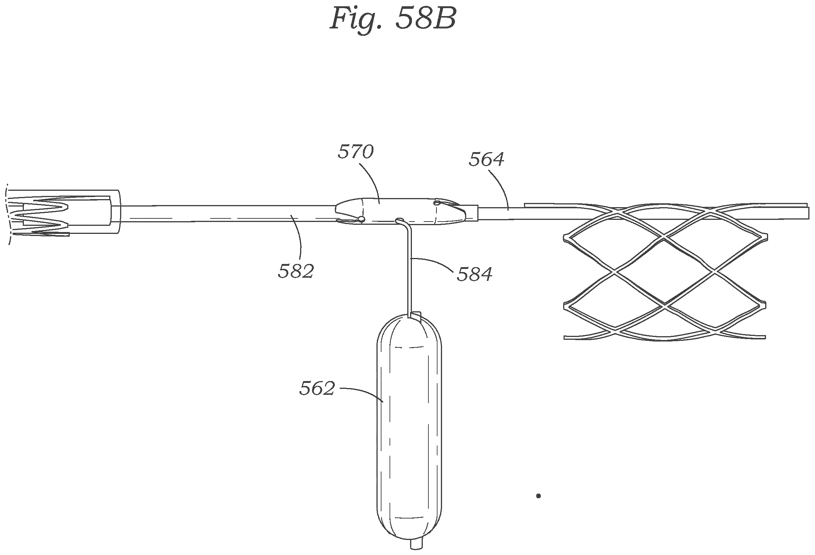

Next, as seen in Figure 57H, the device locks for both the pusher shaft 572 and release shaft 582 are unlocked. Figure 57I then shows advancement of the release shaft by anywhere between 5-10 cm over the central shaft 571. Since the slider coupling 570 remains secured on the release shaft 582, it also advances from the interior vena cava IVC into the right atrium RA. This action results in a spacer "flop," or in other words release of the spacer 562 to fall away from the longitudinal axis of the anchor system 560. Figures 58A and 58B show this transition in greater detail. Reference is also made to Figures 55A/55B which show the suspension member 584 attached to the slider coupling 570 at a hinge 586. Again, the suspension member 584 may be formed homogeneously in one piece with the slider coupling 570, or may be a separate element connected via the hinge 586. It should be noted that the suspension member 584 is located to the outside of the distal end of the release shaft 582, and thus there is no impediment to its pivoting movement. The spacer 562 is free to "flop" in this regard in the interior vena cava IVC once the constriction sheath 574 has been retracted, but will be constrained by the surrounding IVC and only properly released once it is advanced into the right atrium RA.

Figure 57I also shows advancement of the constriction sheath 574 along with the proximal stabilizing stent 568 by virtue of advancement of the central shaft 571. However, the release shaft 582 is advanced farther than the central shaft 571, resulting in spacing between the slider coupling 570 and the distal end of the constriction sheath 574.

During the advancement of the release shaft 582 shown in Figure 57I, the proper placement of the spacer 562 within the atrioventricular valve is determined. That is, the operation proceeds while the heart is beating, and the position of the spacer 562 relative to the valve is seen under visualization. This may involve tracking of the spacer 562 using radiopaque markers on the spacer, but more importantly using fluoroscopic contrast dye injected upstream of the atrioventricular valve. By visualizing the amount of regurgitation through the valve while adjusting the position of the spacer 562, the proper positioning to reduce the regurgitation to a minimum may be determined.

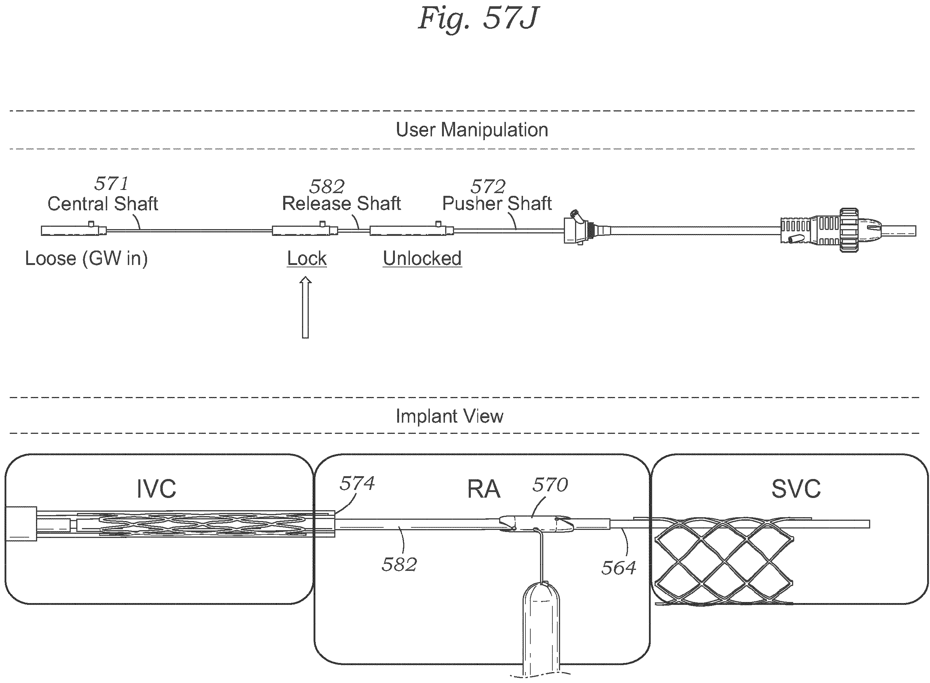

Ultimately, when the position of the spacer 562 is set, it is locked into place. Prior to the release shaft 582 being retracted from within the slider coupling 570, the pusher shaft 572 is advanced to hold the slider coupling stationary, as seen in Figures 57J-57M.

Figure 57J shows locking of the Tuohy-Borst between the release shaft 582 and the central shaft 571. This permits advancement of the pusher shaft 572 and constriction sheath 574 over the release shaft 582. The pusher shaft 572 is advanced into contact with the slider coupling 570.

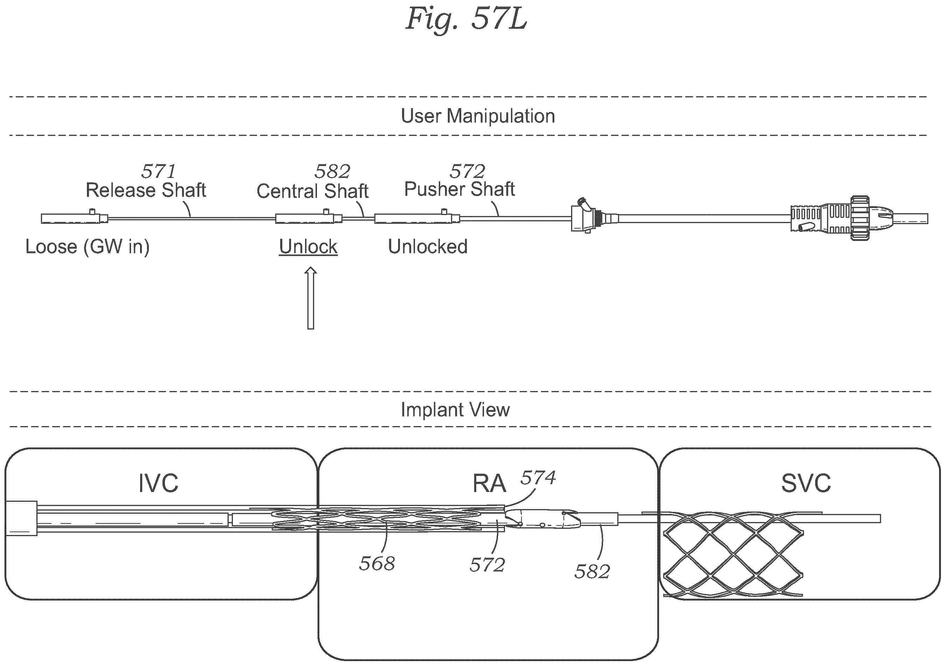

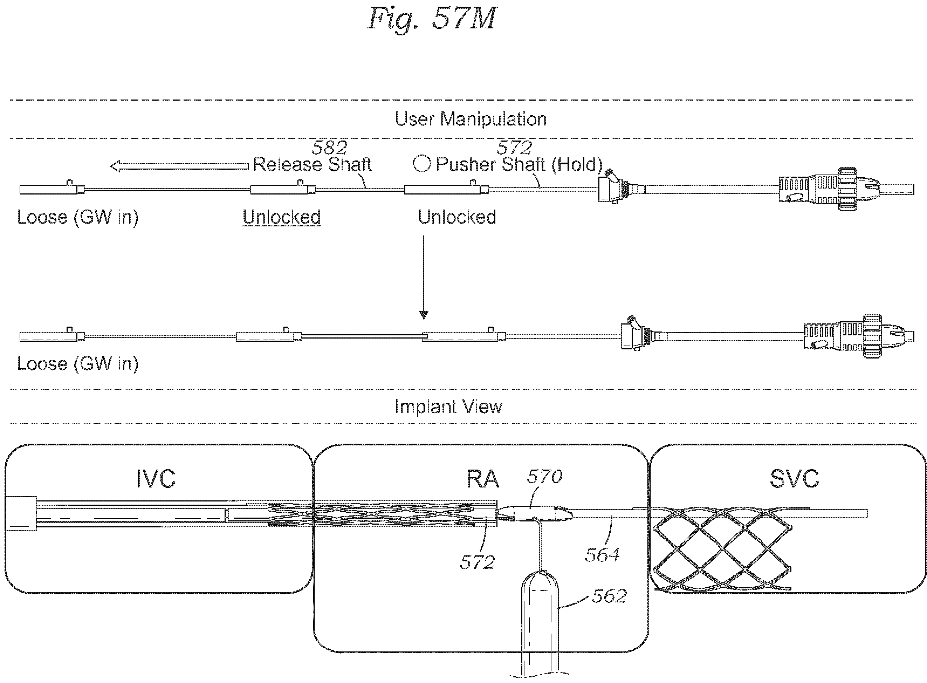

Figure 57L then shows unlocking of the Tuohy-Borst between the release shaft 582 and the central shaft 571. Figure 57M illustrates retraction of the release shaft 582 while holding the pusher shaft 572 stationary. Because the pusher shaft 572 is held in contact with the slider coupling 570, the release shaft 582 can be withdrawn from within the slider coupling. Reference back to Figures 55A and 55B is made to explain the exemplary constriction of the flexible fingers or petals 580 at both ends of the slider coupling 570 onto the caval spanning bar 564, which again is formed by a segment of the central tube 571. This fixes the lateral position of the regurgitation reduction spacer 562 within the right atrium RA, and within the atrioventricular valve leaflets.

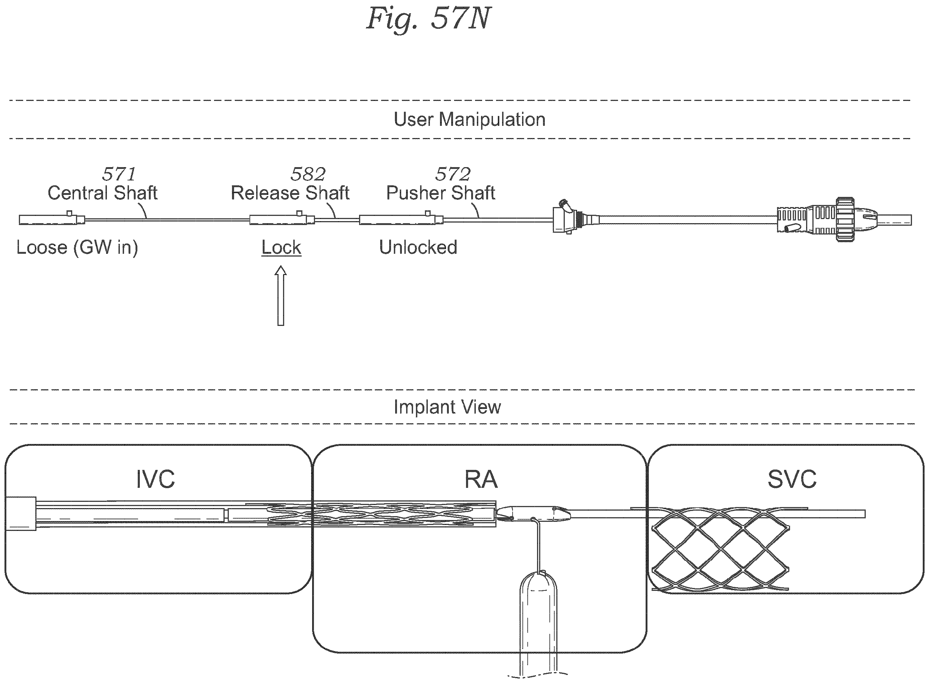

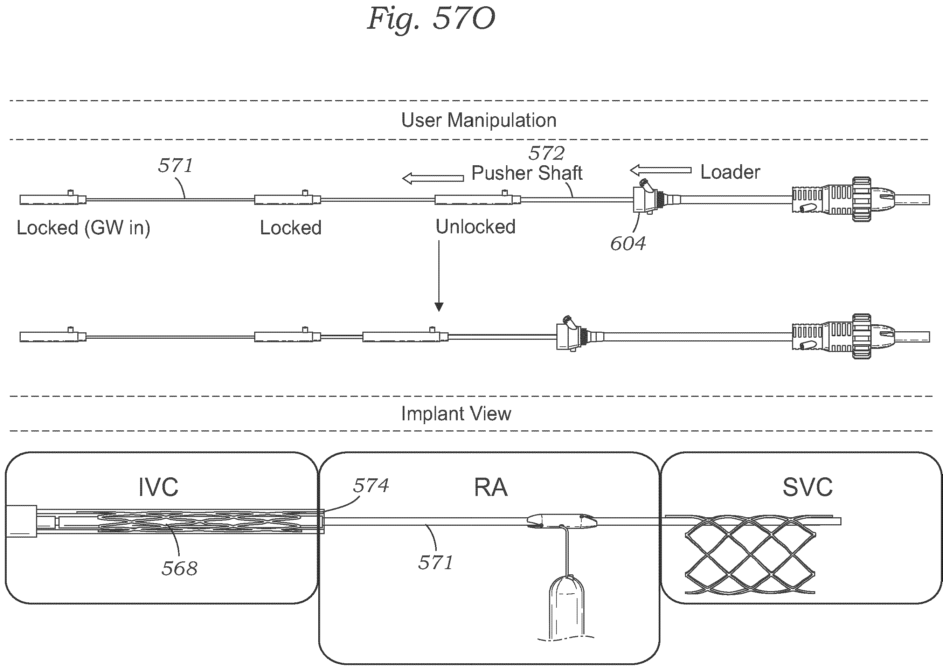

Once the release shaft 582 has been retracted far enough within the pusher shaft 572, it is locked onto the central shaft 571 as seen in Figure 57N. The pusher shaft 572 and loader 604 along with constriction sheath 574 are retracted, as in Figure 57O, so as to position the proximal stabilizing stent 568 within the inferior vena cava IVC.

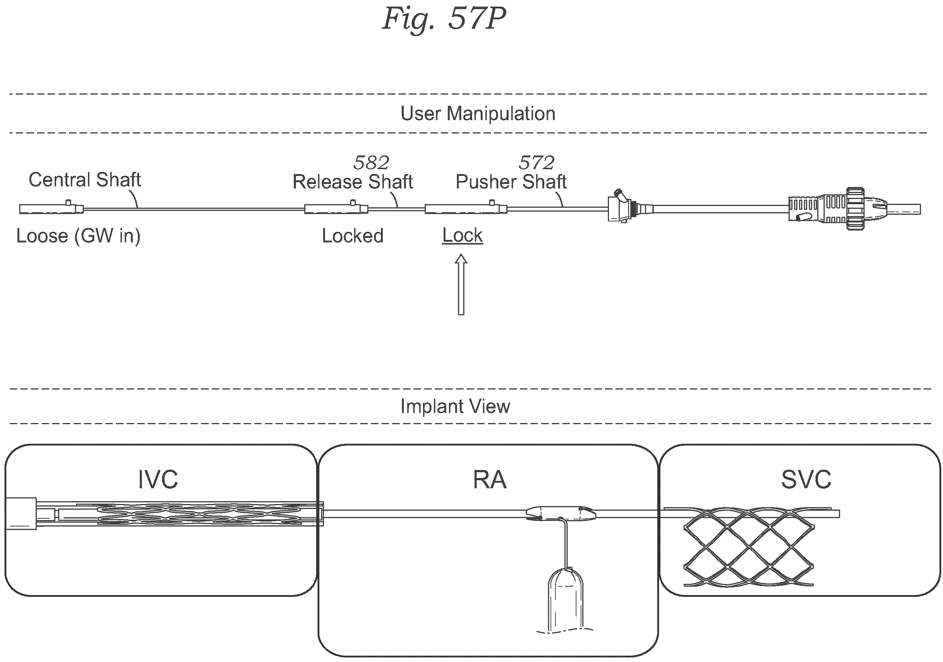

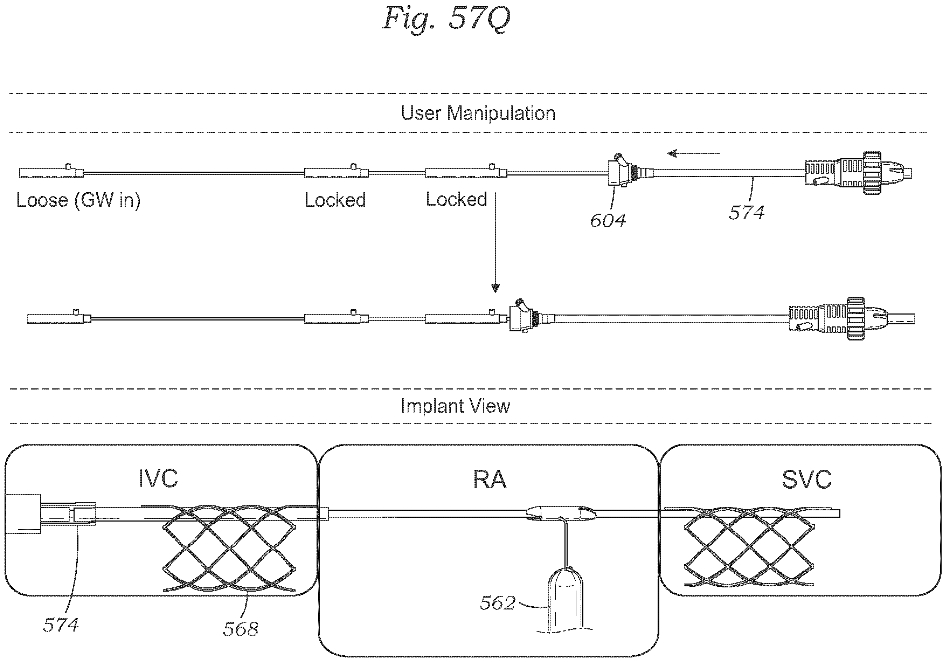

Subsequently, the pusher shaft 572 is locked relative to the release shaft 582, as in Figure 57P. Retraction of the loader 604 pulls the constriction sheath 574 from around the proximal stabilizing stent 568, thus permitting it to expand within the inferior vena cava IVC, as seen in Figure 57Q. This secures the position of the implanted system 560 across the right atrium RA, with the spacer 562 in a position that maximizes regurgitation reduction as verified by visualization, described above.

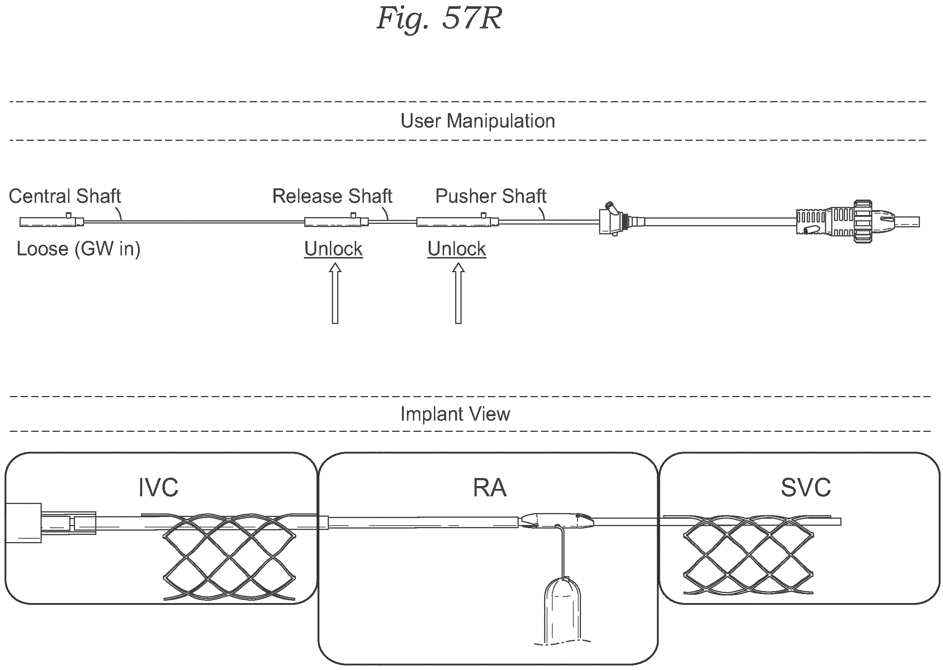

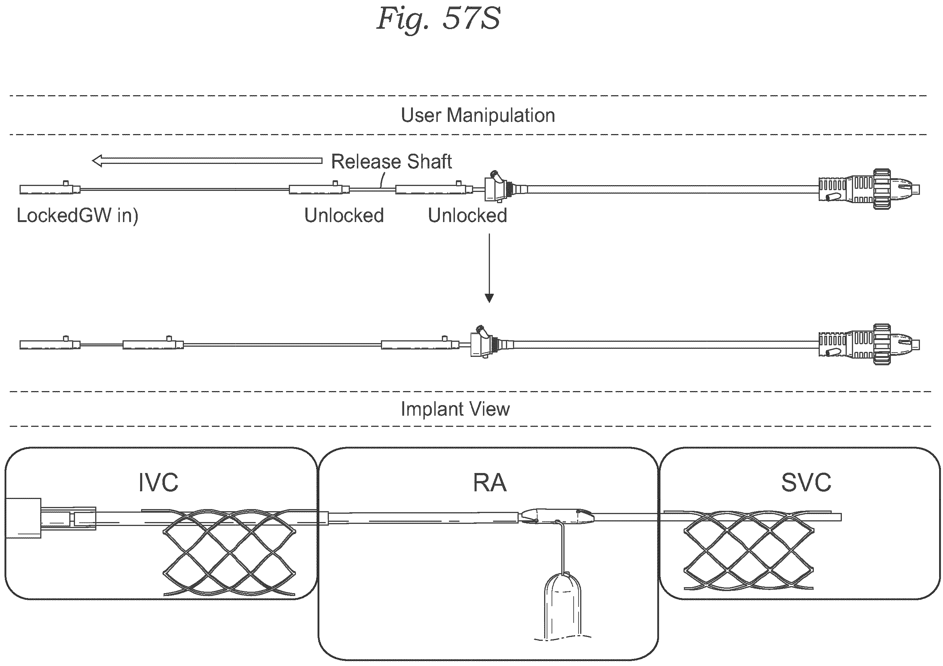

The procedure then involves decoupling the central shaft 571 and the pusher shaft 572 between the proximal delivery ends and the distal implant ends that remain in the body. First, as seen in Figure 57R, the locking devices for both the release shaft 582 and pusher shaft 572 are unlocked. The release shaft 582 is then retracted beyond the junction in the central shaft 571 between its proximal and distal segments, as in Figure 57S. Such a junction may be configured as described above with respect to Figure 56. Relative lateral movement of the proximal and distal segments is sufficient to decouple the two segments, thus leaving just the distal implant segment in the body. In the same way, the proximal and distal segments of the pusher shaft 572 are decoupled after retraction of the constriction sheath 574, leaving the distal segment in the body which is fastened to the proximal stabilizing stent 568. The pusher shaft 572 may also be locked onto the central shaft 571 using a crimp or other such fastener, though the central shaft 571 may simply be left in place extending through the pusher shaft 572. That is, the anchoring of the distal and proximal stabilizing stents 566, 568 may be sufficient to fix the system 560 in place without the additional fixation of the shafts 571, 572. The central shaft 571 would thus be free to slide within the pusher shaft 572, but the relative positions of the stents 566, 568 will remain substantially stable and thus there will be little or no such relative sliding movement of the two shafts.

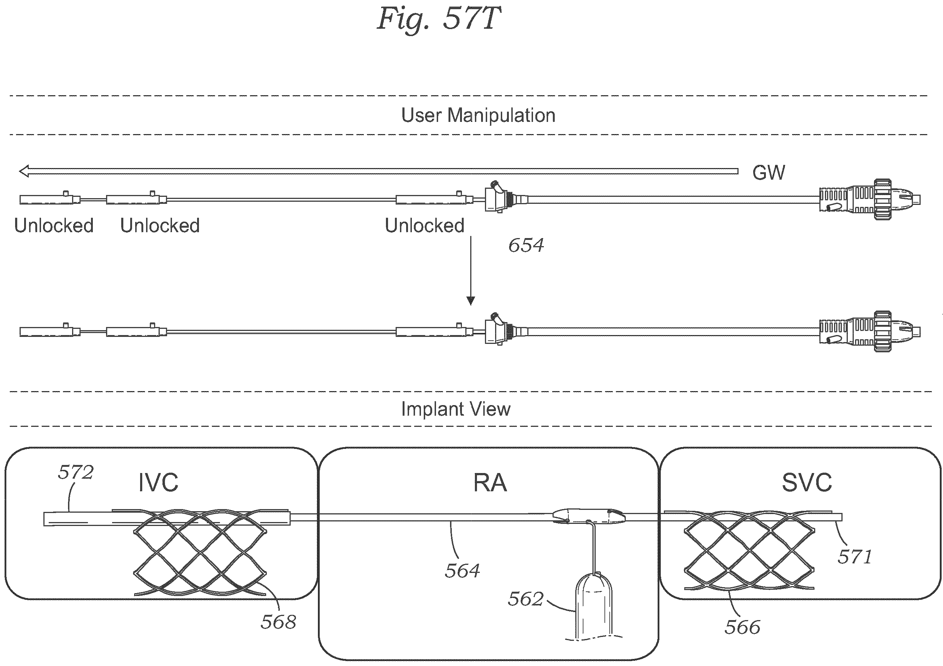

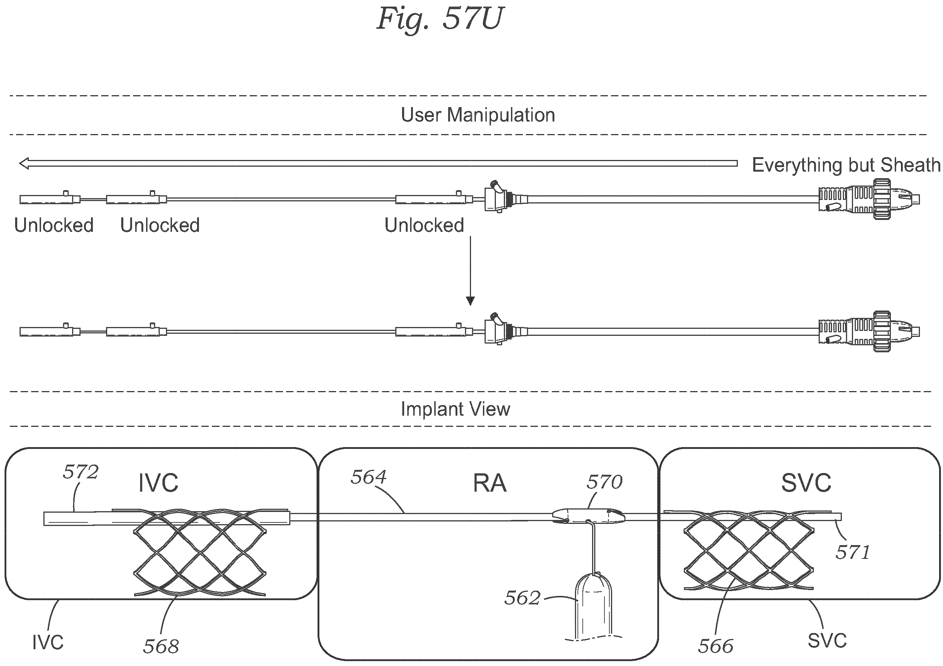

Figure 57T shows removal of the guidewire, and Figure 57U removal of all but the parts of the system 560 that remain in the body. The puncture wound is closed and the patient's atrioventricular valve is repaired.

### Control Handle

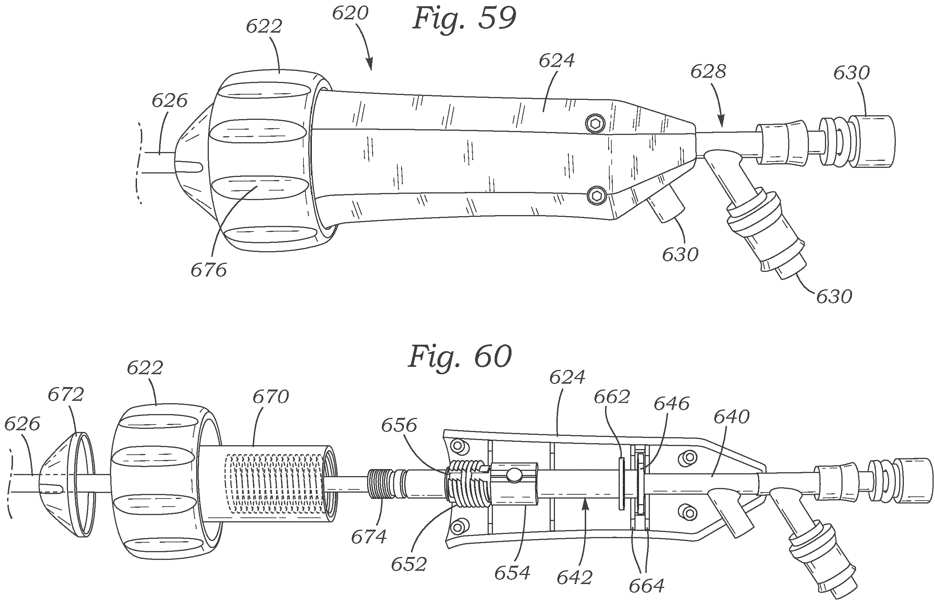

Figure 59 is a perspective view of an exemplary control handle 620 which may be used with various valve regurgitation reduction systems described herein, and Figure 60 is a partial exploded view thereof. In particular, the control handle 620 may be utilized to control the deployment and adjustment of the regurgitation reduction anchoring system 270 shown in Figures 24A and 24B, in which a bend 278 may be formed in flexible shaft 252 by rotating the control ring 277. The control handle 620 of Figure 59 includes a control ring 622 on the distal end of a main housing 624 for bending a flexible shaft 626 extending distally. A Y-fitting 628 on a proximal end provides access ports 630 for passage of instruments such as the tethers 254 that pass through the flexible shaft 252, a guide wire, or irrigation or aspiration tubes, as needed.

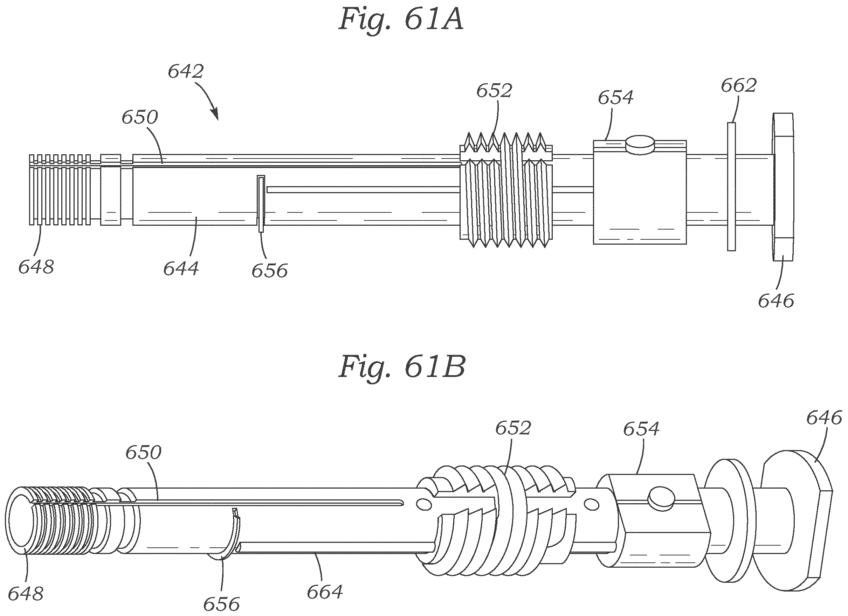

As seen exploded in Figure 60, the main housing 624 may be formed by two halves (one of which is shown) each having a hollow interior and secured together with fasteners. A central access tube 640 extends through the middle of the housing 624 for passage of the various instruments, such as the tethers 254. The access tube 640 extends through a lumen in a control shaft 642 used to bend the distal shaft 626, and which is shown in more detail in Figures 61A and 61B.

With reference to Figures 61A and 61B, the control shaft 642 has an elongated, generally tubular body 644 with a proximal flange 646 and a threaded distal end 648 interrupted by an axial slit 650. A shaft deflection mechanism includes a slide nut 652 and a wire wrapping sleeve 654, both of which comprise short tubular members that may slide axially along the exterior of the control shaft 642. Both the slide nut 652 and sleeve 654 are passed over the distal end 648 of the control shaft 642 and retained thereon by a C-clip 656 positioned within a circumferential groove formed in a midpoint of the control shaft.

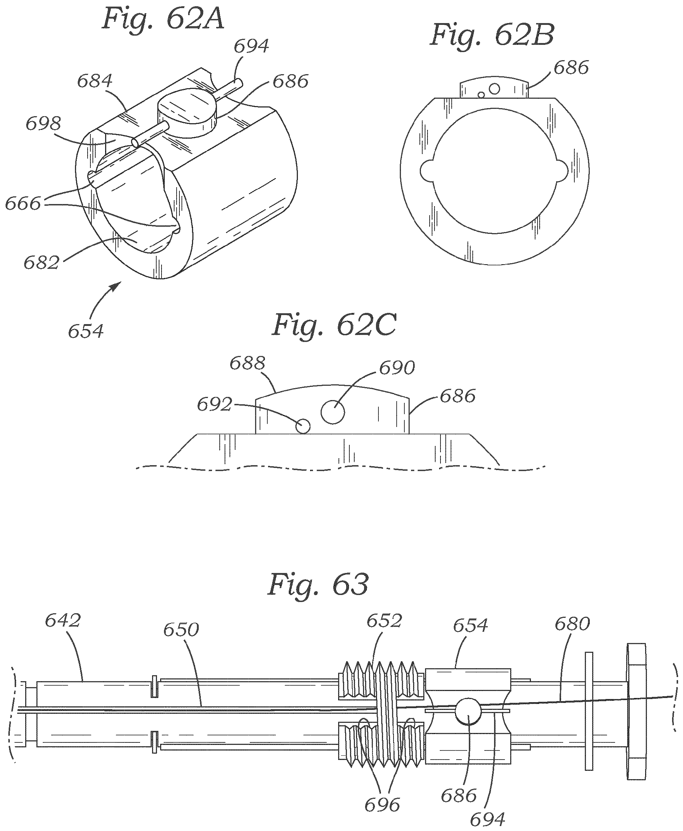

With reference back to Figure 60, the control shaft 642 is secured between the two halves of the main housing 624 by capture of the proximal flange 646 between a pair of bulkheads 660. The control shaft 642 is thus axially fixed within the main housing 624. Both the slide nut 652 and wire wrapping sleeve 654 are free to move axially along the control shaft 642 between the C-clip 656 and a secondary flange 662 on the control shaft. Furthermore, both the slide nut 652 and sleeve 654 have diametrically opposed internal axially-oriented grooves that receive corresponding axial ribs 664 (see Figure 61B) extending outward from the tubular control shaft 642. The grooves 666 in the wire wrapping sleeve 654 are seen in Figure 62A. Consequently, the slide nut 652 and sleeve 654 are constrained from rotation about the axis of the control shaft 642.

The control ring 622 as seen in Figure 60 forms the radially exterior portion of a larger element that interacts with the distal end of the main housing 624. In particular, the control ring 622 is fixed around a tubular shaft section 670 having internal threading that mates with external threading on the slide nut 652. The tubular shaft section 670 extends within the inner cavity of the main housing 624 and causes axial displacement of the slide nut 652 when rotated. In this regard, the assembly of the control ring 622 and shaft section 670 is free to rotate relative to the housing 624 and is constrained thereon by a conical end cap 672, preferably having internal threading that mates with external threading 674 on the distal end 648 of the control shaft 642.

### Deflection Wire Wrapping

Bending of the flexible shaft 626 requires a considerable amount of force applied to a deflection wire. As with many flexible catheters, the deflection wire 680 extends from the proximal control handle 620 and is secured to a fixation point on one side of the distal end of the flexible shaft 626. By pulling on the deflection wire 680, the flexible shaft 626 is caused to deflect in the direction corresponding to the side to which the wire is fixed. By rotating the flexible shaft 626 about its own axis, deflection in any direction is possible. However, given the relatively robust nature of the flexible shaft 626 and its components, such as a guide rail therein, a large tension in the wire is required to bend the shaft. The control handle 620 is thus configured to apply the requisite amount of tension without failure. This enabled partly by a relatively large control ring 622 having gripping features such as axial indents 676 in combination with a faceted housing 624, but also by virtue of a novel arrangement for wrapping a proximal end of the deflection wire to the wire wrapping sleeve 654, as will be described below.

Figures 62A-62C are several views of the wire wrapping sleeve 654 used to secure a proximal end of a deflection wire 680, seen assembled on the control shaft 642 in Figure 63. As mentioned, the sleeve 654 has a short, generally tubular configuration with a central lumen 682 received on the control shaft 642 and having the internal axial anti-rotation grooves 666. A chordal section on the tubular sleeve 654 is removed on one side to create a flat 684. A cylindrical boss 686 extends radially outward from the flat 684 and has an outer arcuate surface 688 that matches the outer diameter of the rest of the tubular sleeve 654.

As seen best in Figure 62C, a pair of axial through bores 690, 692 pass through the cylindrical boss 686. A large through bore 690 extends through the circumferential centerline of the boss 686, while a smaller through bore 692 is formed offset from the centerline and immediately adjacent to the flat 684. As seen in Figure 62A, a rigid, preferably metallic, pin 694 is secured within the larger through bore 690 and extends axially beyond the boss 686.

Now with reference again to Figure 63, the deflection wire 680 that is attached to the distal end of the flexible shaft 626 extends into the lumen of the control shaft 642 and emerges radially from the axial slit 650 just distal with respect to the wire wrapping sleeve 654. The deflection wire 680 is accommodated by axial cutouts 696 in the slide nut 652 and extends to the outside of the flat 684 and through the smaller through bore 692. To help avoid a hard lip over which the deflection wire 680 extends and to reduce friction, the distal end of the flat 684 includes an arcuate cutout 698, as seen in Figure 62A.

As will be explained further below, the deflection wire 680 is wrapped around the upstanding boss 686 and rigid pin 694 in a manner that anchors it securely to the wire wrapping sleeve 654. As explained above, rotation of the assembly of the control ring 622 and shaft section 670 causes axial displacement of the slide nut 652. Proximal displacement of the slide nut 652 urges the sleeve 654 proximally due to the direct contact therebetween, thus pulling on the deflection wire 680. In this way, rotation of the control ring 622 deflects the distal end of the flexible shaft 626. As mentioned above, the magnitude of tension on the deflection wire 680 can be significant, thus necessitating a robust wire as well as a robust arrangement for anchoring the wire to the sleeve 654.

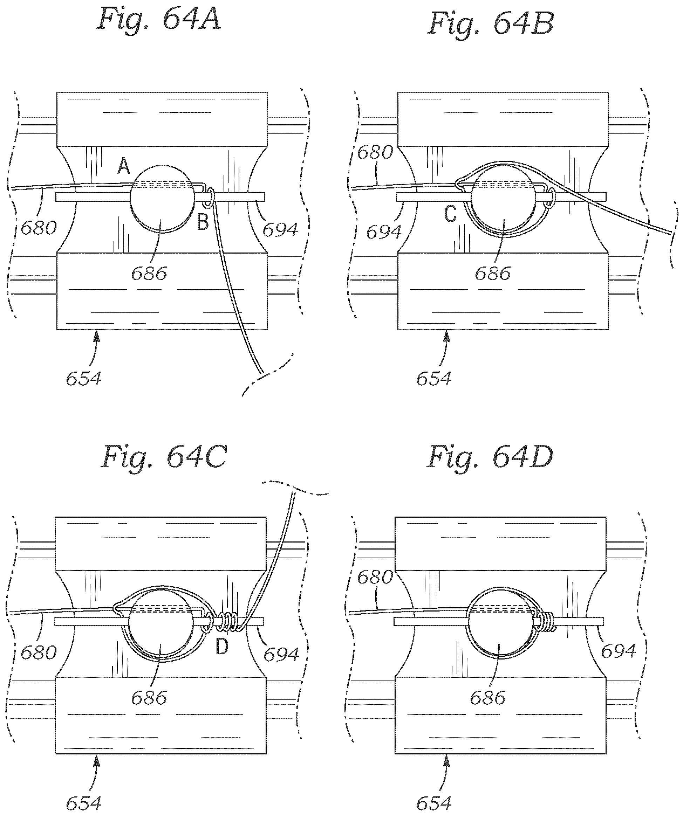

Figures 64A-64D are snapshots of an exemplary sequence for wrapping the deflection wire 680 around the wire wrapping sleeve 654 in a preferred manner that ensures no relative slippage of the wire and sleeve. Initially, as explained, the wire 680 passes through the smaller through bore 692 of the boss 686, as indicated at step A in Figure 64A. The assembler then wraps the wire 680 around the proximal portion of the rigid pin 694, as seen at step B. In Figure 64B, the assembler then wraps the wire 680 in a clockwise direction entirely around the boss 686, passing the wire underneath the distal portion of the rigid pin 694 and over the portion of the wire that extends in a distal direction, as seen at step C. Subsequently, at step D seen in Figure 64C, the wire is again wound at least three times around the proximal portion of the rigid pin 694. Figure 64D shows the completed wire wrapping arrangement after pulling taut the wire 680 and trimming off the end close to the rigid pin 694.

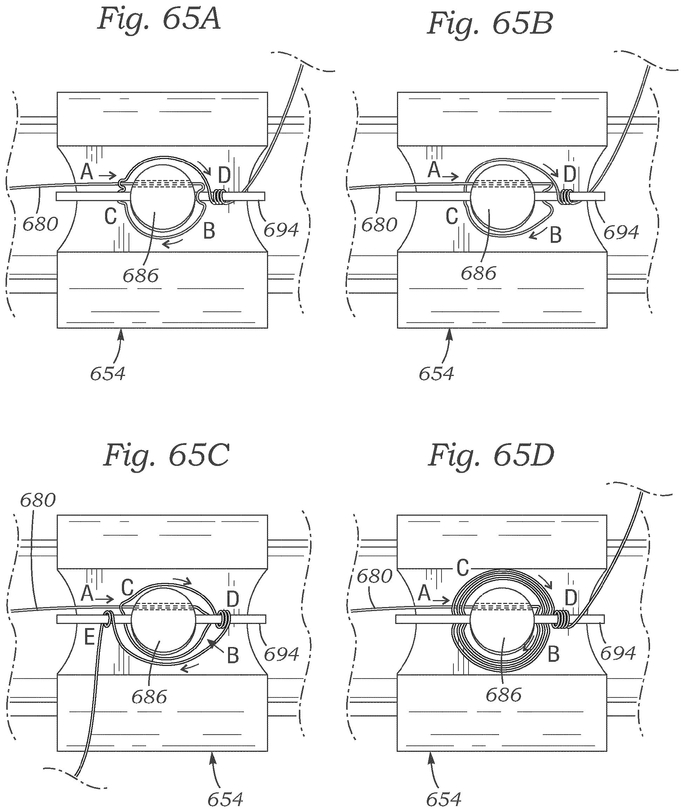

Figures 65A-65D are views showing alternative sequences for wrapping the deflection wire 680 around the wire wrapping sleeve 654. In each wire wrapping variation, the sequence of steps is indicated by alphabetical labels. The wire 680 is shown loosely wrapped in each of these alternatives for the sake of explanation, but would be pulled taut and trimmed in each for the finished product.

Figure 65A shows a sequence similar to that described above with respect to Figures 64A-64D, without the first wrap around the proximal portion of the rigid pin 694 at step B. Instead, the wire is simply passed over the top of the rigid pin 694.

Figure 65B is much the same as the sequence of Figure 65A, however the wire 680 passes under both the distal portion of the rigid pin 694 and itself at step C when being wrapped clockwise around the upstanding boss 686.

In Figure 65C the assembler first passes the wire 680 underneath proximal portion of the rigid pin 694 at step B. The wire is then wrapped clockwise around the boss 686, passing under the distal portion of the pin 694 and over itself at step C. The assembler then loops the wire 680 at least twice around the proximal portion of the pin 694 at step D, and again wraps it clockwise halfway around the boss 686, finishing off with at least one wrap around the distal portion of the pin 694, at step E.

Finally, Figure 65D shows a wrapping configuration in which the assembler wraps the wire 680 three times clockwise around the boss 686 after emerging from the smaller through bore 692. Each of these wraps includes passing first under the distal and proximal portions of the rigid pin 694 and over the wire at steps B and C. Finally, the assembler wraps the wire 680 at least three times around the proximal portion of the rigid pin 694.

Each of the wire wrapping configurations shown and described above with respect to Figures 64-65 provides good tensile strength to the anchor, sufficient to deflect the distal end of the flexible shaft 626. Testing indicates some variation, and a preference for the first-described wrapping configuration of Figures 64A-64D. In particular, repetitive tests of wire indicated no slippage prior to an average break force of the wire of around 150 N, which provides a significant safety margin for the deflection.

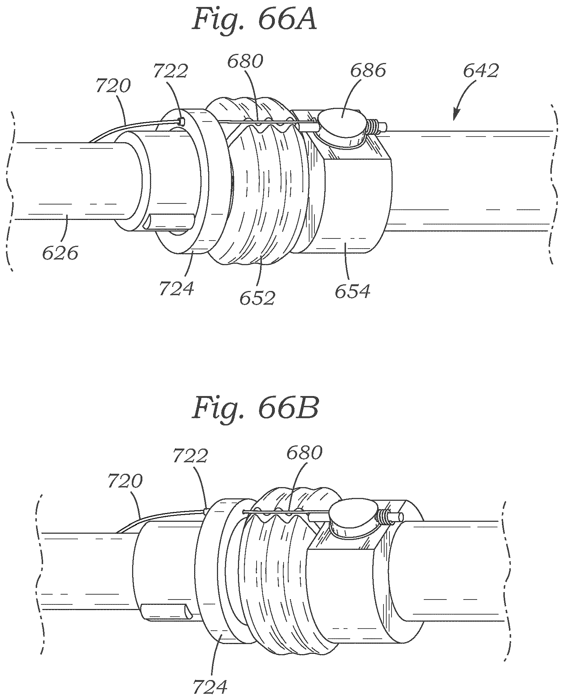

Figures 66A and 66B are perspective views of an alternative assembly of the control shaft 642, slide nut 652 and wire wrapping sleeve 654 which may be used to produce a more acute bend at the distal end of the flexible shaft 626. Namely, a cable sheath 720 surrounds the deflection wire 680 from the control handle to the distal end of the flexible shaft 626. The cable sheath 720 terminates in a ferrule 722 positioned within a distally-facing bore in a stop ring 724. Although not shown, the stop ring 724 is fixed with respect to the control shaft 642. The deflection wire 680 continues a proximal direction is wrapped around the boss 686 of the sleeve 654, as described above. Also not shown, the deflection wire 680 emerges from the cable 720 at the distal end of the flexible shaft 626 and attaches to the shaft. Pulling on the deflection wire 680 can thus create a much tighter bend in the flexible shaft then without the cable 720. This arrangement is akin to a standard brake cable on a bicycle.

The assembly shown in Figures 66A and 66B allows the force from deflection activation to be specifically delivered to a localized zone. This may be used when it is desired to locate the bend in the flexible shaft 626 at a specific region or location, or if there is a need to keep a portion of the catheter unaffected by the activation of a deflection wire. The cable sheath 720 remains completely flexible even when the deflection wire 680 is completely activated under tension. This allows the distal end of the shaft 626 to bend at >90 degrees while the majority of the shaft remains soft and flexible. The cable sheath 720 needs to have some extra free-moving length that can add/remove slack as a catheter is flexed. This is done by wrapping the cable sheath 720 approximately 90-180° helically around the flexible shaft 626, as shown.

While the foregoing is a complete description of the preferred embodiments of the invention, various alternatives, modifications, and equivalents may be used. Moreover, it will be obvious that certain other modifications may be practiced within the scope of the appended claims.

The invention further comprises the following embodiments:
1. A system for anchoring an atrioventricular spacer within an atrioventricular valve between atrium and a ventricle, the valve having leaflets extending inward from a peripheral tissue annulus, comprising:
   a compressible and expandable spacer having a proximal end and a distal end and being sized to fit within the leaflets of the atrioventricular valve and configured to coapt against the leaflets to reduce regurgitation therebetween, the spacer having a long dimension with a length such that the proximal end resides within the atrium and the distal end resides within the ventricle, the spacer having a compressed delivery configuration and a radially expanded implant configuration;
   a suspension member connected to the proximal end of the spacer and extending proximally therefrom;
   a tubular slider coupling defining a longitudinal axis to which the suspension member is hingedly connected such that the long dimension of the spacer may be oriented parallel to the longitudinal axis or pivoted away from the longitudinal axis;
   a central shaft disposed concentrically through the slider coupling and adapted for sliding longitudinal movement relative to the slider coupling, the slider coupling having a locking mechanism that, when activated, prevents longitudinal movement of the slider coupling relative to the central shaft, the central shaft having a distal implant segment and a proximal delivery segment joined at a separable junction, wherein the segments may be decoupled to leave just the distal implant segment of the central shaft in the body;
   a distal stabilizing element fixedly attached to the central shaft on a distal side of the slider coupling, the distal stabilizing element having a compressed delivery configuration and a radially expanded implant configuration adapted to anchor in a vascular ostia opening to the atrium;
   a pusher shaft concentrically disposed around and slidable relative to the central shaft, wherein the pusher shaft is shorter than the central shaft and has a locking member at a proximal end adapted to alternately lock and release the pusher shaft for sliding movement relative to the central shaft, the pusher shaft having a distal implant segment and a proximal delivery segment joined at a separable junction, wherein the segments may be decoupled to leave just the distal implant segment of the pusher shaft in the body;
   a proximal stabilizing element fixedly attached to the pusher shaft on a proximal side of the slider coupling, the proximal stabilizing element having a compressed delivery configuration and a radially expanded implant configuration adapted to anchor in a vascular ostia opening to the atrium; and
   a constriction sheath concentrically arranged around the central shaft, distal stabilizing element, pusher shaft, proximal stabilizing element and spacer, the constriction sheath being oriented along the longitudinal axis and sized to surround and maintain the spacer, distal stabilizing element and proximal stabilizing element in their compressed delivery configurations, and wherein
   the central shaft has a caval spanning segment with a length between the distal stabilizing element and proximal stabilizing element sufficient to span across an atrium between two vascular ostia opening to the atrium, such that the spacer may be pivoted away from the longitudinal axis of the slider coupling and suspended within the atrioventricular valve so as to coapt against the leaflets and reduce regurgitation therebetween.
2. The system of embodiment 1, further including a release shaft concentrically disposed around the central shaft and within the pusher shaft and slidable relative to both the central shaft and the pusher shaft, wherein the release shaft is shorter than the central shaft and longer than the pusher shaft and has a locking member at a proximal end that acts directly on the central shaft and is adapted to alternately lock and release the release shaft for sliding movement relative to the central shaft, wherein the locking member of the pusher shaft acts directly on the release shaft, the release shaft having a distal implant segment and a proximal delivery segment joined at a separable junction, wherein the segments may be decoupled to leave just the distal implant segment of the release shaft in the body.
3. The system of embodiment 2, wherein the slider coupling comprises a generally tubular member having opposite ends and the locking mechanism comprises a plurality of flexible petals on the opposite ends that are biased inward and clamp onto the release shaft, and retraction of the release shaft while holding the slider coupling stationary relative to the central shaft pulls the release shaft from within the slider coupling and permits the petals to clamp onto the central shaft.
4. The system of embodiment 2, wherein the constriction sheath is connected to a proximal hub with a lock that acts directly on the pusher shaft and is adapted to alternately lock and release the constriction sheath for sliding movement relative to the pusher shaft.
5. The system of embodiment 2, wherein the spacer comprises an open cell foam interior covered with a flexible outer cover formed of a polycarbonate urethane.
6. The system of embodiment 5, wherein the distal and proximal stabilizing elements comprise tubular self-expanding Nitinol stents.
7. The system of embodiment 1, further including a delivery sheath concentrically arranged around the constriction sheath, central shaft, distal stabilizing element, pusher shaft, proximal stabilizing element and spacer, the delivery sheath being oriented along the longitudinal axis and sized to advance the constriction sheath with the spacer, distal stabilizing element and proximal stabilizing element in their compressed delivery configurations inside the constriction sheath.
8. The system of embodiment 1, wherein the constriction sheath is connected to a proximal hub with a lock that acts directly on the pusher shaft and is adapted to alternately lock and release the constriction sheath for sliding movement relative to the pusher shaft.
9. The system of embodiment 1, wherein the spacer comprises an open cell foam interior covered with a flexible outer cover formed of a polycarbonate urethane.
10. The system of embodiment 1, wherein the separable junctions of both the central shaft and pusher shaft comprises partial tubular portions adjacent cutout portions on adjacent ends of the respective distal implant segments and proximal delivery segments, the tubular portions fitting into the cutout portions and coupling the segments together when surrounded by an immediately adjacent tubular member.
11. The system of embodiment 1, wherein the distal and proximal stabilizing elements comprise tubular self-expanding Nitinol stents.
12. The system of embodiment 1, wherein the suspension member is hingedly connected to the slider coupling so that the spacer pivots away from the slider coupling in one plane.
13. The system of embodiment 12, wherein the suspension member is formed as a flat strip connected at a living hinge to the slider coupling to provide rotational stability.
14. A system for anchoring an atrioventricular spacer within an atrioventricular valve between an atrium and a ventricle, the valve having leaflets extending inward from a peripheral tissue annulus, comprising:
   a compressible and expandable spacer having a proximal end and a distal end and being sized to fit within the leaflets of the atrioventricular valve and configured to coapt against the leaflets to reduce regurgitation therebetween, the spacer having a long dimension with a length such that the proximal end resides within the atrium and the distal end resides within the ventricle, the spacer having a compressed delivery configuration and a radially expanded implant configuration;
   a plurality of tethers connected to and extending in an outward array from the spacer generally perpendicular to the long dimension; and
   a tissue anchor on a free end of each of the tethers, each tissue anchor being configured to be secured to the tissue annulus and each tether having a variable length to enable the plurality of tethers to be adjusted to be taut between the tissue annulus and the spacer so that the array of the tethers around the spacer holds the spacer centrally within the valve leaflets.
15. The system of embodiment 14, wherein each tether connects to the proximal end of the spacer at a central axis thereof.
16. The system of embodiment 15, further a plurality of ventricular tethers connected to and extending from the distal end of the spacer at a central axis thereof, and a ventricular anchor on a free end of each of the ventricular tethers, each ventricular anchor being configured to be secured to tissue within the ventricle and each tether having a variable length to enable the plurality of ventricular tethers to be adjusted to be taut between the ventricle and the spacer so that the array of the ventricle tethers around the spacer holds the spacer within the valve leaflets.
17. The system of embodiment 14, wherein each tether extends across from one side to an opposite side of the peripheral tissue annulus and bifurcates in a mid-portion in a loop-like snare sized to receive the spacer in the compressed delivery configuration, wherein the snares are delineated by junctions, and wherein expansion of the spacer to the radially expanded implant configuration tightens the snares and forms a harness around the spacer that holds the spacer centrally within the valve leaflets.
18. A system for anchoring an atrioventricular spacer within an atrioventricular valve between an atrium and a ventricle, the valve having leaflets extending inward from a peripheral tissue annulus, comprising:
   a compressible and expandable spacer having a proximal end and a distal end and being sized to fit within the leaflets of the atrioventricular valve and configured to coapt against the leaflets to reduce regurgitation therebetween, the spacer having a long dimension with a length such that the proximal end resides within the atrium and the distal end resides within the ventricle, the spacer having a compressed delivery configuration and a radially expanded implant configuration;
   a plurality of curved struts attached to and extending in an outward array from the proximal end of the spacer and having a length to reach the tissue annulus; and
   a tissue anchor on a free end of each of the curved struts, each tissue anchor being configured to be secured to the tissue annulus so that the array of the curved struts around the spacer holds the spacer centrally within the valve leaflets.
19. The system of embodiment 18, wherein each curved strut emanates from an annular hub attached to the proximal end of the spacer, and each strut first extends in a proximal direction and then gradually curves outward and around so as to end up extending generally in a distal direction.
20. The system of embodiment 19, wherein each curved strut terminates in an inwardly bent eyelet to which one of the anchors attaches.

## Claims

1. A system for anchoring an atrioventricular spacer (250) within an atrioventricular valve between an atrium and a ventricle, the valve having leaflets extending inward from a peripheral tissue annulus, comprising:
a compressible and expandable spacer (250) having a proximal end and a distal end and being sized to fit within the leaflets of the atrioventricular valve and configured to coapt against the leaflets to reduce regurgitation therebetween, the spacer (250) having a long dimension with a length such that the proximal end resides within the atrium and the distal end resides within the ventricle, the spacer (250) having a compressed delivery configuration and a radially expanded implant configuration;
a plurality of tethers (254) connected to and extending in an outward array from the spacer (250); and
a tissue anchor (256) on a free end of each of the tethers (254), each tissue anchor (256) being configured to be secured to tissue and each tether having a variable length to enable the plurality of tethers (254) to be adjusted to be taut between the tissue and the spacer (250) so that the array of the tethers (254) around the spacer (250) holds the spacer (250) centrally within the valve leaflets.

2. The system of claim 1, wherein at least some of the tissue anchors (256) are configured to be secured to the tissue annulus.

3. The system of any one of claims 1 or 2, wherein at least some of the tethers (254) connect to the proximal end of the spacer (250) at a central axis thereof.

4. The system of any one of the preceding claims, wherein at least some of the tethers (254) are ventricular tethers (258) connected to and extending from the distal end of the spacer (250) at a central axis thereof, and at least some of the tissue anchors (256) are ventricular anchors (260) provided on a free end of each of the ventricular tethers (258), each ventricular anchor (260) being configured to be secured to tissue within the ventricle and each ventricular tether having a variable length to enable the plurality of ventricular tethers (258) to be adjusted to be taut between the ventricle and the spacer (250) so that the array of the ventricular tethers (258) around the spacer (250) holds the spacer (250) within the valve leaflets.

5. The system of any one of the preceding claims, wherein the tethers (254) are routed through the spacer (250) to a control handle (262) for length adjustment.

6. The system of claim 5, wherein the control handle (262) enables adjustment of the lengths of the tethers (254) using a plurality of thumb wheels (264).

7. The system of claim 6, further comprising a control ring (266) for locking the position of the tethers (254) with respect to the spacer (250).

8. The system claim 1, further comprising a deployment instrument (270) including a proximal handle (272) and a flexible shaft (252) attached to the proximal handle (272); wherein the spacer (250) is provided on the end of the flexible shaft (252).

9. The system of claim 8, wherein a control ring (266) on the handle can be used to bend the flexible shaft (252).

10. The system of claim 1, wherein each tether extends across from one side to an opposite side of the peripheral tissue annulus and bifurcates in a mid-portion in a loop-like snare (364) sized to receive the spacer (250) in the compressed delivery configuration, wherein the snares (364) are delineated by junctions (366), and wherein expansion of the spacer (250) to the radially expanded implant configuration tightens the snares (364) and forms a harness around the spacer (250) that holds the spacer (250) centrally within the valve leaflets.
